# EUROPEAN PATENT APPLICATION

(11) **EP 4 218 825 A1**
(43) Date of publication of application: **02.08.2023**
(21) Application number: 21872964.8
(22) Date of filing: 24.09.2021
(51) Int. Cl.: A61K 47/64, A61K 38/26, A61P 3/10, A61P 3/04, C07K 14/435, A61K 49/00

(54) **CONJUGATE OF FUNCTIONAL POLYPEPTIDE VARIANT, AND USE THEREOF**

(30) Priority: 25.09.2020 KR 20200125215; 29.01.2021 KR 20210013537; 08.02.2021 KR 20210017853
(71) Applicant: Proabtech Inc., Gwangju 61005 (KR)
(72) Inventor: CHO, Jeong Haeng, Gimpo-si, Gyeonggi-do 10091 (KR); SHIN, Sun Oh, Gwangju 62052 (KR); KIM, Hyun Woo, Seoul 07635 (KR); KIM, Hyeongseok, Seoul 07775 (KR); BAK, Dong Ho, Jeonju-si, Jeollabuk-do 54807 (KR); JEON, Su Jung, Seoul 07678 (KR); KWON, Inchan, Gwangju 61005 (KR); YANG, Byungseop, Seoul 07794 (KR)
(74) Representative: Schön, Christoph
(86) International application number: PCT/KR2021/013077
(87) International publication number: WO 2022/065936

(57) **Abstract**

The present application relates to a functional polypeptide variant-lasting protein conjugate. The functional polypeptide variant-lasting protein conjugate of the present application is prepared by an inverse electron-demand Diels-Alder (IEDDA) reaction using a tetrazine group. In addition, the present application provides a functional polypeptide variant including a non-natural amino acid. The non-natural amino acid included in the functional polypeptide of the present application is characterized by including a tetrazine group or triazine group.

## Description

### FIELD OF THE INVENTION

The present application relates to a functional polypeptide variant-lasting protein conjugate. As one embodiment of the present application, a functional polypeptide variant-lasting protein conjugate, a method of preparing the same, and a use thereof are provided.

In addition, as one aspect of the present application, a functional polypeptide variant including a non-natural amino acid is provided. Here, the non-natural amino acid is characterized by including a tetrazine group or a triazine group.

### BACKGROUND

When polypeptides are used to treat or diagnose diseases, there are several problems in terms of pharmacokinetics. For example, there is a problem in that such polypeptides are continuously removed by glomerular filtration, pinocytosis, and an immune response in a patient's body. Therefore, when a polypeptide is used as a therapeutic or diagnostic agent for a specific disease, it is very important to extend the duration of efficacy by alleviating a removal rate in a patient's body due to the above phenomenon.

To improve the above problem, research is being conducted to improve the half-life by conjugating other compounds or proteins to the polypeptide. For example, to extend the administration cycle of a polypeptide therapeutic agent in the development of a polypeptide therapeutic agent, those of ordinary skill in the art conjugate polyethylene glycol or albumin to the polypeptide, thereby increasing the half-life of the polypeptide in the body. However, in the case of using existing compounds and/or methods, there is a problem in that the production yield is low or the rate of a production reaction is slow.

### DESCRIPTION

### TECHNICAL PROBLEM

The present application is for increasing the half-life of a polypeptide therapeutic agent in the body using a functional polypeptide variant-lasting protein conjugate.

The present application is directed to providing a functional polypeptide variant-lasting protein conjugate, a preparation method thereof, and a use thereof.

Another aspect of the present application provides a functional polypeptide variant including a non-natural amino acid having a tetrazine or triazine group.

### TECHNICAL SOLUTION

An embodiment of the present application provides a functional polypeptide variant-lasting protein conjugate represented by Formula 1:

[Formula 1] P₁-J₁-A₂-J₂-P₂

wherein,
P₁ is a functional polypeptide variant moiety, which is derived from a functional polypeptide variant, wherein the functional polypeptide variant includes a first click chemistry functional group, and the first click chemistry functional group is tetrazine or a derivative thereof, or triazine or a derivative thereof,
A₂ is a second anchor moiety,
wherein the second anchor moiety is any one selected from the group consisting of substituted or unsubstituted C₁₋₂₀ alkylene, substituted or unsubstituted C₁₋₂₀ heteroalkylene, -substituted or unsubstituted C₁₋₂₀ alkylene-[PEG]n-, -substituted or unsubstituted C₁₋₂₀ heteroalkylene-[PEG]n-, -substituted or unsubstituted C₁₋₂₀ alkylene-[PEG]n-substituted or unsubstituted C₁₋₂₀ alkylene-, -substituted or unsubstituted C₁₋₂₀ alkylene-[PEG]n-substituted or unsubstituted C₁₋₂₀ heteroalkylene-, and -substituted or unsubstituted C₁₋₂₀ heteroalkylene-[PEG]n-substituted or unsubstituted C₁₋₂₀ heteroalkylene-,
wherein, the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S,
wherein, the substitution is substitution with one or more non-hydrogen substituents, in which the non-hydrogen substituent is any one selected from the group consisting of halogen, C₁₋₃ alkyl, -NH₂, =O, and =S, and
wherein, n is an integer of 1 to 12;
P₂ is a lasting protein moiety, which is derived from a lasting protein;
J₁ is a first conjugation moiety, wherein the first conjugation moiety is formed by the reaction of the first click chemistry functional group including a tetrazine group or a derivative thereof and a triazine group or a derivative thereof with a second click chemistry functional group which is a dienophile; and
J₂ is a second conjugation moiety, wherein the second conjugation moiety is formed by the reaction of a thiol reactive group with a thiol residue.
In one embodiment, wherein the second anchor moiety may be -A₂₁-A₂₂-A₂₃-, wherein A₂₁ may be -OC(=O)NH-, wherein A₂₂ may be absent, or unsubstituted C₁₋₁₀ alkylene, -unsubstituted C₁₋₁₀ alkylene-[PEG]n-, or -substituted C₁₋₁₀ heteroalkylene-[PEG]n-, wherein n may be an integer of 1 to 12, and wherein A₂₃ may be absent, or - C(=O)NH-, or -C(=O)O-.

In one embodiment, wherein the functional polypeptide variant-lasting protein conjugate represented by Formula 1-3 may be provided:

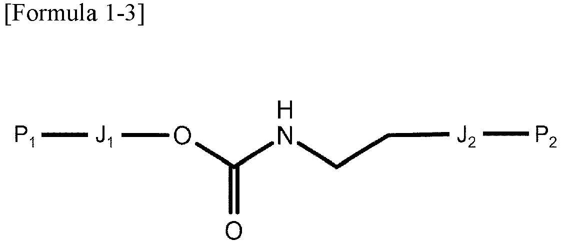

In one embodiment, wherein the functional polypeptide variant-lasting protein conjugate represented by Formula 1-4 may be provided:

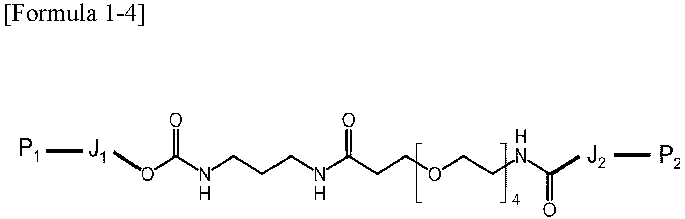

In one embodiment, wherein the second anchor moiety may be a substituted hydrocarbon chain including one or more heteroatoms, wherein the each heteroatom may be selected from N, O, and S, wherein the substitution is substitution with one or more non-hydrogen substituents, and wherein the each non-hydrogen substituent may be any one selected from the group consisting of halogen, C₁₋₃ alkyl, -NH₂, =O, and =S.

In one embodiment, the first click chemistry functional group may be represented by any one of the following structures: 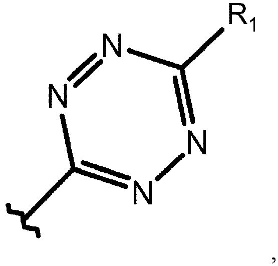 and 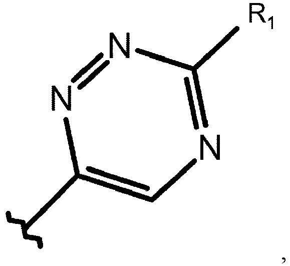 wherein R₁ may be any one selected from H, halogen, C₁₋₃ alkyl, C₃₋₆ cycloalkyl, C₃₋₆ heterocycloalkyl, aryl, and heteroaryl, wherein the heterocycloalkyl or heteroaryl includes one or more heteroatom groups selected from the group consisting -NH-, -O-, -S-, -O-N=, -S(=O)-, and -S(=O)₂-, or one or more heteroatoms selected from the group consisting of O, N, and S.

In one embodiment, the non-natural (unnatural) amino acid included in the functional polypeptide variant may be represented by the following structure: 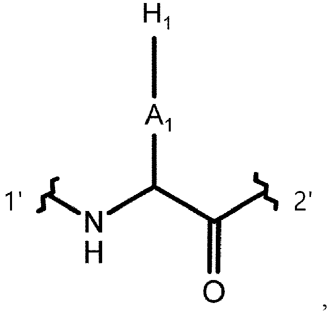 wherein H₁ may be the first click chemistry functional group, wherein the first chemistry functional group may include a tetrazine group or derivative thereof, or a triazine group or derivative thereof; A₁ may be a first anchor moiety, wherein the first anchor moiety is absent or -A₁₁-A₁₂-, wherein A₁₁ may be absent or C₁₋₅ alkylene, and wherein A₁₂ is absent, or any one selected from the group consisting of [arylene]p, - [arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene- heteroarylene-, [heteroarylene]p, -[heteroarylene]p-C₁₋₅ alkylene-, -[heteroarylene]p-C₁₋₅ heteroalkylene-, - heteroarylene-C₁₋₅ alkylene-arylene-, -heteroarylene-C₁₋₅ alkylene-heteroarylene-, - heteroarylene-C₁₋₅ heteroalkylene-arylene-, and - heteroarylene-C₁₋₅ heteroalkylene-heteroarylene-, wherein p may be an integer of 1 to 3, and wherein the heteroalkylene or heteroarylene may include one or more heteroatom groups selected from the group consisting of -NH-, -O-, -S-, -O-N=, -S(=O)-, and -S(=O)₂-, or one or more heteroatoms selected from the group consisting of O, N, and S; and wherein the non-natural amino acid may be linked with neighboring amino acid through 1' and 2' within or at the end of an amino acid sequence of the functional polypeptide, to form the functional polypeptide variant of the present application.

In one embodiment, wherein the non-natural amino acid included in the functional polypeptide variant may be represented by the following structure: 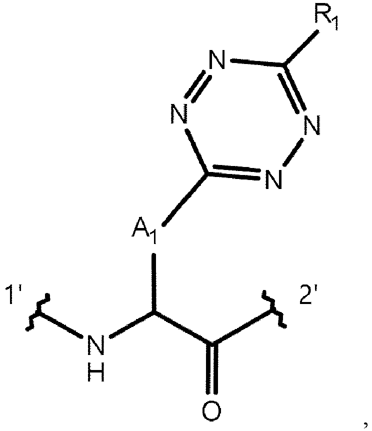 wherein R₁ may be any one selected from the group consisting of H, halogen, methyl, aryl, and heteroaryl.

In one embodiment, wherein the non-natural amino acid included in the functional polypeptide variant may be represented by the following structure: 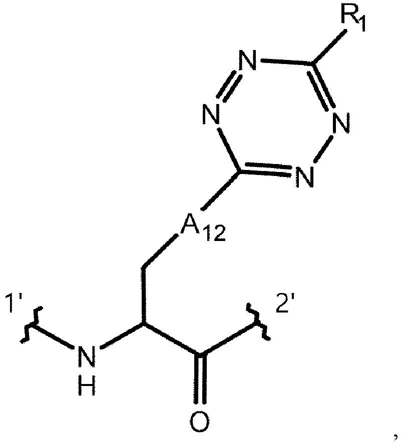 wherein R₁ may be any one selected from the group consisting of H, halogen, methyl, aryl, and heteroaryl.

In one embodiment, wherein the non-natural amino acid included in the functional polypeptide variant may be represented by the following structure: 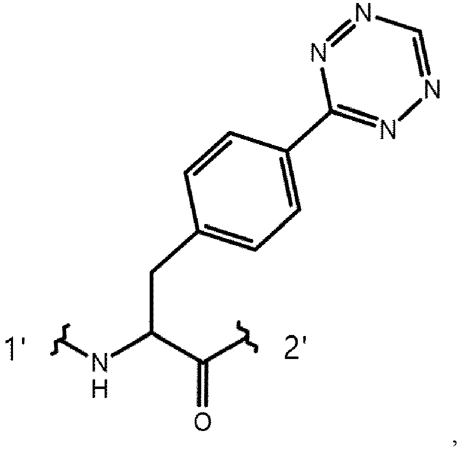 or

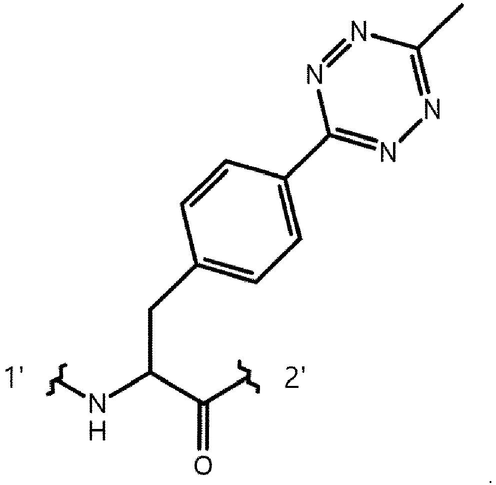

In one embodiment, wherein the second anchor moiety may be derived from a linker, wherein the linker may include a second click chemistry functional group which is a dienophile group reacting with the first click chemistry functional group, a thiol reactive group reacting with a thiol residue, and the second anchor moiety.

In one embodiment, wherein the dienophile group may be any one selected from the group consisting of a trans-bicyclo[6.1.0]nonene group, a trans-cyclooctene (TCO) group, a methylcyclopropene group, a bicyclo[6.1.0]nonyne group, a cyclooctyne group, a norbornene group, a cyclopentene group, and a styrene group.

In one embodiment, wherein the dienophile group may be a trans-cyclooctene (TCO) group.

In one embodiment, wherein the thiol reactive group may be a maleimide group, or an APN group.

In one embodiment, wherein the thiol reactive group may be any one of the following structures: 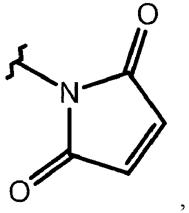 and

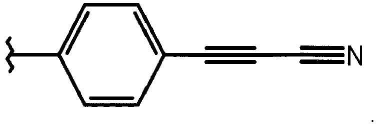

In one embodiment, wherein the first conjugation moiety may be formed by the reaction of the tetrazine group with a trans-cyclooctene group, and wherein the first conjugation moiety may be any one structure selected from the following structures: 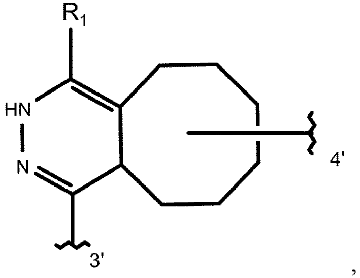 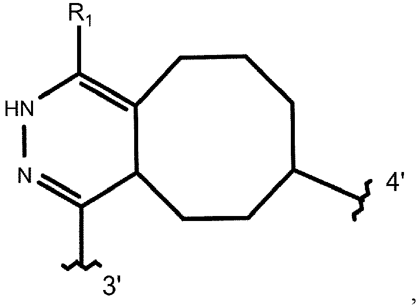 and 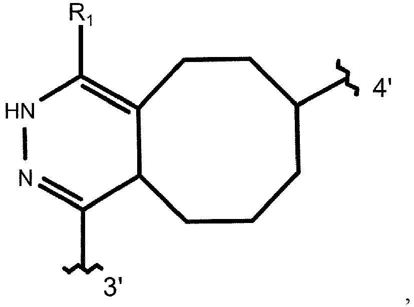 wherein 3' is linked with the functional polypeptide variant moiety, and 4' is linked with the second anchor moiety, and R₁ is any one selected from the group consisting of H, halogen, methyl, aryl, and heteroaryl.

In one embodiment, wherein the first conjugation moiety may be

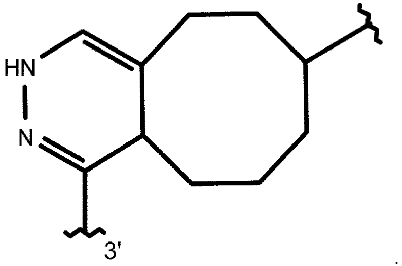

In one embodiment, wherein the second conjugation moiety may be formed by the reaction of the thiol reactive group with the thiol residue contained in a cysteine of the lasting protein, and wherein the second conjugation moiety may be any one selected from the following structures: 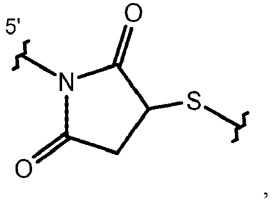 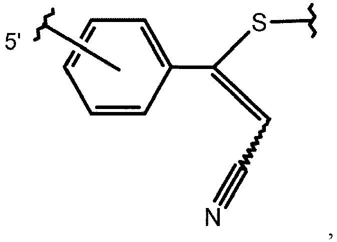 and 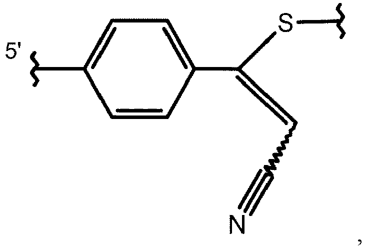 wherein 5' may be linked with the second anchor moiety, and S may be derived from the thiol residue contained in the cysteine of the lasting protein.

In one embodiment, wherein the functional polypeptide variant may be any one selected from the group consisting of a GLP1 variant, an hGH variant, a Uox subunit variant, and an sfGFP variant.

In one embodiment, wherein the functional polypeptide variant may be the GLP1 variant, and wherein the GLP1 variant may have any one sequence selected from the sequences of SEQ ID NOs: 1 to 26.

In one embodiment, wherein the GLP1 variant may have the sequence of SEQ ID NO: 2.

In one embodiment, wherein the functional polypeptide variant may be an hGH variant, wherein the hGH variant may have a sequence in which one or more standard amino acids in SEQ ID NO: 27 are substituted with non-natural amino acids, wherein the standard amino acid substituted with non-natural amino acid may be any one selected from the group consisting of Q29, Y35, L52, E56, E66, Q69, E74, F92, S106, Q122, R127, L128, G131, R134, Q141, T142, Y143, K145, D147, N149, N152, D153, and E186.

In one embodiment, wherein the functional polypeptide variant may be the hGH variant, wherein the hGH variant may include any one sequence selected from the group consisting of the sequences of SEQ ID NOs: 28 to 50.

In one embodiment, wherein the hGH variant may include the sequence of SEQ ID NO: 28.

In one embodiment, wherein the functional polypeptide variant may be an sfGFP variant, wherein the sfGFP variant include a sequence in which one or more standard amino acids in SEQ ID NO: 51 are substituted with non-natural amino acids, wherein the standard amino acid substituted with non-natural amino acid may be any one selected from the group consisting of E8, T11, V13, G26, K28, T40, N41, T51, D78, K103, K109, G118, K128, D131, D135, H150, Q159, K168, D175, D192, V195, N200, P213, and G230.

In one embodiment, wherein the functional polypeptide variant may be the sfGFP variant, wherein the sfGFP variant includes any one sequence selected from the group consisting of the sequences of SEQ ID NOs: 52 to 75.

In one embodiment, wherein the sfGFP variant may include the sequence of SEQ ID NO: 52.

In one embodiment, wherein the lasting protein may be albumin or a variant thereof, or an albumin-binding domain or a variant thereof.

In one embodiment, wherein the lasting protein may be albumin or a variant thereof, which may have any one sequence selected from the group consisting of the sequences of SEQ ID NOs: 76 to 87.

In one embodiment, wherein the lasting protein may be an albumin-binding domain or a variant thereof, wherein the albumin-binding domain may include the sequence of SEQ ID NO: 88.

In one embodiment, wherein the functional polypeptide variant-lasting protein conjugate represented by Formula 1-3 may be provided: 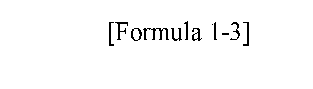 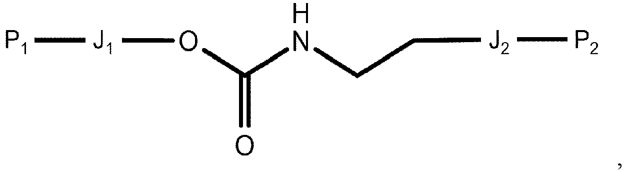 wherein,
the lasting protein is albumin or a variant thereof, or an albumin-binding domain or a variant thereof, and wherein the lasting protein includes a cysteine which includes a thiol residue;
the first conjugation moiety is 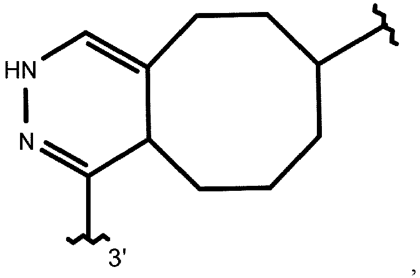
wherein 3' is linked with P₁, and
the second conjugation moiety is 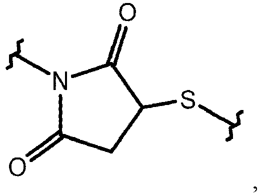 or 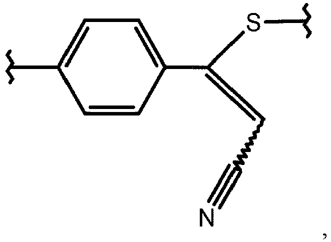
wherein S is derived from the thiol residue of the lasting protein.

In one embodiment, wherein the functional polypeptide variant may be any one selected from a GLP1 variant, an hGH variant, a Uox subunit variant, and an sfGFP variant.

In one embodiment, wherein the functional polypeptide variant may be a GLP1 variant, which may have the sequence of SEQ ID NO: 2, and the lasting protein may have the sequence of SEQ ID NO: 76.

In one embodiment, wherein the functional polypeptide variant may be an hGH variant, which may have the sequence of SEQ ID NO: 28, and the lasting protein may have the sequence of SEQ ID NO: 76.

In one embodiment, wherein the functional polypeptide variant may be an sfGFP variant, which may have the sequence of SEQ ID NO: 52, and the lasting protein may include the sequence of SEQ ID NO: 88.

An embodiment of the present application provides a method of preparing a functional polypeptide variant-lasting protein conjugate, which includes mixing a first composition containing a lasting protein, a second composition containing a linker, and a third composition containing a functional polypeptide variant.

Wherein the lasting protein is albumin or its variant including a thiol residue, or an albumin-binding domain or its variant including a thiol residue, wherein the linker includes a dienophile group which reacts an inverse electron demand Diels-Alder (IEDDA) reaction with a tetrazine group or its derivative, or a triazine group or its derivative, and a thiol reactive group reacting with the thiol residue, and wherein the functional polypeptide variant includes one or more non-natural amino acids each including the tetrazine or its derivative, or the triazine or its derivative.

In one embodiment, wherein the method may comprise: preparing a first reaction composition by mixing the first composition and the second composition; preparing a second reaction composition by mixing the first reaction composition and the third composition; and obtaining the functional polypeptide variant-lasting protein conjugate from the second reaction composition.

An embodiment of the present application provides a method of preparing a functional polypeptide variant-lasting protein conjugate, which includes: preparing a partner fusion protein-lasting protein conjugate by mixing a first composition containing a lasting protein, a second composition containing a linker, and a third composition containing a partner fusion protein; and removing a partner protein from the partner fusion protein-lasting protein conjugate.

Wherein the lasting protein is albumin or its variant, or an albumin-binding domain its variant including a thiol residue, wherein the linker includes a dienophile group which reacts an inverse electron demand Diels-Alder reaction with a tetrazine group or its derivative, or a triazine group or its derivative, and a thiol reactive group reacting with the thiol residue, wherein the partner fusion protein has a [partner protein]-[amino acid-binding moiety]-[functional polypeptide variant] or [partner protein]-[functional polypeptide variant] structure, and wherein the functional polypeptide variant includes one or more non-natural amino acids including the tetrazine or its derivative, or the triazine or its derivative.

In one embodiment, wherein the method may comprise: preparing a first reaction composition by mixing the first composition and the second composition; preparing a second reaction composition including the partner protein-lasting protein conjugate by mixing the first reaction composition and the third composition; preparing a third reaction composition by adding a polypeptide cleaving enzyme to the second reaction composition to remove the partner protein from the partner fusion protein-lasting protein conjugate; and obtaining the functional polypeptide variant-lasting protein conjugate from the third reaction composition.

An embodiment of the present application provides a pharmaceutical composition for treating a target disease including a GLP1 variant-HSA conjugate. Wherein the target disease is any one selected from diabetes, long-acting diabetes, obesity, type 2 diabetes, prediabetes, a liver disease, a metabolic disease, and a cardiovascular disease.

An embodiment of the present application provides a pharmaceutical composition for treating growth hormone deficiency including an hGH variant-HSA conjugate.

An embodiment of the present application provides a method of treating a target disease, which includes administering a composition including a GLP1 variant-HSA conjugate to a subject. Wherein, the target disease may be any one selected from diabetes, long-acting diabetes, obesity, type 2 diabetes, prediabetes, a liver disease, a metabolic disease, and a cardiovascular disease.

An embodiment of the present application provides a method of treating growth hormone deficiency, which includes administering a composition including an hGH variant-HSA conjugate to a subject.

### EFFECTS OF THE INVENTION

As described in the present application, a functional polypeptide variant-lasting protein conjugate is provided. The functional polypeptide variant-lasting protein conjugate has a longer half-life in the body and less immunogenicity.

In addition, the functional polypeptide variant-lasting protein conjugate of the present application is prepared by an inverse electron-demand Diels-Alder reaction (IEDDA) reaction using a tetrazine group or triazine group of a non-natural amino acid included in a functional polypeptide variant, so it has higher production yield, and/or a higher production rate than when using a strain-promoted alkyne-azide cycloaddition (SPAAC) reaction.

### BRIEF DESCRIPTION OF THE DRAWINGS

FIG. 1 is a schematic diagram of a vector encoding sfGFP-GLP1 and a sfGFP-GLP1 variant.
FIG. 2 is a schematic diagram of a transformed cell line for producing a sfGFP-GLP1 variant.
FIG. 3 shows the SDS-PAGE result for a sample including a sfGFP-GLP1 variant obtained from the transformed cell line.
FIG. 4 shows the SDS-PAGE result for a purified sfGFP-GLP1 variant;
FIG. 5 shows the structural formula of Cy3 TCO.
FIG. 6 shows the result confirming whether Cy3 TCO is conjugated to a sfGFP-GLP1 variant through SDS-PAGE.
FIG. 7 shows the reaction between frTet and an HSA-linker complex.
FIG. 8 shows the SDS-PAGE result for samples obtained 0 minutes, 10 minutes, 30 minutes, 1 hour, and 2 hours after the reaction of a sfGFP-GLP1 variant and an HSA-linker1 complex.
FIG. 9 shows SDS-PAGE result for a sfGFP-GLP1 variant-HSA conjugate sample obtained 16 hours after the reaction of a sfGFP-GLP1 variant and a HSA-linker1 complex.
FIG. 10 shows the chromatography result for a purified sfGFP-GLP1 variant-HSA conjugate.
FIG. 11 shows the chromatography result for a GLP1 variant-HSA conjugate obtained after sfGFP removal.
FIG. 12 shows the SDS-PAGE result for a GLP1 variant-HSA conjugate obtained after sfGFP removal.
FIG. 13 shows the chromatography result for a GLP1 variant2-HSA conjugate prepared using a strain-promoted alkyne-azide cycloaddition (SPAAC) reaction.
FIG. 14 shows the chromatography result for a GLP1 variant-HSA conjugate prepared using an inverse electron-demand Diels-Alder (IEDDA) reaction.
FIG. 15 shows the result of the pharmacokinetic evaluation for a GLP1 variant-HSA conjugate.
FIG. 16 is the schematic diagram of a vector encoding sfGFP-hGH and a sfGFP-hGH variant.
FIG. 17 is the schematic diagram of a transformed cell line for producing a sfGFP-hGH variant.
FIG. 18 shows the result for confirming transformed C321delA.exp [pDule C11RS][pTac-sfGFP-hGH-141Amb] cell colonies.
FIG. 19 shows the SDS-PAGE result for a sample including the sfGFP-hGH variant obtained from the transformed cell line.
FIG. 20 shows the result of confirming whether Cy3 TCO is conjugated to the sfGFP-hGH variant through SDS-PAGE.
FIG. 21 shows the SDS-PAGE result for a sample obtained after the reaction of the sfGFP-hGH variant and a HSA-linker1 complex.
FIG. 22 shows the cation exchange chromatography result for separation of a sfGFP-hGH variant-HSA conjugate.
FIGS. 23 and 24 show the SDS-PAGE results for cation exchange chromatography fractions for separation of a sfGFP-hGH variant-HSA conjugate.
FIG. 25 is the SDS-PAGE result of a sample obtained after sfGFP removal from the sfGFP-hGH variant-HSA conjugate.
FIG. 26 is the size exclusion chromatography result for hGH variant-HSA conjugate samples.
FIGS. 27 and 28 are the results of confirming the purity of the hGH variant-HSA conjugate using high performance liquid chromatography.
FIG. 29 is the result of confirming the pharmacokinetics (PK) of the hGH variant-HSA conjugate.
FIG. 30 is the chromatography result for a purified sfGFP variant-ABD conjugate.
FIG. 31 is the result of purification of the purified sfGFP variant-ABD conjugate.
FIG. 32 is the result of confirming the PK of the sfGFP variant-ABD conjugate.
FIG. 33 is the H NMR result of a non-natural amino acid frTet.
FIG. 34 shows the SDS-PAGE results for AgUox-WT and AgUox-frTet: (A) represents the Coomassie Brilliant Blue (CBB) staining result for an AgUox variant, Mw is a molecular weight marker, BI indicates before induction, and AI indicates after induction; (B) represents a CBB staining protein gel for a purified AgUox variant; and (C) represents a fluorescence image and CBB stained protein gel for a AgUox variant incubated in the condition of the presence or absence of TCO-Cy3.
FIG. 35 shows the flight mass spectra of trypsin-digested fragments of AgUox-WT(A) and AgUox-frTet(B). The weight of a wild-type Arthrobacter globiformis-derived urate oxidase fragment (AgUox-WT fragment) YNTVEVDFDAVYASVR (SEQ ID NO: 111) was compared with an urate oxidase variant fragment (AgUox-frTet fragment) YNTVXVDFDAVYASVR (SEQ ID NO: 112, X indicates frTet). The fragment peak AVIETHPEIDEIKMSLPNK (SEQ ID NO: 113) was used as a control.
FIG. 36 shows the protein gel of the reaction mixture of MAL-HSA (indicated as MAL) or APN-HSA (indicated as APN), and AgUox-frTet. Mw represents the molecular weight standard.
FIG. 37 shows the size exclusion chromatogram and protein gel of the fraction of the conjugate mixture of AgUox-frTet, and a MAL-HSA (A) or APN-HSA (B). The eluted fraction was loaded on the protein gel, and then stained with Coomassie Brilliant Blue.
FIG. 38 shows the PK profile result for AgUox-WT and AgUox-HSA conjugates. The serum activities of remaining AgUox-WT and AgUox-HSA conjugates were measured at the early period (0 to 84 hours) and the late period (84 to 120 hours). t^{e}_{1/2} and t^{l}_{1/2} represent the half-life of serum at the early and late periods, respectively.

### DETAILED DESCRIPTION

### Definition of Terms

Unless defined otherwise, all technical and scientific terms used herein have the meaning commonly understood by one of ordinary skill in the art of the subject of the present application.

As used herein, and unless otherwise specified, the terms below have the meanings ascribed thereto.

In some embodiments, chemical structures are disclosed with their corresponding chemical names. In case of a dispute, the meaning should be determined by the chemical structure, which takes precedence over determination by the chemical name.

The "halogen" or "halo" includes fluor, chloro, bromo, and iodo, included in the elements of the halogen group in the periodic table.

The term "hetero" used herein refers to compounds or groups containing at least one heteroatom. The term "heteroatom" is an atom, not carbon or hydrogen, which includes, for example, B, Si, N, P, O, S, and Se, and preferably, multivalent elements such as N, O, and S, or monovalent elements such as F, Cl, Br, and I, but the present application is not limited thereto.

The term "substituted" used herein means that at one or more hydrogen atoms on an atom are replaced with a substituent including deuterium and hydrogen variants, in which the valence of the atom is normal and the substituted compound is stable. When the substituent is oxygen (O), this means that two hydrogen atoms are substituted. When one substituent is halogen (e.g., Cl, F, Br, and I), this means that one hydrogen atom is substituted with halogen. When there are two or more substituents in one group, the substituents in the group may be the same or different. Unless stated otherwise, the type and number of substituents may be arbitrary as long as they are chemically achievable.

The "alkyl" or "alkane" refer to linear or branched hydrocarbons, which are completely saturated. Generally, an alkyl group has 1 to approximately 20 carbon atoms, and preferably, 1 to approximately 10 carbon atoms unless defined otherwise. The linear and branched alkyl groups may include, for example, methyl, ethyl, n-propyl, iso-propyl, n-butyl, sec-butyl, tert-butyl, iso-butyl, pentyl, hexyl, heptyl, octyl, nonyl, and decyl. The alkyl groups may have a cyclic structure. For example, the term "C_{x-y}" is intended to include a residue containing x or y number of carbons in a chain or ring when used with an alkyl group. For example, the term "C_{x-y} alkyl" refers to substituted or unsubstituted, linear alkyl groups, branched alkyl groups or cyclic alkyl groups, each of which has x or y number of carbons in a chain, and exemplarily includes a haloalkyl group such as difluoromethyl or 2,2,2-trifluoroethyl. C₀ alkyl refers to hydrogen. Examples of C₁₋₄ alkyl include methyl, ethyl, n-propyl, iso-propyl, n-butyl, sec-butyl, tert-butyl, iso-butyl, difluoromethyl, and 2,2,2-trifluoroethyl, but the present application is not limited thereto.

The term "heteroalkyl" used herein refers to alkyls including one or more heteroatoms.

The term "alkylene" used as a molecule itself or a part of a molecule, refers to divalent radicals derived from alkyl. The alkylene may include -CH₂-, -CH₂CH₂-, - CH₂CH₂CH₂-, and -CH₂CH₂CH₂CH₂-, but the present application is not limited thereto. For example, the alkylene may be used as C₂ alkylene, representing an alkylene group containing two carbon atoms in the main chain. In the present application, the C_{x-y} alkylene (when the 'alkylene' is used alone without the term "substituted" or "hetero") is used to include all alkylenes having X or Y number of carbons (C), which is substituted or unsubstituted, which contains or does not contain heteroatom.

The term "heteroalkylene" used as a molecule itself or a part of a molecule, refers to divalent radicals derived from heteroalkyl. Examples of heteroalkylene groups include -CH₂₋CH₂-O-CH₂-CH₂- and -CH₂-O-CH₂-CH₂-NH-CH₂-, but the present application is not limited thereto. A heteroalkylene group may include one or more heteroatoms at a location other than an end of the chain or branch, in which the heteroatoms may be the same or different from each other. The heteroalkylene group may include one or more heteroatoms at an end of a chain or branch, or both ends of the chain and branch, and each heteroatom may be the same or different from each other.

The "cycloalkane" or "cycloalkyl" refers to completely saturated cyclic hydrocarbons. The "cycloalkyl" includes monocyclic and polycyclic rings. Unless defined otherwise, generally, monocyclic cycloalkyl groups have 3 to approximately 10 carbon atoms, and generally, 3 to 8 carbon atoms. Rings except the first ring of polycyclic cycloalkyl may be selected from saturated, unsaturated and aromatic rings. The cycloalkyl includes a bicyclic molecule, in which 1, 2, or 3 or more atoms are shared between two rings.

The "aryl" group refers to groups derived from an aromatic ring, or an aromatic hydrocarbon such as phenyl or naphthyl. The "aryl" includes monocyclic and polycyclic rings.

The "heteroaryl" group refers to an aromatic ring group including one or more heteroatoms. Examples of heteroaryl groups may include a pyridine group, a diazine group, a triazole group, and a triazine group, and a tetrazine group.

The "heteroaryl" group refers to an aromatic group including one or more heteroatoms.

The compound of the present application may have a specific geometric or stereoisomeric form. Unless stated otherwise, when the compound is disclosed in the present application, isomers of the compound, including cis- and trans-isomers, (-)- and (+)-enantiomers, (R)- and (S)-enantiomers, diastereomers, a (D)-isomer, a (L)-isomer, and a racemic are included in the scope of the present application. That is, when there is no specific indication related to isomers in the formulas disclosed in the present application, the disclosed formulas include all possible isomers.

The term "approximately" used herein refers to being close to a certain quantity, and means an amount, level, value, number, frequency, percentage, dimension, size, amount, weight or length changed to approximately 30, 25, 20, 25, 10, 9, 8, 7, 6, 5, 4, 3, 2 or 1% with respect to the reference amount, level, value, number, frequency, percentage, dimension, size, amount, weight or length.

The "click chemistry reaction" used herein, introduced by K. Barry Sharpless (Scripps Research Institute), is a concept that explains complementary chemical functional groups and chemical reactions designed for two molecules to form covalent bonds rapidly and stably. The click chemistry reaction does not mean a specific reaction, but the concept of a rapid and stable reaction. The click chemistry reaction is modular, has a wide range, has a high yield, is not importantly considered for byproduct, is stereospecific, is physiologically stable, has a large thermodynamic driving force (e.g., 84 kJ/mol or more), and has high atom economy. Examples of the click chemistry reaction include 1) Huisgen 1,3-dipolar cycloaddition (Tornoe et al., refer to Journal of Organic Chemistry (2002) 67: 3075-3064, etc.), 2) Diels-Alder reaction, 3) nucleophilic addition for small strained rings such as epoxide and aziridine, 4) nucleophilic addition for an activated carbonyl group, and 5) addition reaction for C-C double bonds or triple bonds. The meaning of the click chemistry reaction has to be properly interpreted according to the context, and includes all other meanings that are recognized by those of ordinary skill in the art.

The "biorthogonal reaction" used herein means an selective reaction between residues introduced from the outside, not a reaction with an intrinsic molecule *in vivo.* When a reaction is "biorthogonal," it has the characteristic of being very stable in the body, since intrinsic molecule in the body is not involved in the reaction or a reaction product.

The "standard amino acid" used herein refers to 20 types of amino acids synthesized through the transcription and translation of genes in the body of an organism. Specifically, the standard amino acids include alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys K), methionine (Met, M), phenylalanine (Phe, F), proline (Pro, P), serine (Ser, S), threonine (Thr, T), tryptophan (Trp, W), tyrosine (Tyr, Y), and valine (Val, V). Each standard amino acid has a corresponding DNA codon, and may be represented by the common one-letter or three-letter notation for amino acids. The subject referred to by the term "standard amino acid" should be appropriately interpreted according to the context, and includes all other meanings that can be recognized by those of ordinary skill in the art.

The term "non-natural amino acid" used herein refers to an amino acid which is artificially synthesized without being synthesized in the body. The examples of non-natural amino acid include 4-(1,2,3,4-tetrazin-3-yl) phenylalanine and 4-(6-methyl-s-tetrazin-3-yl)phenylalanine, etc.. Since the non-natural amino acid has no corresponding DNA codon and cannot be expressed in common one-letter or three-letter notation for amino acids, description is made using other characters and additional supplementation. The subject referred to by the term "non-natural amino acid" should be appropriately interpreted according to the context, and includes all other meanings that can be recognized by those of ordinary skill in the art.

Unless stated otherwise, when describing a peptide sequence in the specification, it is written in the direction from the N-terminus to the C-terminus using the one-letter or three-letter notation for amino acids. For example, when expressed as RNVP, it indicates a peptide in which arginine, asparagine, valine, and proline are sequentially linked in the direction from the N-terminus to the C-terminus. In another example, when expressed as Thr-Leu-Lys, it indicates a peptide in which threonine, leucine, and lysine are sequentially linked from the N-terminus to the C-terminus. In the case of amino acids that cannot be expressed by the one-letter or three-letter notation, other characters are used and additionally supplemented.

The term "immunogenicity" used herein, lexically means "the property of acting as antigen capable of eliciting an immune response". There are various methods for measuring the immunogenicity of a specific antigen, and these methods may be suitably adopted or designed depending on the purpose. For example, there may be 1) a method of confirming whether an IgG, IgA, and/or IgE-type antibody is produced in a subject's body when the antigen is administered to the subject's body, 2) a method of confirming the period of producing the IgG, IgA, and/or IgE-type antibody according to an administration cycle, 3) a method of confirming a titer of the induced antibody against the antigen, and 4) a method of measuring an effect caused by an action mechanism when the action mechanism of the induced antibody is identified, but the present application is not limited thereto. The subject referred to by the term immunogenicity should be properly interpreted according to the context, and includes all other meanings that can be recognized by those of ordinary skill in the art.

The term "treatment" refers to an approach for obtaining beneficial or desirable clinical results. For the purpose of the present application, beneficial or desirable clinical results include, but are not limited to, the alleviation of a symptom, reduction in disease extent, the stabilization of a disease state (i.e., not worsening), the delay or slowing of disease progression, the prevention of a disease, the improvement or temporary alleviation and reduction (partially or totally) in disease state, whether detection is or is not possible. The treatment refers to both therapeutic treatments and preventive or prophylactic methods.

The "patient" refers to an animal in need of treatment that can be achieved by the molecule of the present application. Animals that can be treated according to the present application include vertebrates, and particularly mammals such as a murine, bovine, canine, equine, feline, ovine, porcine and primate (including human and non-human primates).

### [Problems of related art]

### Problems of related art

There is a problem in that a polypeptide with a low molecular weight is eliminated from the body through filtration in the glomerulus, or eliminated from the body by the reticuloendothelial system (RES) regardless of a molecular weight. Accordingly, a polypeptide administered into the body's blood has a problem of a short half-life, which in turn reduces the efficacy of a drug or shortens an administration cycle, causing discomfort to a patient.

As related art for improving the short half-life of such a polypeptide, there is a method of linking or bonding polyethylene glycol (PEG) to a polypeptide. However, such a method has a problem such as difficulty in controlling the number of attached PEGs, the occurrence of immunogenicity by PEG, or a decrease in activity of a peptide due to the linkage of PEG to the active site of the peptide or a site affecting it.

As another related art, there is an albumination method for linking or bonding albumin to a polypeptide. However, similar to the above, this method had a problem of a significant decrease in the activity of a polypeptide by randomly linking albumin to the active site of the polypeptide or a site affecting it.

In the method using albumination, the inventors conjugated a polypeptide with albumin through strain-promoted alkyne-azide cycloaddition (SPAAC; e.g., reaction of an azide group with a dibenzocyclooctyn group). However, such a method has a problem of a low yield because the rate of the strain-promoted cycloaddition (SPAAC), which is a coupling reaction of AzF and DBCO, is slow. In addition, there is a problem in that an AzF residue, which is not coupled due to the slow reaction rate, is present, and may be exposed to cause an unintended reaction in the body.

Therefore, the present application provides a polypeptide-albumin conjugate that solves the above-described problems such as a short half-life and a low production yield, and a preparation method thereof.

### [Characteristics of present application]

The present application provides a functional polypeptide variant-lasting protein conjugate (functional polypeptide variant-persistent protein conjugate) in which a lasting (persistent) protein is site-specifically linked to a functional polypeptide variant.

The functional polypeptide variant-lasting protein conjugate of the present application has the following characteristics while solving the above-described problems.
(i) The conjugate of the present application uses albumin as a lasting protein. The albumin has the following advantages: albumin 1) is a material consisting of most of the plasma proteins, and is very stable in the human body and exhibits little immunogenicity, 2) is not easily filtered in the glomerulus due to the electrostatic repulsion of the albumin molecule, and 3) has a long cycle of decomposition in the body due to a recycling action mediated by the neonatal Fc receptor (FcRn) of endothelial cells.
(ii) In the conjugate of the present application, the lasting protein is site-specifically bound to a location that does not affect the activity of the polypeptide. Therefore, even if having a conjugate form, the polypeptide is not reduced in activity and efficacy.
(iii) The conjugate of the present application is prepared using an inverse electron-demand Diels-Alder (IEDDA) reaction, which is a type of biorthogonal reaction. Since a chemical functional group involved in the conjugation reaction is not present in the body molecules, the polypeptide-albumin conjugate prepared through the preparation method has an advantage of very high binding stability even when introduced into the body.
(iv) The method of preparing a conjugate of the present application using an IEDDA reaction has a higher preparation speed and a very higher preparation yield compared with the case of using a strain-promoted azide-alkyne cycloaddition (SPAAC) reaction. Accordingly, the preparation method of the present application further has an economic effect of reducing required time and costs.

The functional polypeptide variant-lasting protein conjugate provided in the present application will be described in detail below.

### [Functional polypeptide variant-lasting protein conjugate]

The present application provides a functional polypeptide variant-lasting protein conjugate.

The functional polypeptide variant-lasting protein conjugate of the present application is prepared by site-specifically linking a (i) functional polypeptide variant and a (ii) lasting protein via a (iii) linker.

In one embodiment of the present application, a functional polypeptide variant-lasting protein conjugate represented by Formula 1 below is provided:

[Formula 1] P₁-J₁-A₂-J₂-P₂

Here,
P₁ is a functional polypeptide variant moiety,
A₂ is a second anchor moiety,
P₂ is a lasting protein moiety,
J₁ is a first conjugation moiety, and
J₂ is a second conjugation moiety.
P₁, A₂, P₂, J₁, and J₂ will be described in detail below.

### (i) P₁: functional polypeptide variant moiety

In Formula 1, P₁ is a functional polypeptide variant moiety, derived from a functional polypeptide variant.

Here, the functional polypeptide variant includes one or more non-natural amino acids, wherein the non-natural amino acid includes a first click chemistry functional group.

The non-natural amino acid may include an amino acid including a diene functional group that can make an IEDDA reaction as a first click chemistry functional group. Specifically, the diene functional group may be a tetrazine functional group or a derivative thereof, and/or a triazine functional group or a derivative thereof.

In one embodiment, the non-natural amino acid included in the functional polypeptide variant may be represented by the following structure. 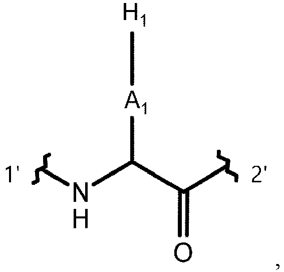 Here,
H₁ is a first click chemistry functional group, which includes a tetrazine functional group or derivative thereof, or a triazine functional group or derivative thereof,
A₁ is a first anchor moiety, which is absent or -A₁₁-A₁₂-,
wherein A₁₁ is absent or C₁₋₅ alkylene, and
wherein A₁₂ is absent or any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p, - [heteroarylene]p-C₁₋₅ alkylene-, -[heteroarylene]p-C₁₋₅ heteroalkylene-, - heteroarylene-C₁₋₅ alkylene-arylene-, -heteroarylene-C₁₋₅ alkylene- heteroarylene-, - heteroarylene-C₁₋₅ heteroalkylene-arylene-, and -heteroarylene-C₁₋₅ heteroalkylene-heteroarylene-, wherein p is an integer of 1 to 3,
wherein the heteroalkylene or the heteroarylene includes one or more heteroatom groups selected from the group consisting of -NH-, -O-, -S-, -O-N=, - S(=O)-, and -S(=O)₂-, or one or more heteroatoms selected from the group consisting of O, N, and S.

The non-natural amino acid is linked with a neighboring amino acid in the amino acid sequence of the functional polypeptide within or at the end of, via 1' and 2' sites, thereby forming the functional polypeptide variant of the present application. That is, at a specific site in the amino acid sequence constituting the functional polypeptide, instead of the originally present amino acid, the non-natural amino acid is substituted or the non-natural amino acid is inserted to constitute the functional polypeptide.

Here, in one embodiment, the first click chemistry functional group may be represented by the following structure (tetrazine): 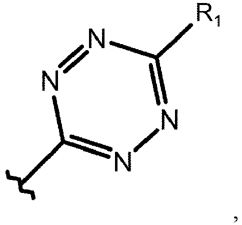
wherein R₁ is any one selected from H, halogen, C₁₋₃ alkyl, C₃₋₆ cycloalkyl, C₃₋₆ heterocycloalkyl, aryl, and heteroaryl,
wherein the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.

Here, in one embodiment, the first click chemistry functional group may be represented by the following structure (triazine): 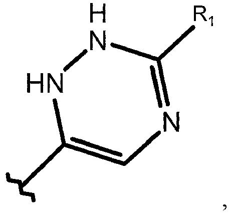
wherein R₁ is any one selected from H, halogen, C₁₋₃ alkyl, C₃₋₆ cycloalkyl, C₃₋₆ heterocycloalkyl, aryl, and heteroaryl,
wherein, the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.

Here, in one embodiment, the first click chemistry functional group may be represented by the following structure (triazine): 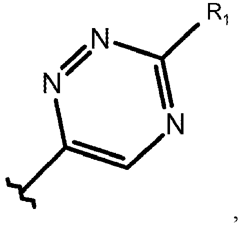
wherein R₁ is any one selected from H, halogen, C₁₋₃ alkyl, C₃₋₆ cycloalkyl, C₃₋₆ heterocycloalkyl, aryl, and heteroaryl,
wherein the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.

### (ii) A₂: second anchor moiety

In Formula 1, A₂₋ is a second anchor moiety. The second anchor moiety disclosed in the specification refers to a linked component between a first conjugation moiety of a polypeptide variant-linker and a second conjugation moiety of a lasting protein-linker.

The second anchor moiety serves to adjust a distance between the functional polypeptide variant moiety and the lasting protein moiety. As long as it is a compound conventionally used for distance adjustment in the art, the second anchor moiety is not particularly limited.

In one embodiment, the second anchor moiety may be a substituted hydrocarbon chain including one or more heteroatoms. Here, the heteroatom is each independently selected from N, O, and S, and the substitution may be a substitution with one or more non-hydrogen substituents, wherein each of the non-hydrogen substituent may independently be any one selected from the group consisting of halogen, C₁₋₃ alkyl, -NH₂, =O, and =S.

In one example, the second anchor moiety is any one selected from substituted or unsubstituted C₁₋₂₀ alkylene, substituted or unsubstituted C₁₋₂₀ heteroalkylene, - substituted or unsubstituted C₁₋₂₀ alkylene-[PEG]n-, -substituted or unsubstituted C₁₋₂₀ heteroalkylene-[PEG]n-, -substituted or unsubstituted C₁₋₂₀ alkylene-[PEG]n-substituted or unsubstituted C₁₋₂₀ alkylene-, -substituted or unsubstituted C₁₋₂₀ alkylene-[PEG]n-substituted or unsubstituted C₁₋₂₀ heteroalkylene-, and -substituted or unsubstituted C₁₋₂₀ heteroalkylene-[PEG]n-substituted or unsubstituted C₁₋₂₀ heteroalkylene-,
wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S,
wherein the substitution is substitution with one or more non-hydrogen substituents, each of the non-hydrogen substituent is any one selected from the group consisting of a halogen, a C₁₋₃ alkyl, -NH₂, =O, and =S, and
n is an integer of 1 to 12.

In one embodiment, in Formula 1, A₂ is -A₂₁-A₂₂-A₂₃-,
wherein A₂₁ is absent or -OC(=O)NH-,
wherein A₂₂ is absent, or any one selected from substituted or unsubstituted C₁₋₁₀ alkylene, substituted or unsubstituted C₁₋₁₀ heteroalkylene, -substituted or unsubstituted C₁₋₁₀ alkylene-[PEG]n-, -substituted or unsubstituted C₁₋₁₀₋ heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S, and
wherein the substitution is substitution with one or more non-hydrogen substituents, wherein each of the non-hydrogen substituent is any one selected from the group consisting of a halogen, a C₁₋₃ alkyl, -NH₂, =O, and =S, and
wherein A₂₃ is absent, or -C(=O)NH- or -C(=O)O-, and
there is no case in which A₂₁, A₂₂, and A₂₃ are all absent at the same time.

In one embodiment, in Formula 1, A₂ is-A₂₁-A₂₂-,
wherein A₂₁ is absent, or -OC(=O)NH-,
wherein A₂₂ is absent, or any one selected from substituted or unsubstituted C₁₋₁₀ alkylene, substituted or unsubstituted C₁₋₁₀ heteroalkylene, -substituted or unsubstituted C₁₋₁₀ alkylene-[PEG]n-, -substituted or unsubstituted C₁₋₁₀₋ heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S, and
the substitution is substitution with one or more non-hydrogen substituents, each of the non-hydrogen substituent is any one selected from the group consisting of a halogen, a C₁₋₃ alkyl, -NH₂, =O, and =S, and
there is no case in which A₂₁ and A₂₂ are both absent at the same time.

Here, the second anchor moiety is derived from a linker.

In one embodiment, the linker of the present application is represented by Formula 2 below. Here, the linker includes a group that can bind with the polypeptide variant (H₂)-anchor (A₂)-a group that can bind with the lasting protein (B).

[Formula 2] H₂-A₂-B

In Formula 2, H₂ is a second click chemistry functional group. Here, the second click chemistry functional group is a dienophile group that can react with a tetrazine or triazine group which is the first click chemistry functional group included in the above-described P₁.

Here, the dienophile group may be, for example, any one selected from a trans-bicyclo[6.1.0]nonene group, a trans-cyclooctene (TCO) group, a methylcyclopropene group, a bicyclo[6.1.0]nonyne group, a cyclooctyne group, a norbornene group, a cyclopentene group, and a styrene group, but the present application is not limited thereto.

Here, in Formula 2, A₂ is the second anchor moiety, which is described above.

In one example, in Formula 2, A₂ may be -A₂₁-A₂₂-A₂₃- or -A₂₁-A₂₂-, and the detailed description thereof is the same as described above.

In Formula 2, B is a thiol reactive group that can react with a thiol residue of the lasting protein.

Here, the thiol reactive group may be, for example, a maleimide group, or a 3-arylpropiolonitrile (APN) group.

In one embodiment, when the thiol reactive group is the maleimide group, B may be represented by the following structure:

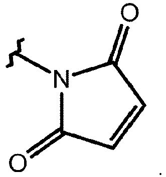

In one embodiment, when the thiol reactive group is the APN group, B may be represented by any one of the following structures: 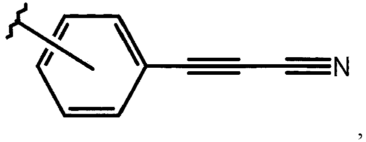 and

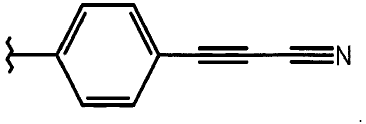

### (iii) P₂: lasting protein moiety

In Formula 1, P₂ is a lasting (long-acting) protein moiety, derived from a lasting protein. For example, when the thiol residue of the lasting protein reacts with the thiol reactive group of a linker to form a conjugate, the part derived from the lasting protein in the conjugate is referred to as the lasting protein moiety.

In one embodiment, the lasting protein may be, for example, any one selected from albumin or a variant thereof, and an albumin-binding domain (ABD) or a variant thereof, but the present application is not limited thereto. In one embodiment, the albumin may be serum albumin or a recombinant human serum albumin. In one embodiment, the albumin may be human serum albumin, bovine serum albumin, or ovalbumin, but the present application is not limited thereto.

### (iv) J₁: First conjugation moiety

In Formula 1, J₁ is a first conjugation moiety, which is formed by the reaction between two materials below:
the first click chemistry functional group (H₁) including the tetrazine functional group or its derivative or the triazine functional group or its derivative of the non-natural amino acid included in the functional polypeptide variant; and
a second click chemistry functional group (H₂), which is the dienophile included in the linker.

In one embodiment, the first conjugation moiety may be represented by any one selected from the following structures. 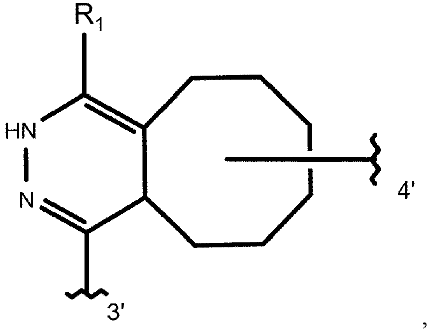 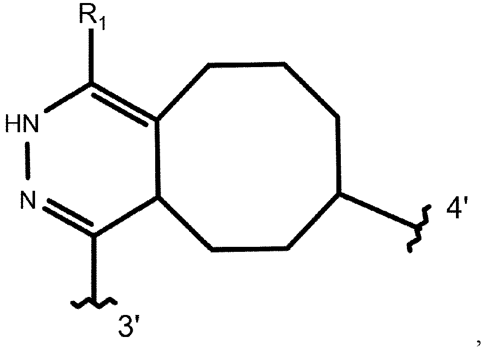 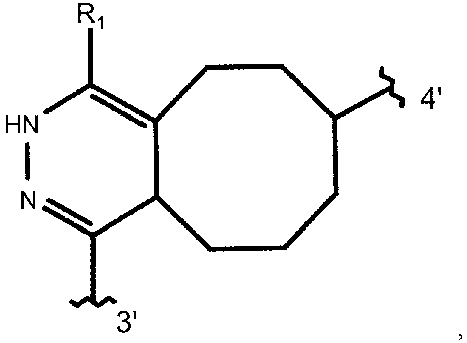 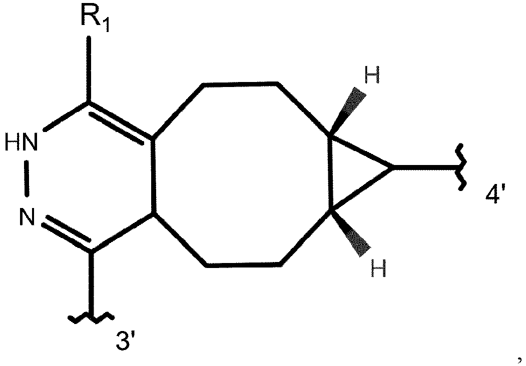 and

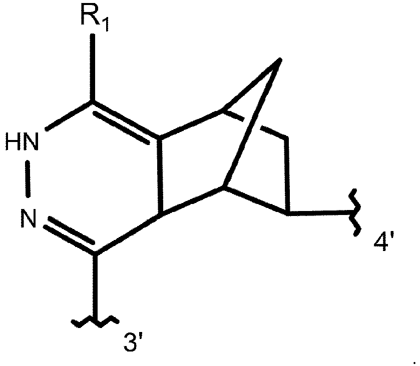

Here, 3' is linked with a functional polypeptide variant moiety. Specifically, here, it is linked with the non-natural amino acid of the functional polypeptide variant.

Here, 4' is linked with the second anchor moiety.

Here, R₁ is any one selected from H, halogen, C₁₋₃ alkyl, C₃₋₆ cycloalkyl, C₃₋₆ heterocycloalkyl, aryl, and heteroaryl.

### (v) J₂: Second conjugation moiety

In Formula 1, J₂ is a second conjugation moiety, which is formed by the reaction between the thiol reactive group of the linker and the thiol residue of the lasting protein.

In one embodiment, the second conjugation moiety may be represented by any one selected from the following structures: 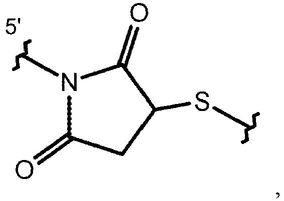 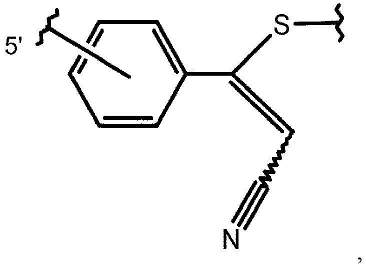 and

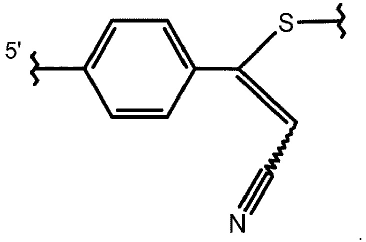

Here, S is derived from the thiol group of the cysteine included in the lasting protein, and
5' is linked with A₂ in Formula 1.

The functional polypeptide variant-lasting protein conjugate of the present application is prepared by reacting a functional polypeptide variant, a linker, and a lasting protein. The functional polypeptide variant, the linker, and the lasting protein will be described in detail below.

### Functional polypeptide variant

In one embodiment of the present application, a functional polypeptide variant is provided.

The functional polypeptide variant of the present application may include one or more non-natural amino acids, and may be site-specifically conjugated with a lasting protein via the non-natural amino acid residues.

In one embodiment, the present application provides a functional polypeptide variant including one non-natural amino acid.

In one embodiment, the present application provides a functional polypeptide variant including two non-natural amino acids.

In one embodiment, the present application provides a functional polypeptide variant including three non-natural amino acids.

The functional polypeptide variant of the present application may be prepared by introducing a non-natural amino acid through tRNA in the process of translating RNA into a polypeptide.

A nucleic acid codon corresponding to a non-natural amino acid is not present in nature. In order to biosynthesize a protein including such a non-natural amino acid in cells, it is necessary to solve the problem in that there is no nucleic acid codon corresponding to the non-natural amino acid. To this end, i) an orthogonal tRNA/synthetase pair having a function of introducing a non-natural amino acid into a sequence by recognizing a stop codon, and ii) a nucleic acid encoding a non-natural amino acid site in the sequence of the urate oxidase variant with a stop codon are used.

To sum up, to prepare the functional polypeptide variant, a method of (1) expressing the polypeptide variant (2) in an expression cell line (3) using foreign suppressor tRNA and foreign tRNA synthetase (4) based on a vector encoding the polypeptide variant is used. The method will be described in detail below.

### Non-natural amino acid

A non-natural amino acid used in the present application may be an amino acid including a diene functional group, which can cause IEDDA reaction. Specifically, the diene functional group may be a tetrazine functional group or its derivative, and/or a triazine functional group or its derivative. In the specification, the tetrazine functional group or its derivative is also expressed as a "tetrazine group," and the triazine functional group or its derivative is also expressed as a "triazine group."

Through the tetrazine or triazine functional group, the following functional polypeptide variant may react IEDDA reaction with another molecule including a dienophile.

In one embodiment, the non-natural amino acid may be represented by Formula 4 below: 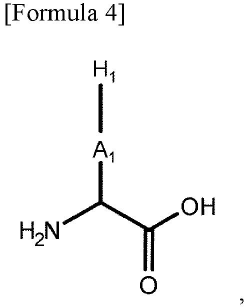 wherein
H₁ is a first click chemistry functional group, wherein the first click chemistry functional group is selected from the tetrazine functional group or its derivative; and a triazine functional group or its derivative, and
A₁ is a first anchor moiety, which is absent or -A₁₁-A₁₂-,
wherein A₁₁ is absent, or a C₁₋₅ alkylene,
wherein A₁₂ is absent, or any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p, - [heteroarylene]p-C₁₋₅ alkylene-, -[heteroarylene]p-C₁₋₅ heteroalkylene-, - heteroarylene-C₁₋₅ alkylene-arylene-, -heteroarylene-C₁₋₅ alkylene-heteroarylene-, - heteroarylene-C₁₋₅ heteroalkylene-arylene-, and -heteroarylene-C₁₋₅ heteroalkylene-heteroarylene-, wherein p is an integer of 1 to 3, and
wherein the heteroalkylene or the heteroarylene includes one or more heteroatom groups selected from the group consisting of -NH-, -O-, -S-, -O-N=, - S(=O)-, and -S(=O)₂-, or one or more heteroatoms selected from the group consisting of O, N, and S.

Here, in one embodiment, the first click chemistry functional group may be represented by the following structure: 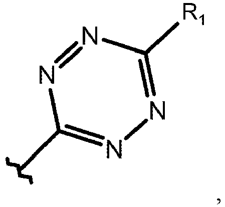
wherein R₁ is any one selected from H, halogen, C₁₋₃ alkyl, C₃₋₆ cycloalkyl, C₃₋₆ heterocycloalkyl, aryl, and heteroaryl,
wherein the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.

Here, in one embodiment, the first click chemistry functional group may be represented by the following structure: 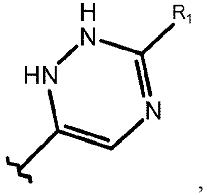
wherein R₁ is any one selected from H, halogen, C₁₋₃ alkyl, C₃₋₆ cycloalkyl, C₃₋₆ heterocycloalkyl, aryl, and heteroaryl,
wherein, the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.

Here, in one embodiment, the first click chemistry functional group may be represented by the following structure: 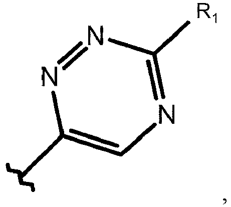
wherein R₁ is any one selected from H, halogen, C₁₋₃ alkyl, C₃₋₆ cycloalkyl, C₃₋₆ heterocycloalkyl, aryl, and heteroaryl,
wherein, the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.

In one embodiment, the non-natural amino acid may be represented by Formula 4-1 below: 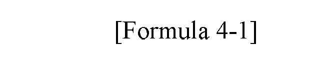 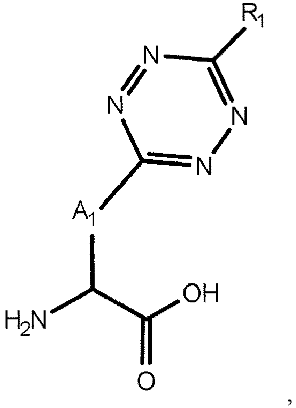 wherein
A₁ is a first anchor moiety, wherein the first anchor moiety is absent or -A₁₁-A₁₂-,
wherein A₁₁ is absent or C₁₋₅ alkylene,
wherein A₁₂ is absent, or any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p, -
[heteroarylene]p-C₁₋₅ alkylene-, -[heteroarylene]p-C₁₋₅ heteroalkylene-, - heteroarylene-C₁₋₅ alkylene-arylene-, -heteroarylene-C₁₋₅ alkylene-heteroarylene-, - heteroarylene-C₁₋₅ heteroalkylene-arylene-, and -heteroarylene-C₁₋₅ heteroalkylene-heteroarylene-, wherein p is an integer of 1 to 3,
R₁ is any one selected from H, halogen, C₁₋₃ alkyl, C₃₋₆ cycloalkyl, C₃₋₆ heterocycloalkyl, aryl and heteroaryl.

In one embodiment, the non-natural amino acid may be represented by Formula 4-2 below: 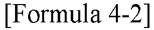 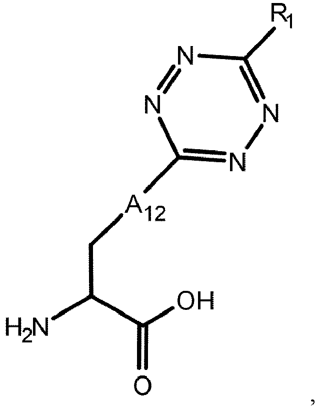 wherein
A₁₂ is absent, or any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p, -[heteroarylene]p-C₁₋₅ alkylene-, -[heteroarylene]p-C₁₋₅ heteroalkylene-, -heteroarylene-C₁₋₅ alkylene-arylene-, -heteroarylene-C₁₋₅ alkylene- heteroarylene-, -heteroarylene-C₁₋₅ heteroalkylene-arylene-, and -heteroarylene-C₁₋₅ heteroalkylene-heteroarylene-, in which p is an integer of 1 to 3, and
R₁ is any one selected from H, halogen, methyl, aryl, and heteroaryl.

In one embodiment, the non-natural amino acid may be 4-(1,2,4,5-tetrazin-3-yl)phenylalanine represented by Formula 4-3 below, or 4-(6-methyl-1,2,4,5-tetrazin-3-yl)phenylalanine represented by Formula 4-4 below.

Hereinafter, the 4-(1,2,4,5-tetrazin-3-yl)phenylalanine is referred to as frTet, and the 4-(6-methyl-1,2,4,5-tetrazin-3-yl)phenylalanine is referred to as Tet_v2.0. 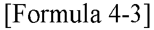 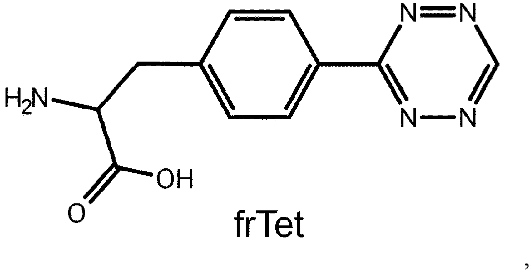 and

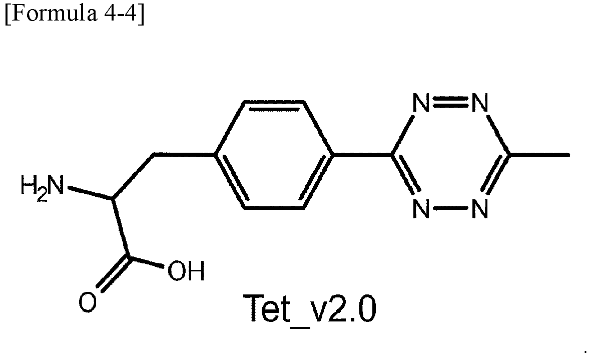

In one embodiment, the non-natural amino acid may be selected from the group consisting of 4-(1,2,3,4-tetrazin-3-yl) phenylalanine (frTet), 4-(6-methyl-1,2,4,5-tetrazin-3-yl)phenylalanine (Tet_ v2.0), 4-(6-methyl-s-tetrazin-3-yl)phenylalanine, 3-(4-(1,2,4-triazin-6-yl)phenyl)-2-aminopropanoic acid, 2-amino-3-(4-(2-(6-methyl-1,2,4,5-tetrazin-3-yl)ethyl)phenyl)propanoic acid, 2-amino-3-(4-(6-phenyl-1,2,4,5-tetrazin-3-yl)phenyl)propanoic acid, 3-(4-((1,2,4,5-tetrazin-3-yl)amino)phenyl)-2-aminopropanoic acid, 3-(4-(2-(1,2,4,5-tetrazin-3-yl)ethyl)phenyl)-2-aminopropanoic acid, 3-(4-((1,2,4,5-tetrazin-3-yl)thio)phenyl)-2-aminopropanoic acid, 2-amino-3-(4-((6-methyl-1,2,4,5-tetrazin-3-yl)thio)phenyl)propanoic acid, 3-(4-((1,2,4,5-tetrazin-3-yl)oxy)phenyl)-2-aminopropanoic acid, 2-amino-3-(4-((6-methyl-1,2,4,5-tetrazin-3-yl)oxy)phenyl)propanoic acid, 3-(4'-(1,2,4,5-tetrazin-3-yl)-[1,1'-biphenyl]-4-yl)-2-aminopropanoic acid, 2-amino-3-(4'-(6-methyl-1,2,4,5-tetrazin-3-yl)-[1,1'-biphenyl]-4-yl)propanoic acid, 2-amino-3-(6-(6-(pyridin-2-yl)-1,2,4,5-tetrazin-3-yl)pyridin-3-yl)propanoic acid, 3-(4-(1,2,4,5-tetrazin-3-yl)phenyl)-2-aminopropanoic acid, and 2-amino-3-(4-(6-methyl-1,2,4,5-tetrazin-3-yl)phenyl)propanoic acid.

### Functional polypeptide variant including non-natural amino acid

In one embodiment of the present application, a functional polypeptide variant including a non-natural amino acid is disclosed.

### Functional polypeptide variant and functional polypeptide

Functional polypeptide variant provided in the present application includes one or more non-natural amino acids, and here, the non-natural amino acid is the same as described above.

The functional polypeptide variant provided in the present application may consist of 10 to 500 amino acids, and have one or more functions. Here, the function means a desired effect that can be achieved by a polypeptide, and includes the original function of a peptide that does not include non-natural amino acid. Here, the functional polypeptide variant may have a desired function as a single polypeptide, or by assembling two or more sub-unit polypeptides.

Here, the function may be, for example, treatment or alleviation of a certain disease, the delay of the onset of a certain disease, recovery, the diagnosis of a certain disease, the generation of fluorescence, the interaction with a different material, a structural change, or the promotion or inhibition of the production of a different material, but the present application is not limited thereto.

That is, the functional polypeptide variant in the present application is a polypeptide which includes one or more non-natural amino acids, consists of 10 to 500 amino acids, and exhibits one or more desired functions.

In one embodiment of the present application, the functional polypeptide variant may be a polypeptide in which one or more standard amino acids included in the functional polypeptide are substituted with non-natural amino acid(s), or in which one or more non-natural amino acids are inserted into the functional polypeptide.

### Selection of non-natural amino acid substitution position

As described above, in a functional polypeptide variant, one or more amino acids in the original amino acid sequence of a polypeptide are substituted with non-natural amino acid(s), or one or more non-natural amino acids are inserted.

Here, a position in the polypeptide for substitution with non-natural amino acid may be selected considering the following factors.

First, the activity of a desired polypeptide is considered. Since the structure and function of the original functional polypeptide should be affected as little as possible, amino acids that play an important role in the activity and structure of the functional polypeptide may not be substituted with a non-natural amino acid.

In addition, solvent accessibility is considered. Since the non-natural amino acid should be bonded with a linker during the preparation of functional polypeptide variant-lasting protein conjugate, it is advantageous to substitute an amino acid at a position with relatively high solvent accessibility in the three-dimensional structure of the functional polypeptide.

That is, various methods may be used to minimize an effect on the structure and function of the functional polypeptide and select a site with high solvent accessibility. For example, through molecular modeling calculation, a candidate position which exhibits similar intrinsic atomic energy to that of the functional polypeptide and has high solvent accessibility may be selected.

In one embodiment, to prepare a functional polypeptide variant, a position in the functional polypeptide sequence, which is substituted with a non-natural amino acid, may be determined with reference to a molecular modeling simulation result. Specifically, the molecular modeling simulation result may be the scoring result of the Rosetta molecular modeling package.

### Structure of non-natural amino acid in functional polypeptide variant

Non-natural amino acid included in a functional polypeptide variant may be represented by the following structure: 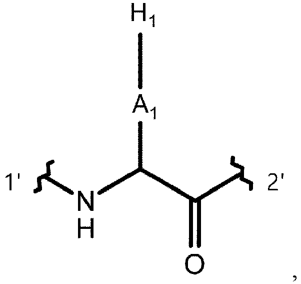 wherein
H₁ includes a first click chemistry functional group, which includes a tetrazine functional group or derivative thereof, or a triazine functional group or derivative thereof,
A₁ is a first anchor moiety, which is absent or -A₁₁-A₁₂-,
wherein A₁₁ is absent, or C₁₋₅ alkylene, and
wherein A₁₂ is absent, or any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p, - [heteroarylene]p-C₁₋₅ alkylene-, -[heteroarylene]p-C₁₋₅ heteroalkylene-,- heteroarylene-C₁₋₅ alkylene-arylene-, -heteroarylene-C₁₋₅ alkylene-heteroarylene-, - heteroarylene-C₁₋₅ heteroalkylene-arylene-, and -heteroarylene-C₁₋₅ heteroalkylene-heteroarylene-, wherein p is an integer of 1 to 3, and
wherein the heteroalkylene or the heteroarylene includes one or more heteroatom groups selected from the group consisting of -NH-, -O-, -S-, -O-N=, - S(=O)-, and -S(=O)₂-, or one or more heteroatoms selected from the group consisting of O, N, and S.

The non-natural amino acid is linked to a neighboring amino acid in the amino acid sequence or at the end of the functional polypeptide at 1' and 2', thereby forming the functional polypeptide variant of the present application. That is, at a specific position of the amino acid sequence constituting the functional polypeptide, instead of the originally existing standard amino acid, the non-natural amino acid is substituted or the non-natural amino acid is inserted, thereby forming the functional polypeptide variant.

Here, in one embodiment, the first click chemistry functional group may be represented by the following structure: 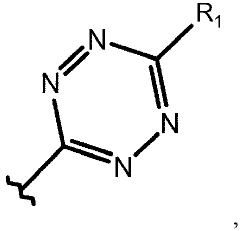
wherein R₁ is any one selected from H, halogen, C₁₋₃ alkyl, C₃₋₆ cycloalkyl, C₃₋₆ heterocycloalkyl, aryl, and heteroaryl,
wherein the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.

Here, in one embodiment, the first click chemistry functional group may be represented by the following structure: 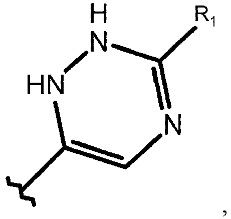
wherein R₁ is any one selected from H, halogen, C₁₋₃ alkyl, C₃₋₆ cycloalkyl, C₃₋₆ heterocycloalkyl, aryl, and heteroaryl, wherein
the heterocycloalkyl or heteroaryl includes one or more heteroatom groups selected from the group consisting of -NH-, -O-, -S-, -O-N=, -S(=O)-, and -S(=O)₂-, or one or more heteroatoms selected from the group consisting of O, N, and S.

Here, in one embodiment, the first click chemistry functional group may be represented by the following structure: 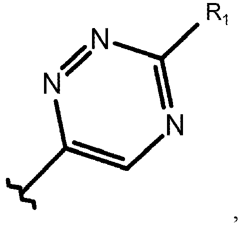
wherein R₁ is any one selected from H, halogen, C₁₋₃ alkyl, C₃₋₆ cycloalkyl, C₃₋₆ heterocycloalkyl, aryl, and heteroaryl, wherein
the heterocycloalkyl or heteroaryl includes one or more heteroatom groups selected from the group consisting of -NH-, -O-, -S-, -O-N=, -S(=O)-, and -S(=O)₂-, or one or more heteroatoms selected from the group consisting of O, N, and S.

In one embodiment, a non-natural amino acid included in functional polypeptide variant may be represented by the following structure: 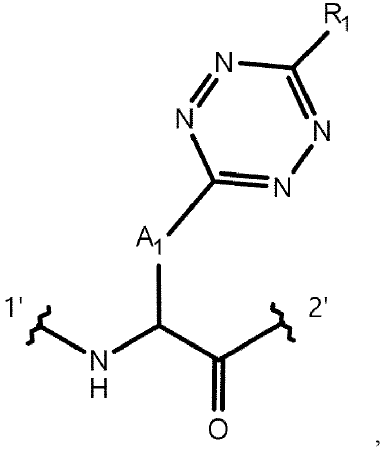 wherein
A₁ is a first anchor moiety, which is absent or -A₁₁-A₁₂-,
wherein A₁₁ is absent, or C₁₋₅ alkylene,
wherein A₁₂ is absent, or any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p, - [heteroarylene]p-C₁₋₅ alkylene-, -[heteroarylene]p-C₁₋₅ heteroalkylene-, - heteroarylene-C₁₋₅ alkylene-arylene-, -heteroarylene-C₁₋₅ alkylene- heteroarylene-, - heteroarylene-C₁₋₅ heteroalkylene-arylene-, and -heteroarylene-C₁₋₅ heteroalkylene-heteroarylene-, in which p is an integer of 1 to 3,
R₁ is any one selected from H, a halogen, C₁₋₃ alkyl, C₃₋₆ cycloalkyl, C₃₋₆ heterocycloalkyl, aryl and heteroaryl.

In one embodiment, the non-natural amino acid included in functional polypeptide variant may be represented by the following structure: 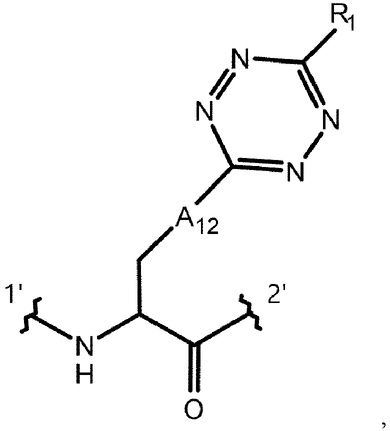 wherein
A₁₂ is absent, or any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p, -[heteroarylene]p-C₁₋₅ alkylene-, -[heteroarylene]p-C₁₋₅ heteroalkylene-, -heteroarylene-C₁₋₅ alkylene-arylene-, -heteroarylene-C₁₋₅ alkylene-heteroarylene-, -heteroarylene-C₁₋₅ heteroalkylene-arylene-, and -heteroarylene-C₁₋₅ heteroalkylene- heteroarylene-, in which p is an integer of 1 to 3,
R₁ is any one selected from H, halogen, methyl, aryl, and heteroaryl.

In one embodiment, a non-natural amino acid included in a functional polypeptide variant may be represented by the following structure:

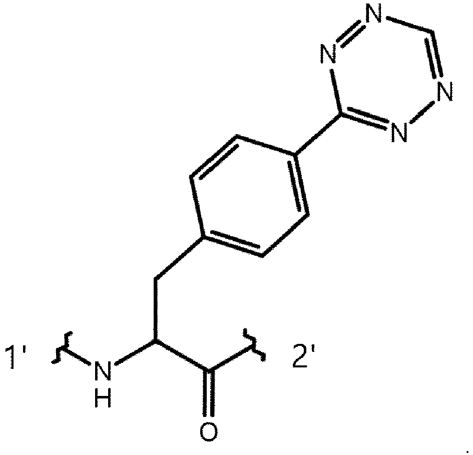

In one embodiment, a non-natural amino acid included in a functional polypeptide variant may be represented by the following structure:

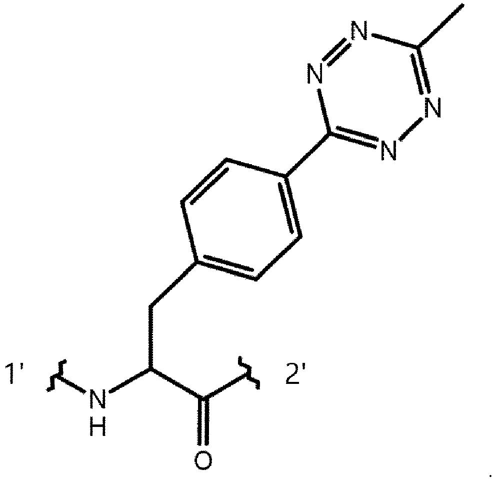

### Specific example of functional polypeptide variant of the present application

In one embodiment, a functional polypeptide variant of the present application may be a glucagon-like peptide-1 (GLP1) variant, a human growth hormone (hGH) variant, a green fluorescent protein (GFP) variant, or a urate oxidase (Uox) subunit variant.

### GLPI variant

In one embodiment of the present application, the functional polypeptide variant may be a GLP1 variant.

In one embodiment, in the GLP1 variant, one or more amino acids included in glucagon-like peptide-1 (GLP1) may be substituted with the above-described non-natural amino acid(s) or the non-natural amino acid(s) may be inserted thereinto.

For example, the GLP1 variant may be a polypeptide having any one amino acid sequence selected from SEQ ID NOs: 1 to 26.

The GLP1 variant will be described in detail in [GLP1 variant-HSA conjugate] below.

### hGH variant

In one embodiment of the present application, the functional polypeptide variant may be an hGH variant. Here, in the hGH variant, one or more amino acids included in the hGH variant may be substituted with the above-described non-natural amino acid(s) or the non-natural amino acid(s) may be inserted thereinto.

For example, the hGH variant may be a polypeptide including any one amino acid sequence selected from SEQ ID NOs: 28 to 50.

The hGH variant will be described in detail in [hGH variant-HSA conjugate] below.

### GFP variant

In one embodiment of the present application, the functional polypeptide variant may be a GFP variant. Here, in the GFP variant, one or more amino acids included in the GFP variant may be substituted with the above-described non-natural amino acid(s) or the non-natural amino acid(s) may be inserted thereinto.

In one embodiment, the GFP variant may be a super folder GFP (sfGFP) variant. Here, in the sfGFP variant, one or more amino acids included in the sfGFP variant may be substituted with the above-described non-natural amino acid(s) or the non-natural amino acid(s) may be inserted thereinto.

For example, the sfGFP variant may be a polypeptide including any one amino acid sequence selected from SEQ ID NOs: 52 to 75.

The sfGFP variant will be described in detail in [sfGFP variant-HSA conjugate] below.

### Uox subunit variant

In one embodiment of the present application, the functional polypeptide variant may be a urate oxidase (Uox) subunit variant. Here, in the Uox subunit variant, one or more standard amino acids included in the Uox subunit may be substituted with the above-described non-natural amino acid(s) or the non-natural amino acid(s) may be inserted thereinto.

In one embodiment, in the Uox subunit variant, compared with a wild-type Uox subunit, one or more standard amino acid(s) in the sequence may be substituted with non-natural amino acid(s). For example, the wild-type Uox subunit may be a Uox subunit derived from a microorganism selected from *Aspergillus Flavus, Arthrobacter globiformis*, and *Candidas Utilis.*

### Method of preparing functional polypeptide variant

### Summary of method of preparing functional polypeptide variant

The present application discloses a method of preparing a functional polypeptide variant. In the method of preparing a functional polypeptide variant, the following components are involved: a cell line to express the functional polypeptide variant; foreign suppressor tRNA (foreign-derived suppressor tRNA) recognizing a specific stop codon; foreign tRNA synthetase (foreign-derived tRNA synthetase); and a vector encoding the functional polypeptide variant in which the non-natural amino acid is encoded by using the stop codon.

Here, the foreign suppressor tRNA and the foreign tRNA synthetase are not intrinsic suppressor tRNA and tRNA synthetase of the expression cell line, but derived from cells other than the expression cell line. Accordingly, the foreign suppressor tRNA is characterized by not reacting with the intrinsic tRNA synthetase of the expression cell line. The foreign tRNA synthetase i) only reacts with the foreign suppressor tRNA, and ii) exhibits activity only on non-natural amino acid to be included in the functional polypeptide variant. As a result, when the foreign tRNA synthetase is used, the non-natural amino acid can be specifically linked to the foreign tRNA synthetase, and through this, the non-natural amino acid can be introduced into peptide sequence.

The method of preparing a functional polypeptide variant is a method of expressing the functional polypeptide variant in the cell line using the foreign suppressor tRNA and foreign tRNA synthetase, based on the vector encoding the functional polypeptide variant. In the method of preparing the functional polypeptide variant, the order of each process is not particularly limited, and additional processes may be included as needed if the functional polypeptide variant can be expressed well in the cell line .

### Functional polypeptide variant expression cell line

The method of preparing the functional polypeptide variant is characterized by obtaining the functional polypeptide variant by expressing the functional polypeptide variant in cell line. The functional polypeptide variant expression cell line is not particularly limited as long as it can produce the functional polypeptide variant. However, when a release factor recognizing stop codon in the cell line properly functions, the release factor competitively acts with the foreign tRNA to decrease a yield. Accordingly, it is preferable to use a cell line in which the release factor recognizing stop codon is inactivated.

In one embodiment, a cell line expressing the functional polypeptide variant may be selected from the following:
*Escherichia* sp.; *Erwinia* sp.; *Serratia* sp.; *Providencia* sp.; *Corynebacterium* sp.; *Pseudomonas* sp.; *Leptospira* sp.; *Salmonellar* sp.; *Brevibacterium* sp.; *Hyphomonas* sp.; *Chromobactorium* sp.; *Norcardia* sp.; *Fungi;* and *Yeast.*

In one embodiment, the cell line may be a cell line in which the release factor recognizing stop codon to terminate translation is inactivated. Specifically, the stop codon may be selected from an amber codon (5'-UAG-3'), an ocher codon (5'-UAA-3'), and an opal codon (5'-UGA-3').

### Foreign suppressor tRNA

The foreign suppressor tRNA is tRNA serving to recognize the specific stop codon, and does not react with the intrinsic tRNA synthetase of the expression cell line. The foreign suppressor tRNA specifically reacts with the foreign tRNA synthetase, and the foreign tRNA synthetase serves to link the non-natural amino acid to the foreign suppressor tRNA. As a result, the foreign suppressor tRNA can recognize the specific stop codon, and can introduce the non-natural amino at the corresponding position.

In one embodiment, the suppressor tRNA may recognize one that is selected from the amber codon (5'-UAG-3'), the ocher codon (5'-UAA-3'), and the opal codon (5'-UGA-3'). Preferably, the suppressor tRNA may recognize the amber codon. For example, the suppressor tRNA may be *Methanococcus jannaschii-derived* suppressor tRNA (MjtRNA^{Tyr}_{CUA}) (Yang et.al, Temporal Control of Efficient In Vivo Bioconjugation Using a Genetically Encoded Tetrazine-Mediated Inverse-Electron-Demand Diels-Alder Reaction, Bioconjugate Chemistry, 2020, 2456-2464).

### Foreign tRNA synthetase

The foreign tRNA synthetase selectively reacts with a specific non-natural amino acid, and serves to link the specific non-natural amino acid to the foreign suppressor tRNA. The foreign tRNA synthetase does not react with the intrinsic suppressor tRNA of the expression cell line, and specifically reacts only with the foreign suppressor tRNA. In one embodiment, the tRNA synthetase may have a ability to link the non-natural amino acid including tetrazine or its derivative and/or triazine or its derivative to the foreign suppressor tRNA. In one embodiment, the tRNA synthetase may be *Methanococcus jannaschii-derived* tyrosyl-tRNA synthetase (MjTyrRS) (Yang et.al, Temporal Control of Efficient In Vivo Bioconjugation Using a Genetically Encoded Tetrazine-Mediated Inverse-Electron-Demand Diels-Alder Reaction, Bioconjugate Chemistry, 2020, 2456-2464). Preferably, the tRNA synthetase may be a C11 variant of the MjTyrRS.

### Orthogonal tRNA/synthetase pair

In the specification, 1) foreign suppressor tRNA specifically reacting only with a foreign tRNA synthetase, and 2) the foreign tRNA synthetase collectively refer to an orthogonal tRNA/synthetase pair. In the method of preparing a functional polypeptide variant disclosed herein, it is important to express the orthogonal tRNA/synthetase pair in the expression cell line, and the method is not particularly limited as long as it can achieve the above-described purpose. In one embodiment, the method of preparing a functional polypeptide variant includes transforming the cell line with a vector capable of expressing the orthogonal tRNA/synthetase pair. Specifically, the vector that can express the orthogonal tRNA/synthetase pair may be pDule_C11 suggested by Yang et.al (Temporal Control of Efficient In Vivo Bioconjugation Using a Genetically Encoded Tetrazine-Mediated Inverse-Electron-Demand Diels-Alder Reaction, Bioconjugate Chemistry, 2020, 2456-2464).

### Vector encoding functional polypeptide variant

The method of preparing a functional polypeptide variant includes transfecting a vector encoding the functional polypeptide variant into an expression cell line. Hereinafter, this will be described in further detail in "Vector encoding functional polypeptide variant."

### Example of method of preparing functional polypeptide variant

In one embodiment, the method of preparing a functional polypeptide variant includes the following process:
preparing a cell line including a vector capable of expressing orthogonal tRNA/synthetase pair and a vector encoding a functional polypeptide variant,
wherein the orthogonal tRNA/synthetase pair includes a foreign suppressor tRNA and foreign tRNA synthetase,
the functional polypeptide variant includes one or more non-natural amino acids, wherein the non-natural amino acid includes a tetrazine group or derivative group thereof, and
in the vector encoding the functional polypeptide variant, a codon corresponding to the non-natural amino acid is the amber codon (5'-UAG-3'); and
incubating the cell line in a medium containing the non-natural amino acid comprising the tetrazine group or derivative group thereof,
wherein, the foreign suppressor tRNA is capable of recognizing the amber codon (5'-UAG-3'),
the foreign tRNA synthetase is capable of linking the non-natural amino acid to the foreign suppressor tRNA, and
thereby, the cell line expresses the peptide comprising the non-natural amino acid which is located at the position corresponding to the amber codon when the vector encoding the functional polypeptide variant is expressed.

### Vector encoding functional polypeptide variant

The present application discloses a vector encoding a functional polypeptide variant. The vector encoding the functional polypeptide variant is characterized in that a non-natural amino acid in the functional polypeptide variant sequence is encoded using a stop codon. In one embodiment, in the vector encoding the functional polypeptide variant, among the functional polypeptide variant sequence, standard amino acids may be encoded by codons corresponding to the standard amino acids found in nature, and a non-natural amino acid may be encoded by a stop codon. For example, the stop codon may be selected from the amber codon (5'-UAG-3'), the ocher codon (5'-UAA-3'), and the opal codon (5'-UGA-3'). In another example, the stop codon may be selected from 5'-TAG-3', 5'-TAA-3', and 5'-TGA-3'. In one embodiment, the vector encoding the functional polypeptide variant may be codon-optimized for the expression cell line. For example, the vector encoding the functional polypeptide variant may be codon-optimized for *E. coli.*

### Lasting protein

As described above, in the present application, for an increase in half-life and a decrease in immunogenicity of the functional polypeptide variant, the lasting (long-acting) protein is conjugated to the functional polypeptide variant.

Here, a specific amino acid residue, for example, an amine group or thiol group, of the lasting protein may be used.

In one embodiment, a thiol residue of cysteine included in the lasting protein is used. That is, the thiol residue reacts with the thiol reactive group at one end of the linker.

The long-acting protein may be, for example, albumin or a variant thereof, or an albumin-binding domain or a variant thereof, but the present application is not limited thereto.

### Albumin or variant thereof

In one embodiment, the lasting protein may be albumin or variant thereof.

Albumin is a simple protein that is widely distributed in body fluid, and serves as a transport protein that binds to various molecules for transport thereof. A representative example of albumin is serum albumin.

In one embodiment, the albumin of the present application may be mammalian albumin, for example, serum albumin. For example, the albumin may be any one selected from human serum albumin (HSA), bovine serum albumin (BSA), ovalbumin, other vertebrate albumin, and variants thereof. They may be a wild-type or recombinant form.

As an exemplary example, the albumin is wild-type or recombinant human serum albumin. The human serum albumin has a long half-life of two weeks or more. This is because such albumin 1) is not easily filtered in the glomerulus due to the electrostatic repulsion of the albumin molecule, and 2) has a long cycle of decomposition in the body due to a recycling action mediated by the neonatal Fc receptor (FcRn) of endothelial cells.

In one embodiment, the human serum albumin or its variant may include the following sequence.












 (SEQ ID NO: 76)

In one embodiment, the human serum albumin or its variant may include any one sequence selected from the following sequences: 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
 
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQMSTPTLVEVSRNLGKVGSKCCKHPEAKRMPCAEDYLSV
VLNQLCVLHEKTPVSDRVTKCCTESLVNRRPCFSALEVDETYVPKEFNAETF
TFHADICTLSEKERQIKKQTALVELVKHKPKATKEQLKAVMDDFAAFVEKC
CKADDKETCFAEEGKKLVAASQAALGL (SEQ ID NO: 77);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSAPTLVEVSRNLGKVGSKCCKHPEAKRMPCAEDYLSVV
LNQLCVLHEKTPVSDRVTKCCTESLVNRRPCFSALEVDETYVPKEFNAETFT
FHADICTLSEKERQIKKQTALVELVKHKPKATKEQLKAVMDDFAAFVEKCC
KADDKETCFAEEGKKLVAASQAALGL (SEQ ID NO: 78);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
 
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNARTFT
FHADICTLSEKERQIKKQTALVELVKHKPKATKEQLKAVMDDFAAFVEKCC
KADDKETCFAEEGKKLVAASQAALGL (SEQ ID NO: 79);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAGTFT
FHADICTLSEKERQIKKQTALVELVKHKPKATKEQLKAVMDDFAAFVEKCC
KADDKETCFAEEGKKLVAASQAALGL (SEQ ID NO: 80);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
 
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFT
FHADICTLSEKERQIKKQTALVELVKHKPKATKEQLKAAMDDFAAFVEKCC
KADDKETCFAEEGKKLVAASQAALGL (SEQ ID NO: 81);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFT
FHADICTLSEKERQIKKQTALVELVKHKPKATKEQLKAVMDDFAAFVEKCC
KADDKETCFAEEGYKLVAASQAALGL (SEQ ID NO: 82);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
 
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLIEVSRNLGKVGSKCCKHPEAKRMPCAEDYLSVV
LNQLCVLHEKTPVSDRVTKCCTESLVNRRPCFSALEVDETYVPKEFNAETFT
FHADICTLSEKERQIKKQTALVELVKHKPKATKEQLKAVMDDFAAFVEKCC
KADDKETCFAEEGKKLVAASQAALGL (SEQ ID NO: 83);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRDLGKVGSKCCKHPEAKRMPCAEDYLSVV
LNQLCVLHEKTPVSDRVTKCCTESLVNRRPCFSALEVDETYVPKEFNAETFT
FHADICTLSEKERQIKKQTALVELVKHKPKATKEQLKAVMDDFAAFVEKCC
KADDKETCFAEEGKKLVAASQAALGL (SEQ ID NO: 84);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
 
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCVEDYLSVV
LNQLCVLHEKTPVSDRVTKCCTESLVNRRPCFSALEVDETYVPKEFNAETFT
FHADICTLSEKERQIKKQTALVELVKHKPKATKEQLKAVMDDFAAFVEKCC
KADDKETCFAEEGKKLVAASQAALGL (SEQ ID NO: 85);
 
DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP
SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCAEDYLSVV
LNQLCVLHEKMPVSDRVTKCCTESLVNRRPCFSALEVDETYVPKEFNAETFT
FHADICTLSEKERQIKKQTALVELVKHKPKATKEQLKAVMDDFAAFVEKCC
KADDKETCFAEEGKKLVAASQAALGL (SEQ ID NO: 86);
 and

DAHKSEVAHRFKDLGEENFKALVLIAFAQYLQQCPFEDHVKLVNEV
TEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPY
FYAPELLFFAKRYKAAFTECCQAADKAACLLPKLDELRDEGKASSAKQRLK
CASLQKFGERAFKAWAVARLSQRFPKAEFAEVSKLVTDLTKVHTECCHGDL
LECADDRADLAKYICENQDSISSKLKECCEKPLLEKSHCIAEVENDEMPADLP

SLAADFVESKDVCKNYAEAKDVFLGMFLYEYARRHPDYSVVLLLRLAKTY
ETTLEKCCAAADPHECYAKVFDEFKPLVEEPQNLIKQNCELFEQLGEYKFQN
ALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFT
FHADICTLSEKERQIKKQTALVELVKHKPKATKEQLKAVMDDFTAFVEKCC
KADDKETCFAEEGKKLVAASQAALGL (SEQ ID NO: 87).

When the lasting protein is human serum albumin, the lasting protein-linker complex is formed by reaction of thiol residue of cysteine included in the human serum albumin and thiol reactive group at one end of the linker. Here, the structure formed by the reaction of the thiol residue and the thiol reactive group is represented by J₂ (second conjugation moiety) in Formula 1 of the present application.

In one embodiment, in Formula 1 of the present application, J₂ may be a structure formed by the reaction of the thiol reactive group and the thiol residue of cysteine 34 (Cys 34) of the sequence of SEQ ID NO: 76.

### Albumin-binding domain or a variant thereof

In one embodiment, the long-acting protein of the present application may be an albumin-binding domain (ABD) or a variant thereof. The albumin-binding domain interacts (non-covalently) with albumin. Such the albumin-binding domain is conjugated to a protein therapeutic agent or a polypeptide therapeutic agent, thereby improving the pharmacokinetics of the protein or polypeptide therapeutic agent in the body.

In addition, a conjugate in which the functional polypeptide variant of the present application and albumin are linked via the albumin-binding domain (ABD) may be obtained. Here, the functional polypeptide variant and albumin are indirectly linked (e.g., interaction through a non-covalent bond) via the albumin-binding domain (ABD) to form a conjugate structure.

Here, the conjugate of the present application includes both (a) the case consisting of the functional polypeptide variant, and the albumin-binding domain (ABD), and (b) the case consisting of the functional polypeptide variant, the albumin-binding domain (ABD), and the albumin.

In one embodiment, the lasting protein may be the albumin-binding domain (ABD) or variant thereof.

The albumin-binding domain is a three-helical protein domain found in various surface proteins of gram-positive bacteria.

Here, in one embodiment, the albumin-binding domain variant may be a peptide represented by the amino acid sequence of SEQ ID NO: 88.

MRGSHHHHHHGCLAEAKVLANRELDKYGVSDFYKRLINKAKTVEG
VEALKLHILAALPQA (SEQ ID NO: 88)

The lasting protein-linker complex (a) is formed by the reaction of a thiol residue of cysteine included in the albumin-binding domain variant and a thiol reactive group of one end of a linker. Here, the structure formed by the reaction of the thiol residue of the cysteine included in the albumin-binding domain variant and the thiol reactive group is expressed as J₂ (second conjugation moiety) of Formula 1 in the present application.

In one embodiment, in Formula 1 of the present application, J₂ may be a structure formed by the reaction of a thiol reactive group and a thiol residue of cysteine 12 (Cys 12) of the sequence of SEQ ID NO: 88.

### Linker

As described above, in the preparation of a conjugate of the present application, a functional polypeptide variant and a long-acting protein are linked using a linker, and thus a second anchor moiety (A₂) is derived from the linker.

The linker has a second click chemistry functional group, which is a dienophile capable of reacting an IEDDA reaction with a tetrazine group or triazine group at one end, and a thiol reactive group capable of reacting with thiol residue at the other end.

Here, the linker may include a second anchor moiety between the second click chemistry functional group and the thiol reactive group. The second anchor moiety serves to adjust the length (distance) between the second click chemistry functional group and the thiol reactive group.

In one embodiment, the second anchor moiety may be a substituted hydrocarbon chain including one or more heteroatoms, and wherein the heteroatoms may be selected from N, O, and S, wherein the substitution is performed substitution with one or more non-hydrogen substituents, and the non-hydrogen substituent may be any one selected from the group consisting of a halogen, C₁₋₃ alkyl, -NH₂, =O, and =S.

In one embodiment, the linker is represented by Formula 2 below. The linker includes a group (H₂) capable of binding to a polypeptide variant-anchor (A₂)-a group (B) capable of binding to a lasting protein:

[Formula 2] H₂-A₂-B,

In Formula 2,
H₂ is a second click chemistry functional group. Here, the second click chemistry functional group is a dienophile group capable of having an IEDDA reaction with a tetrazine or triazine group, which is a first click chemistry functional group having a non-natural amino acid of the functional polypeptide variant.

Here, the dienophile group may be, for example, any one of a transbicyclo[6.1.0]nonene group, a trans-cyclooctene (TCO) group, a methylcyclopropene group, a bicyclo[6.1.0]nonyne group, a cyclooctyne group, a norbornene group, a cyclopentene group, and a styrene group, but the present application is not limited thereto.

In Formula 2, A₂ is a second anchor moiety, which is any one selected from substituted or unsubstituted C₁₋₂₀ alkylene, substituted or unsubstituted C₁₋₂₀ heteroalkylene, -substituted or unsubstituted C₁₋₂₀ alkylene-[PEG]n-, -substituted or unsubstituted C₁₋₂₀ heteroalkylene-[PEG]n-, -substituted or unsubstituted C₁₋₂₀ alkylene-[PEG]n-substituted or unsubstituted C₁₋₂₀ alkylene-, -substituted or unsubstituted C₁₋₂₀ alkylene-[PEG]n-substituted or unsubstituted C₁₋₂₀ heteroalkylene-, and -substituted or unsubstituted C₁₋₂₀ heteroalkylene-[PEG]n-substituted or unsubstituted C₁₋₂₀ heteroalkylene-,

Here, the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S,
the substitution is performed substitution with one or more non-hydrogen substituents, and the non-hydrogen substituent is any one selected from the group consisting of halogen, C₁₋₃ alkyl, -NH₂, =O, and =S, and
n is an integer of 1 to 12.

In Formula 2, B is a thiol reactive group that can react with a thiol residue of a lasting protein.

Here, the thiol reactive group may be, for example, a maleimide group, or a 3-arylpropiolonitrile (APN) group.

In one embodiment, the dienophile group reacts with a tetrazine or triazine group included in the functional polypeptide variant to form J₁ (first conjugation moiety).

In one embodiment, the thiol reactive group reacts with a thiol residue of cysteine included in a lasting protein to form J₂ (second conjugation moiety).

In one embodiment, the second anchor moiety may be substituted C₁₋₅ heteroalkylene. In one embodiment, the second anchor moiety may be -substituted C₁₋₅ heteroalkylene-[PEG]n-. In one embodiment, the second anchor moiety may be -substituted C₁₋₁₀ heteroalkylene-[PEG]n-substituted C₁₋₅ heteroalkylene-. Here, the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S. Here, the substitution is substitution with one or more non-hydrogen substituents, and the non-hydrogen substituent is any one selected from the group consisting of halogen, C₁₋₃ alkyl, -NH₂, =O, and =S. Here, in one embodiment, n may be an integer of 1 to 8.

In one embodiment, the linker of the present application may be represented by Formula 2-1 below:

[Formula 2-1] H₂-A₂₁-A₂₂-A₂₃-B

Here,
H₂ is a second click chemistry functional group, which is a dienophile group reacting with tetrazine or an analogue thereof,
B is a thiol reactive group, which is a maleimide group or an APN group, and
-A₂₁-A₂₂-A₂₃- is a second anchor moiety.
Here, A₂₁ is absent, or -OC(=O)NH-;
A₂₂ is absent, or any one selected from the group consisting of a substituted or unsubstituted C₁₋₁₀ alkylene, a substituted or unsubstituted C₁₋₁₀ heteroalkylene, - substituted or unsubstituted C₁₋₁₀ alkylene-[PEG]n-, -substituted or unsubstituted C₁₋₁₀-heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S, and
wherein the substitution is substitution with one or more non-hydrogen substituents, and the non-hydrogen substituent is any one selected from the group consisting of a halogen, C₁₋₃ alkyl, -NH₂, =O, and =S;
A₂₃ is absent, or -C(=O)NH-, or -C(=O)O-; and
there is no case in which A₂₁, A₂₂, and A₂₃ are all absent at the same time.

In one embodiment, the linker of the present application may be represented by Formula 2-2 below:

[Formula 2-2] H₂-A₂₁-A₂₂-B

Here,
H₂ is a second click chemistry functional group, which is a dienophile group reacting with tetrazine or an analogue thereof,
B is a thiol reactive group, and
-A₂₁-A₂₂- is a second anchor moiety.
Here, A₂₁ is absent, or -OC(=O)NH-;
A₂₂ is absent, or any one selected from a substituted or unsubstituted C₁₋₁₀ alkylene, a substituted or unsubstituted C₁₋₁₀ heteroalkylene, -substituted or unsubstituted C₁₋₁₀ alkylene-[PEG]n-, -substituted or unsubstituted C₁₋₁₀₋ heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S, and
wherein the substitution is substitution with one or more non-hydrogen substituents, and the non-hydrogen substituent is any one selected from the group consisting of a halogen, C₁₋₃ alkyl, -NH₂, =O, and =S; and
there is no case in which A₂₁ and A₂₂ are both absent at the same time.

In one embodiment, the linker of the present application may be represented by Formula 2-3 below: 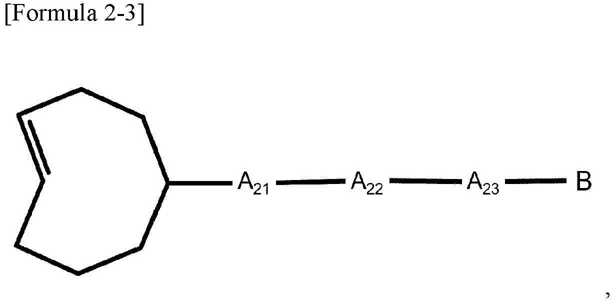 wherein
B is a thiol reactive group, which is a maleimide group or an APN group, and
-A₂₁-A₂₂-A₂₃- is a second anchor moiety.

Here, A₂₁ is absent, or -OC(=O)NH-;
A₂₂ is absent, or any one selected from substituted or unsubstituted C₁₋₁₀ alkylene, substituted or unsubstituted C₁₋₁₀ heteroalkylene, -substituted or unsubstituted C₁₋₁₀ alkylene-[PEG]n-, -substituted or unsubstituted C₁₋₁₀-heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
wherein the heteroalkylene is one or more heteroatom groups selected from the group consisting of -NH-, -O-, -S-, -O-N=, -S(=O)-, and -S(=O)₂-, or one or more heteroatoms selected from O, N, and S, and
wherein the substation is performed with one or more non-hydrogen substituents, and the non-hydrogen substituent is any one selected from the group consisting of a halogen, C₁₋₃ alkyl, -NH₂, =O, and =S;
A₂₃ is absent, or -C(=O)NH-, or -C(=O)O-; and
there is no case in which A₂₁, A₂₂, and A₂₃ are all absent at the same time.

In one embodiment, the linker of the present application may be represented by Formula 2-4 below: 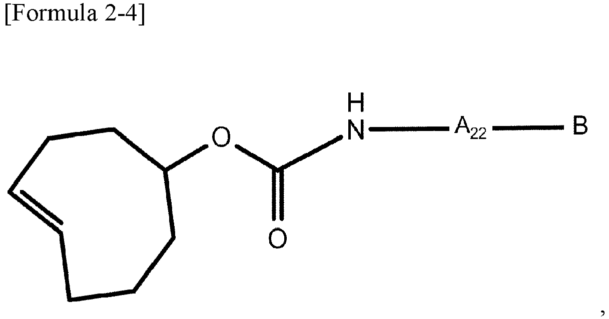
wherein B is a thiol reactive group, which is a maleimide group or an APN group, and
A₂₂ is absent, or any one selected from substituted or unsubstituted C₁₋₁₀ alkylene, substituted or unsubstituted C₁₋₁₀ heteroalkylene, -substituted or unsubstituted C₁₋₁₀ alkylene-[PEG]n-, -substituted or unsubstituted C₁₋₁₀-heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, in which n is an integer of 1 to 12,
the heteroalkylene is one or more heteroatom groups selected from the group consisting of -NH-, -O-, -S-, -O-N=, -S(=O)-, and -S(=O)₂-, or one or more heteroatoms selected from the group consisting of O, N, and S, and
the substation is performed with one or more non-hydrogen substituents, and the non-hydrogen substituent is any one selected from the group consisting of halogen, C₁₋₃ alkyl, -NH₂, =O, and =S.

In one embodiment, the linker may be any one of the compounds represented by the following formulas:

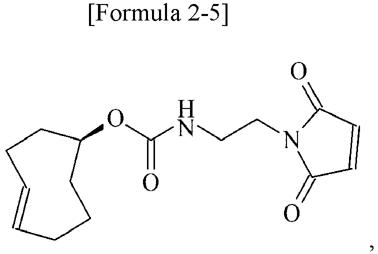

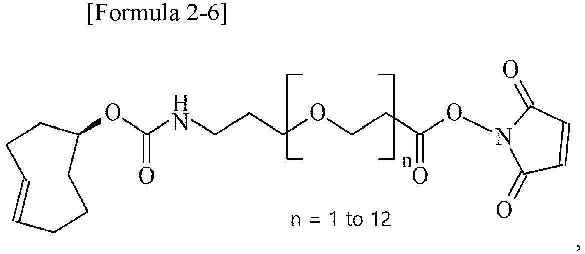

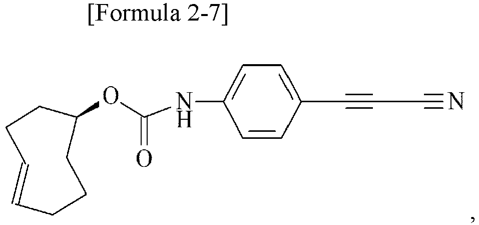

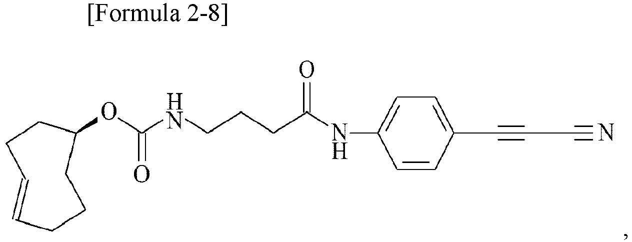

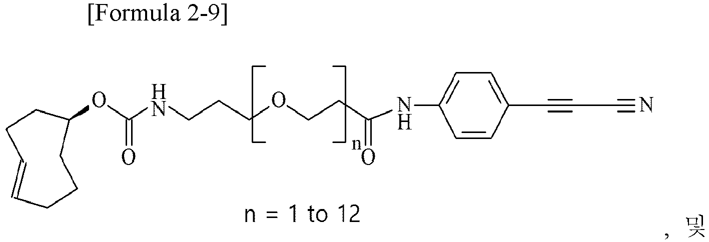

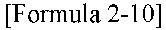

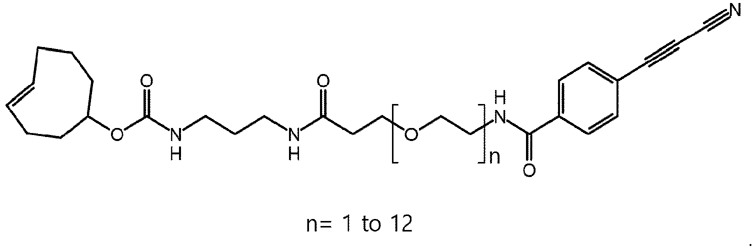

### Method of preparing functional polypeptide variant-lasting protein conjugate 1

In the present application, a method of preparing a functional polypeptide variant-lasting protein conjugate is disclosed. The following components are involved in the method of preparing the functional polypeptide variant-lasting protein conjugate: a functional polypeptide variant; a linker; and a lasting protein. Here, each component is the same as described above.

The method of preparing the functional polypeptide variant-lasting protein conjugate largely includes the following steps:
preparing a functional polypeptide variant-linker complex by the reaction of a tetrazine group included in the functional polypeptide variant and a dienophile group of the linker (functional polypeptide variant-linker conjugation reaction); and
preparing a lasting protein-linker complex by the reaction of a thiol residue of cysteine included in the lasting protein and a thiol reactive group of the linker (lasting protein-linker conjugation reaction).

Here, the order of the functional polypeptide variant-linker conjugation reaction and the lasting protein-linker conjugation reaction is not limited, and both reactions may be simultaneously performed. In addition, an intermediate product of the reaction may be produced depending on the order of each reaction. Hereinafter, the method of preparing a functional polypeptide variant-lasting protein conjugate will be described in detail.

### Method of preparing functional polypeptide variant-lasting protein conjugate

### 1-1: first reacting a lasting protein with a linker

In one embodiment, the conjugate of the present application may be prepared by first reacting a lasting protein with a linker to prepare the lasting protein-linker complex, and reacting the prepared lasting protein-linker complex with a functional polypeptide variant.

In one embodiment, the method of preparing a functional polypeptide variant-lasting protein conjugate includes the following steps:
Contacting a lasting protein with a linker, wherein a lasting protein-linker complex is prepared by the reaction between a thiol residue of cysteine included in the lasting protein and a thiol reactive group included in the linker; and
contacting the long-acting protein-linker complex with a functional polypeptide variant, wherein a functional polypeptide variant-lasting protein conjugate is prepared by the reaction between a dienophile included in the lasting protein-linker complex and a tetrazine or triazine group included in the functional polypeptide variant.

### Method of preparing functional polypeptide variant-lasting protein conjugate

### 1-2: first reacting a functional polypeptide variant with a linker

In one embodiment, a conjugate of the present application may be prepared by first reacting a functional polypeptide variant with a linker to prepare a functional polypeptide variant-linker complex, and reacting the prepared functional polypeptide variant-linker complex with a lasting protein.

In one embodiment, the method of preparing a functional polypeptide variant-lasting protein conjugate includes:
contacting a functional polypeptide variant with a linker, wherein a functional polypeptide variant-linker complex is prepared by the reaction between a tetrazine group included in the functional polypeptide variant and a dienophile group included in the linker; and
contacting the functional polypeptide variant-linker complex with a lasting protein, wherein a functional polypeptide variant-lasting protein is prepared by the reaction between a thiol reactive group included in the functional polypeptide variant-linker complex and a thiol residue of cysteine included in the lasting protein.

### Method of preparing functional polypeptide variant-lasting protein conjugate

### 1-3: reacting all of a functional polypeptide variant, a linker, and a lasting protein at the same time

In one embodiment, the method of preparing a conjugate of the present application may be performed by adding all reactants and simultaneously reacting them.

In one embodiment, the method of preparing a functional polypeptide variant-lasting protein conjugate includes:
contacting a functional polypeptide variant including a tetrazine group, a linker including a dienophile group and a thiol reactive group, and a lasting protein including a thiol residue.

Here, in one embodiment, the functional polypeptide variant and the linker are conjugated by an IEDDA reaction between the tetrazine group and the dienophile group, and the linker and the lasting protein are conjugated through the reaction between the thiol reactive group and the thiol residue, thereby the functional polypeptide variant-lasting protein conjugate is prepared.

### Method of preparing functional polypeptide variant-lasting protein conjugate 2: preparation using partner fusion protein

A polypeptide or a polypeptide variant may be provided by a chemical synthesizing method or a biological method such as expression using host cells according to a size of the polypeptide or the polypeptide variant.

When the polypeptide or a variant thereof is used through expression in host cells, a polypeptide with a small molecular weight has a problem of low expression efficiency in host cells, so a method using a partner protein may be used.

That is, the polypeptide with a low molecular weight may be expressed by being fused with a partner protein (hereinafter, referred to as a partner fusion protein), thereby improving expression efficiency. Here, the partner protein is a protein with a molecular weight of a certain level or more, and it is not limited as long as it is a protein having no difficulty in expressing the polypeptide due to being fused with the polypeptide disclosed in the present application.

More specifically, when such a partner protein is used, the following components are involved in the method of preparing a conjugate of the present application:
a functional polypeptide variant; a partner protein; a linker; and a lasting protein.

Here, the linker and the lasting protein are described above.

The partner fusion protein including the functional polypeptide variant and the partner protein will be described below.

### Partner fusion protein

As described above, to solve the problems related to the expression of a polypeptide with a small molecular weight, the polypeptide may be expressed by being fused with a partner protein. In the specification, the fusion of a polypeptide and a partner protein is referred to as a "partner fusion protein."

The "partner protein" is a protein with a certain amount of molecular weight, there is no significant limitation as long as it is a protein that has no difficulty in expression by fusion with the functional polypeptide variant of the present application.

In one embodiment, the partner protein may be a maltose-binding protein or glutathione S-transferase.

In one embodiment, the partner protein may be a fluorescent protein. In one specific example, the partner protein may be a self-fluorescent protein such as a green fluorescent protein (GFP), a superfolder green fluorescent protein (sfGFP), HcRed, DsRed, a cyan fluorescent protein (CFP), a yellow fluorescent protein (YFP) or a blue fluorescent protein (BFP).

In one embodiment, the partner protein may be a ubiquitin or small ubiquitinrelated modifier (SUMO) protein. However, the partner protein is not limited thereto.

In one embodiment, the partner fusion protein may be obtained by expression using one sequence in which the functional polypeptide variant and the partner protein are directly linked.

In one embodiment, the partner fusion protein may be obtained by expression in a configuration in which the functional polypeptide variant and the partner protein are linked with each other via an amino acid linking moiety. In one specific example, the amino acid linking moiety may include the amino acid sequence GGKKKKKGG, but the present application is not limited thereto.

Accordingly, as an embodiment, a partner fusion protein having the structure of [partner protein]-[amino acid linking moiety]-[functional polypeptide variant] may be provided.

In one embodiment of the present application, a partner fusion protein having the structure of [partner protein]-[functional polypeptide variant] may be provided.

In one embodiment of the present application, to prepare the functional polypeptide variant with a small molecular weight, after preparing such the partner fusion protein, a method of separating the partner protein may be used.

In one embodiment, in the case of a partner fusion protein having the structure of [partner protein]-[amino acid linking moiety]-[functional polypeptide variant], a desired functional polypeptide variant may be obtained by treatment of a polypeptide cleavage enzyme. Here, in one example, the treated polypeptide cleavage enzyme may be a protease. As a specific example, when the amino acid linking moiety includes the amino acid sequence IEGR, the partner protein may be separated by treatment of the Factor Xa enzyme, thereby obtaining a functional polypeptide variant.

In another embodiment, in the case of a partner fusion protein having the structure of [partner protein]-[functional polypeptide variant], a desired functional polypeptide variant may be obtained by treating an enzyme directly acting on the partner protein. As a specific example, when the partner protein is ubiquitin, a functional polypeptide variant may be obtained by treatment of the USP-1 enzyme. When the partner protein is SUMO, a functional polypeptide variant may be separately obtained by treating SUMO protease.

### Method of preparing partner fusion protein

In the present application, a method of preparing the partner fusion protein is disclosed. The following components are involved in the method of preparing a partner fusion protein: a cell line expressing a partner fusion protein; tRNA synthetase having a function of including a non-natural amino acid in a polypeptide by recognizing a specific stop codon; and a vector encoding the partner fusion protein by encoding a non-natural amino acid using the specific stop codon.

The method of preparing a functional polypeptide variant is a method of expressing a partner fusion protein in the cell line, based on a vector encoding the partner fusion protein, in which the tRNA synthetase is involved. In the method of preparing the partner fusion protein, when the partner fusion protein can be expressed in the cell line, the order of each process is not particularly limited, and additional processes may be included as needed.

In one embodiment, the method of preparing the partner fusion protein includes the following steps:
transfecting a cell line with a nucleic acid encoding tRNA synthetase and a nucleic acid encoding a partner fusion protein; and
culturing the cell line is cultured in a medium containing a non-natural amino acid,
wherein, the partner fusion protein has the structure of [partner protein]-[amino acid linking moiety]-[functional polypeptide variant] or [partner protein]-[functional polypeptide variant],
the functional polypeptide variant includes one or more non-natural amino acids, the tRNA synthetase has a function of recognizing the amber codon (5'-UAG-3') and inserting the non-natural amino acid into the sequence of a polypeptide to be synthesized, and in the nucleic acid encoding the functional polypeptide fusion variant, a codon corresponding to the non-natural amino acid is the amber codon (5'-UAG-3').

Here, the tRNA synthetase is the same as described in the part relating to the method of preparing the functional polypeptide variant of the present application.

Here, the cell line used to express the partner fusion protein is the same as described in the part relating to the method of preparing the functional polypeptide variant.

### Method of preparing functional polypeptide variant-lasting protein conjugate

### using partner fusion protein

In one embodiment of the present application, a method of preparing a functional polypeptide variant-lasting protein conjugate including the following steps is provided:
(i) preparing a partner fusion protein having the structure of [partner protein]-[amino acid linking moiety]-[functional polypeptide variant] or [partner protein]-[functional polypeptide variant] using host cells;
(ii) preparing a first conjugate (partner fusion protein-lasting protein conjugate) by conjugating a lasting protein to the partner fusion protein, wherein the first conjugate (partner fusion protein-lasting protein conjugate) may be obtained by independently or simultaneously performing two conjugation reactions as follows:
   - partner fusion protein-linker conjugation reaction
      A partner fusion protein-linker complex is prepared by reacting a tetrazine or triazine group included in a polypeptide variant of the partner fusion protein and a dienophile group of the linker, and
   - lasting protein-linker conjugation reaction
      A lasting protein-linker complex is prepared by reacting a thiol residue of cysteine included in the lasting protein and a thiol reactive group of the linker; and
(iii) treating a polypeptide cleavage enzyme to the first conjugate (partner fusion protein-lasting protein conjugate).

In the case of the partner fusion protein having the structure of [partner protein]-[amino acid linking moiety]-[functional polypeptide variant], the polypeptide cleavage enzyme may have a function of cleaving the amino acid linking moiety, or a function of directly cleaving the functional polypeptide variant.

In the case of the partner fusion protein having the structure of [partner protein]-[functional polypeptide variant], the polypeptide cleavage enzyme may have a function of directly cleaving the partner protein or functional polypeptide variant.

In this step, the final product, such as a second conjugate (polypeptide variant-long-acting protein conjugate), is prepared by separating and removing the partner protein from the partner fusion protein-HSA conjugate.

In another embodiment of the present application, a method of preparing a functional polypeptide variant-lasting protein conjugate including the following steps is provided:
(i) preparing a partner fusion protein having the structure of [partner protein]-[amino acid linking moiety]-[functional polypeptide variant] or [partner protein]-[functional polypeptide variant] using host cells;
(ii) obtaining a functional polypeptide variant by treating the obtained partner fusion protein with a polypeptide cleavage enzyme, the detailed description thereof is the same as above; and
(iii) performing conjugation using the obtained functional polypeptide variant, a linker and a lasting protein according to the above-described method.

In still another embodiment of the present application, a partner fusion protein-linker complex may be prepared by reacting a partner fusion protein and a linker. In this case, a functional polypeptide variant-linker complex may be prepared by treating the partner fusion protein-linker complex with a polypeptide cleavage enzyme. Here, the functional polypeptide variant-linker complex and the lasting protein may be conjugated as described above.

A method of preparing a partner fusion protein-lasting protein conjugate will be described in detail below.

### Partner fusion protein-long-acting protein conjugate and method of preparing

### the same

A partner fusion protein-long-acting protein conjugate according to one embodiment of the present application may be represented by Formula 3 below:

[Formula 3] fP'-J₁-A₂-J₂-P₂

Here, fP' is a partner fusion protein moiety, which is derived from the partner fusion protein.

In one embodiment, the partner fusion protein may have the structure of [partner protein]-[amino acid linking moiety]-[functional polypeptide variant] or [partner protein]-[functional polypeptide variant].

Here, each of J₁, A₂, J₂, and P₂ is as disclosed in Formula 1.

A method of preparing a partner fusion protein-lasting protein conjugate of the present application is disclosed below.

The following components are involved in the method of preparing a partner fusion protein-lasting protein conjugate: a partner fusion protein; a linker; and a lasting protein. Here, each component is the same as described above.

Specifically, the method of preparing the partner fusion protein-lasting protein conjugate includes:
preparing a partner fusion protein-linker complex by reacting a tetrazine group included in a functional polypeptide variant of the partner fusion protein and a dienophile group of the linker (partner fusion protein-linker conjugation reaction); and
preparing a lasting protein-linker complex by reacting a thiol residue of cysteine included in the lasting protein and a thiol reactive group of the linker (lasting protein-linker conjugation reaction).

Here, the order of the partner fusion protein-linker conjugation reaction and the lasting protein-linker conjugation reaction is not limited, and both reactions may be simultaneously performed. In addition, intermediate products of the reactions may be produced according to the order of the reactions. Hereinafter, the method of preparing the partner fusion protein-lasting protein conjugate will be described in detail.

In one embodiment, a partner fusion protein-lasting protein conjugate may be prepared by preparing a lasting protein-linker complex by first reacting a lasting protein and a linker, and reacting the prepared lasting protein-linker complex and a partner fusion protein.

In another embodiment, a partner fusion protein-lasting protein conjugate may be prepared by preparing a partner fusion protein-linker complex by first reacting a partner fusion protein and a linker, and reacting the prepared partner fusion protein-linker complex and a lasting protein.

In still another embodiment, a partner fusion protein-lasting protein conjugate may be prepared by simultaneously reacting a partner fusion protein, a linker, and a lasting protein.

Here, in each embodiment, the tetrazine or triazine group of the functional polypeptide variant included in the partner fusion protein is linked with the dienophile group of the linker through an IEDDA reaction, and the thiol residue of the lasting protein is linked by the reaction with the thiol reactive group of the linker.

As described above, the functional polypeptide variant-lasting protein conjugate of the present application is prepared by site-specifically binding the lasting protein to the functional polypeptide variant using a chemical method, particularly, an IEDDA reaction. In this case, optionally, a partner protein may be used. Such the preparation method of the present application has the following advantages.

### Characteristic of method of preparing functional polypeptide variant-lasting protein conjugate 1 - High in vivo stability using biorthogonal reaction

According to the method of preparing the functional polypeptide variant-lasting protein conjugate, the functional polypeptide variant and the lasting protein are conjugated using an IEDDA reaction, which is one of the bioorthogonal reactions. Since a chemical functional group involved in the conjugation reaction is not present in molecules of the body, it has an advantage that the functional polypeptide variant-lasting protein conjugate prepared by the preparation method has very high binding stability *in vivo.*

### Characteristic of method of preparing functional polypeptide variant-lasting protein conjugate 2 - Very high yield due to IEDDA reaction

In the method of preparing the functional polypeptide variant-lasting protein conjugate, to conjugate the functional polypeptide variant and the linker, an inverse electron demand Diels-Alder reaction (IEDDA reaction) is used. Since the IEDDA reaction occurs at a very fast reaction rate and the creation of a reaction environment is easy, when a conjugate using the IEDDA reaction is prepared, it has a very high yield, compared to the case of using a strain-promoted azide-alkyne cycloaddition (SPAAC) reaction.

### Kit for preparing functional polypeptide variant-lasting protein conjugate

In one embodiment of the present application, a kit for preparing the functional polypeptide variant-lasting protein conjugate, which includes the following components, is provided:
a functional polypeptide variant; a linker; and a lasting protein.

Here, the functional polypeptide variant, the linker, and the lasting protein are the same as described above.

In one embodiment of the present application, the a for preparing the functional polypeptide variant-long-acting protein conjugate, which includes the following, is provided:
a partner fusion protein; a linker; and a long-acting protein.

Here, the partner fusion protein, the linker, and the lasting protein are the same as described above.

In one embodiment, the kit may further include a polypeptide cleavage enzyme.

### Specific example of functional polypeptide variant-lasting protein conjugate

In one embodiment of the present application, a functional polypeptide variant-lasting protein conjugate represented by Formula 1 below is provided:

[Formula 1] P₁-J₁-A₂-J₂-P₂,

wherein P₁ is a functional polypeptide variant moiety.

Here, the functional polypeptide variant moiety is derived from the functional polypeptide variant, which includes one or more non-natural amino acids, wherein the non-natural amino acid includes a first click chemistry functional group, wherein the first click chemistry functional group is a tetrazine or triazine group.

Here, A₂ is a second anchor moiety.

The second anchor moiety is derived from a linker represented by Formula 2 below:

[Formula 2] H₂-A₂-B,

Here, H₂ is a second click chemistry functional group, which is a dienophile group having an IEDDA reaction with the tetrazine group or triazine group,
A₂ is the second anchor moiety,
wherein the second anchor moiety is any one selected from substituted or unsubstituted C₁₋₂₀ alkylene, substituted or unsubstituted C₁₋₂₀ heteroalkylene, - substituted or unsubstituted C₁₋₂₀ alkylene-[PEG]n-, -substituted or unsubstituted C₁₋₂₀ heteroalkylene-[PEG]n-, -substituted or unsubstituted C₁₋₂₀ alkylene-[PEG]n-substituted or unsubstituted C₁₋₂₀ alkylene-, -substituted or unsubstituted C₁₋₂₀ alkylene-[PEG]n-substituted or unsubstituted C₁₋₂₀ heteroalkylene-, and -substituted or unsubstituted C₁₋₂₀ heteroalkylene-[PEG]n-substituted or unsubstituted C₁₋₂₀ heteroalkylene-,
wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S,
wherein the substitution is substitution with one or more non-hydrogen substituents, and the non-hydrogen substituent is any one selected from the group consisting of a halogen, C₁₋₃ alkyl, -NH₂, =O, and =S, and
wherein n is an integer of 1 to 12,
wherein B is a thiol reactive group reacting with a thiol residue.

Here, P₂ is a lasting protein moiety.

Here, J₁ is a first conjugation moiety, which is the structure formed by the reaction between the tetrazine group or triazine group and the second click chemistry functional group.

Here, J₂ is a second conjugation moiety, which is the structure formed by the reaction between the thiol reactive residue and the thiol residue included in the lasting protein.

In one embodiment of the present application, a functional polypeptide variant-lasting protein conjugate represented by Formula 1-1 below is provided:

[Formula 1-1] P₁-J₁-A₂₁-A₂₂-A₂₃-J₂-P₂,

wherein P₁ is a functional polypeptide variant moiety.

Here, the functional polypeptide variant moiety is derived from the functional polypeptide variant, wherein the functional polypeptide variant includes one or more non-natural amino acids, wherein the non-natural amino acid includes a first click chemistry functional group, wherein the first click chemistry functional group is tetrazine or triazine group.

Here, -A₂₁-A₂₂-A₂₃- is a second anchor moiety.

Here, A₂₁ is absent, or -OC(=O)NH-;
A₂₂ is absent, or any one selected from substituted or unsubstituted C₁₋₁₀ alkylene, substituted or unsubstituted C₁₋₁₀ heteroalkylene, -substituted or unsubstituted C₁₋₁₀ alkylene-[PEG]n-, -substituted or unsubstituted C₁₋₁₀₋ heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
Wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S, and
wherein the substitution is substitution with one or more non-hydrogen substituents, and the non-hydrogen substituent is any one selected from the group consisting of a halogen, C₁₋₃ alkyl, -NH₂, =O, and =S;
A₂₃ is absent, or -C(=O)NH- or -C(=O)O-; and
there is no case in which A₂₁, A₂₂, and A₂₃ are all absent at the same time.

Here, the second anchor moiety is derived from a linker represented by Formula 2-1 below:

[Formula 2-1] H₂-A₂₁-A₂₂-A₂₃-B

Here,
H₂ is a second click chemistry functional group, which is a dienophile group reacting with a tetrazine or triazine group, and
B is a thiol reactive group, which is a maleimide group or an APN group.
Here, P₂ is a lasting protein moiety.

Here, J₁ is a first conjugation moiety, which is the structure formed by the reaction between the tetrazine or triazine group and the second click chemistry functional group.

Here, J₂ is a second conjugation moiety, which is the structure formed by the reaction between the thiol reactive residue and the thiol residue included in the lasting protein.

In one embodiment of the present application, a functional polypeptide variant-long-acting protein conjugate represented by Formula 1-2 below is provided:

[Formula 1-2] P₁-J₁-A₂₁-A₂₂-J₂-P₂,

wherein P₁ is a functional polypeptide variant moiety.

Here, the functional polypeptide variant moiety is derived from the functional polypeptide variant, the functional polypeptide includes one or more non-natural amino acids, and the non-natural amino acid includes a first click chemistry functional group, wherein the first click chemistry functional group is a tetrazine group or triazine group.

Here, -A₂₁-A₂₂- is a second anchor moiety.

Here, A₂₁ is absent, or -OC(=O)NH-,
Here, A₂₁ is absent, or -OC(=O)NH-,
A₂₂ is absent, or any one selected from substituted or unsubstituted C₁₋₁₀ alkylene, substituted or unsubstituted C₁₋₁₀ heteroalkylene, -substituted or unsubstituted C₁₋₁₀ alkylene-[PEG]n-, -substituted or unsubstituted C₁₋₁₀-heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S, and
wherein the substitution is substitution with one or more non-hydrogen substituents, and the non-hydrogen substituent is any one selected from the group consisting of a halogen, C₁₋₃ alkyl, -NH₂, =O, and =S, and
there is no case in which A₂₁ and A₂₂ are both absent at the same time.

Here, the second anchor moiety is derived from a linker represented by Formula 2-2 below:

[Formula 2-2] H₂-A₂₁-A₂₂-B

Here,
H₂ is a second click chemistry functional group, which is a dienophile group reacting with tetrazine or triazine group, and
B is a thiol reactive group.
Here, P₂ is a long-acting protein moiety.

Here, J₁ is a first conjugation moiety, which is the structure formed by the reaction between the tetrazine group or triazine group and the second click chemistry functional group.

Here, J₂ is a second conjugation moiety, which is the structure formed by the reaction between the thiol reactive residue and the thiol residue included in the lasting protein.

In one embodiment, the dienophile group may be any one of a transbicyclo[6.1.0]nonene group, a trans-cyclooctene (TCO) group, a methylcyclopropene group, a bicyclo[6.1.0]nonyne group, a cyclooctyne group, a norbornene group, a cyclopentene group, and a styrene group.

In one embodiment, the thiol reactive group may be a maleimide group, or a 3-arylpropiolonitrile (APN) group.

In one embodiment, the thiol reactive group may be any one selected from the following structures: 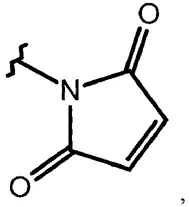 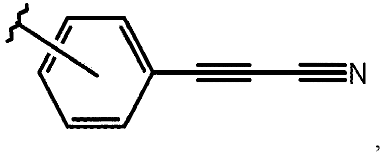 and

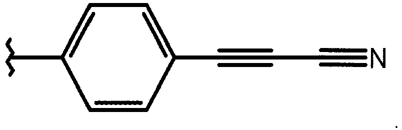

In one embodiment, the first conjugation moiety may be represented by any one of the following structures: 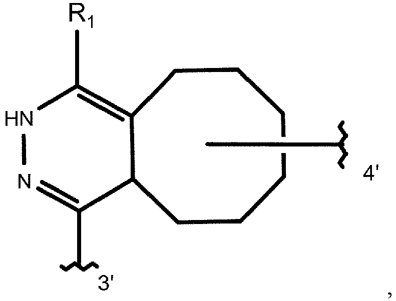 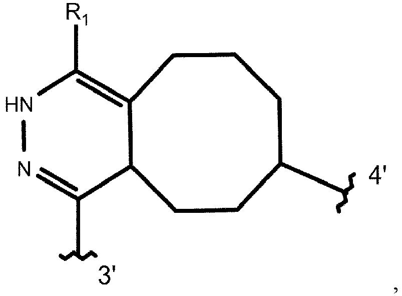 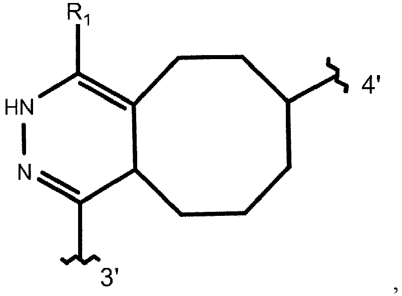 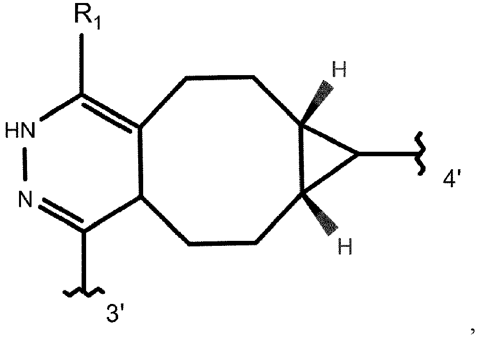 and

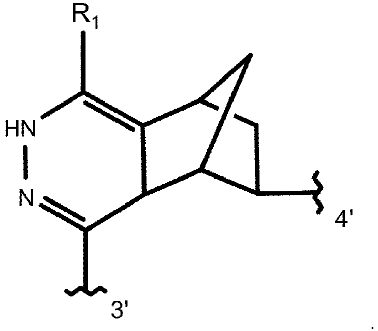

Here,
R₁ is any one selected from H, halogen, C₁₋₃ alkyl, C₃₋₆ cycloalkyl, C₃₋₆ heterocycloalkyl, aryl, and heteroaryl, and
3' may be linked with a functional polypeptide variant moiety, and 4' may be linked with a second anchor moiety.

In one embodiment, 4' is linked with A₂ in the functional polypeptide variant-lasting protein conjugate.

In one embodiment, 4' is linked with A₂₁ in the functional polypeptide variant-lasting protein conjugate.

In one embodiment, 4' is linked with A₂₂ in the functional polypeptide variant-lasting protein conjugate.

In one embodiment, 4' is linked with A₂₃ in the functional polypeptide variant-lasting protein conjugate.

In one embodiment, the second conjugation moiety may be represented by any one of the following structures: 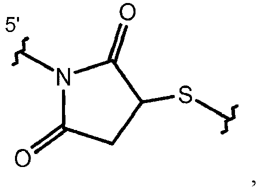 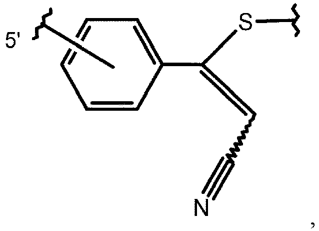 and

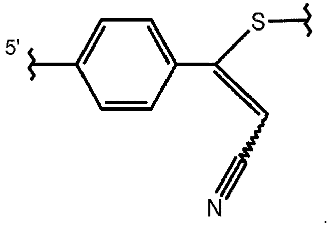

Here, S may be derived from a thiol residue of cysteine included in the lasting protein, and
5' may be linked with the second anchor moiety.

In one embodiment, 5' is linked with A₂ in the functional polypeptide variant-lasting protein conjugate.

In one embodiment, 5' is linked with A₂₁ in the functional polypeptide variant-lasting protein conjugate.

In one embodiment, 5' is linked with A₂₂ in the functional polypeptide variant-lasting protein conjugate.

In one embodiment, 5' is linked with A₂₃ in the functional polypeptide variant-lasting protein conjugate.

In one embodiment, a functional polypeptide variant-long-acting protein conjugate represented by Formula 1-3 below is provided: 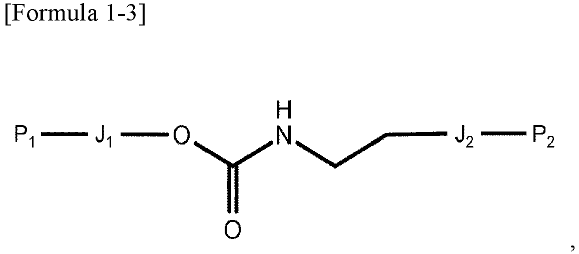 wherein P₁ is a functional polypeptide variant moiety.

Here, the functional polypeptide variant moiety is derived from the functional polypeptide variant which includes one or more non-natural amino acids, and the non-natural amino acid includes a first click chemistry functional group which is a tetrazine or triazine group.

Here, J₁ is a first conjugation moiety, which is represented by any one of the following structures: 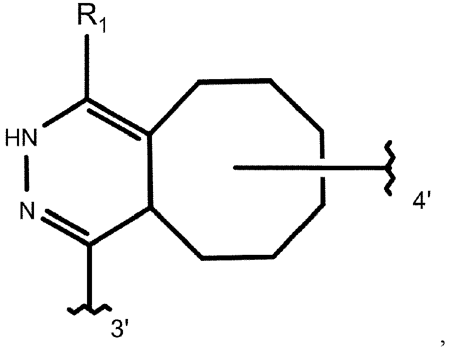 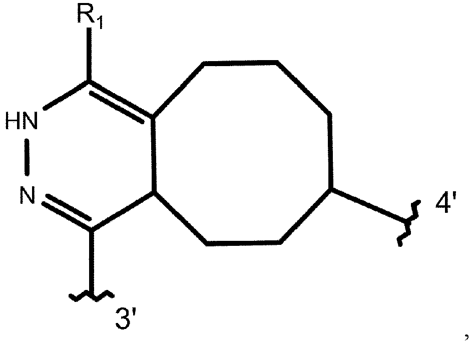 and 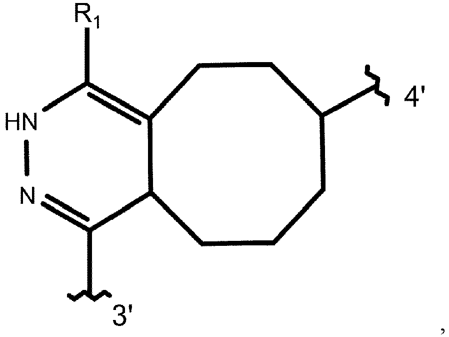
wherein R₁ is any one selected from H, halogen, C₁₋₃ alkyl, C₃₋₆ cycloalkyl, C₃₋₆ heterocycloalkyl, aryl, and heteroaryl, and
3' may be linked with the functional polypeptide variant moiety, and 4' may be linked with O.

Here, J₂ is a second conjugation moiety, which is represented by any one of the following structures: 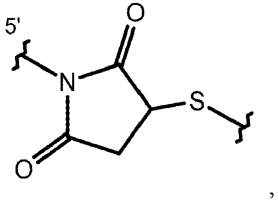 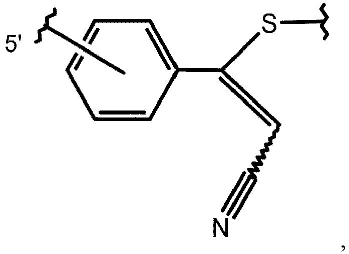 and 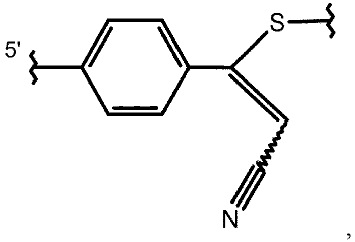
wherein S may be derived from a thiol residue of cysteine included in the long-acting protein, and
5' may be linked with the second anchor moiety.

In one embodiment, a functional polypeptide variant-lasting protein conjugate represented by Formula 1-4 below is provided: 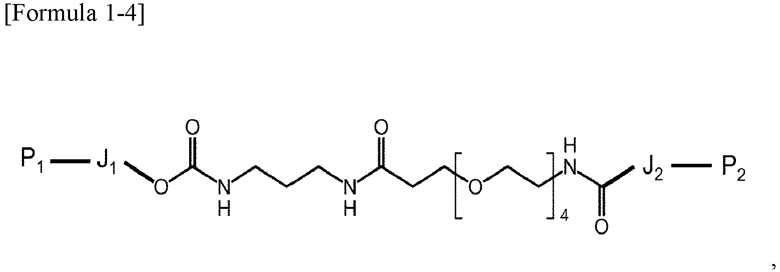 wherein P₁ is a functional polypeptide variant moiety.

Here, the functional polypeptide variant moiety is derived from the functional polypeptide variant which includes one or more non-natural amino acids, and the non-natural amino acid includes a first click chemistry functional group which is a tetrazine or triazine group.

Here, J₁ is a first conjugation moiety, which is represented by any one of the following structures: 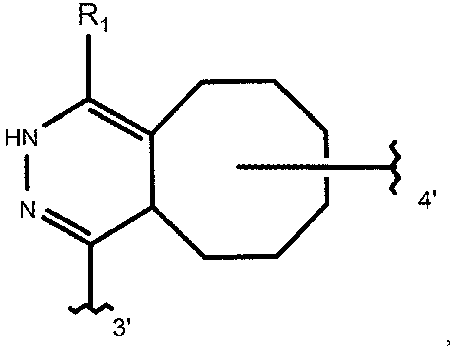 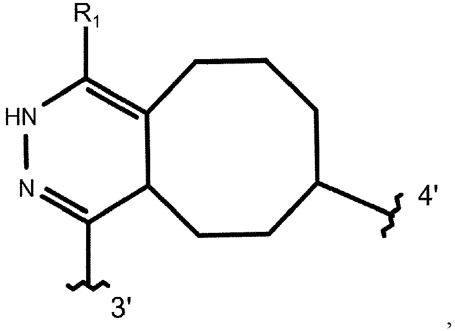 and 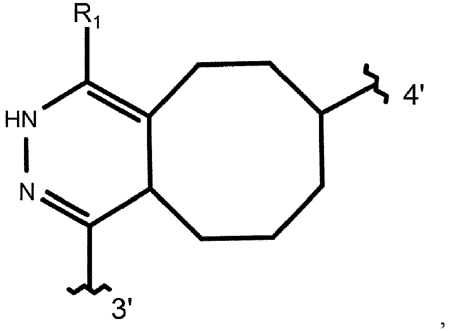
wherein R₁ is any one selected from H, halogen, C₁₋₃ alkyl, C₃₋₆ cycloalkyl, C₃₋₆ heterocycloalkyl, aryl, and heteroaryl, and
3' may be linked with the functional polypeptide variant moiety, and 4' may be linked with O.

Here, J₂ is a second conjugation moiety, which is represented by any one of the following structures: 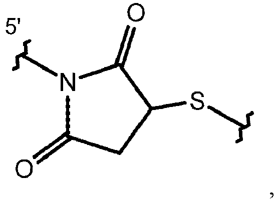 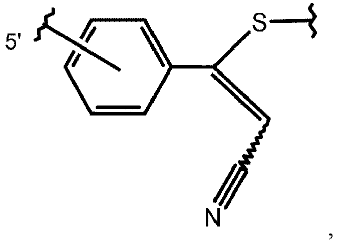 and 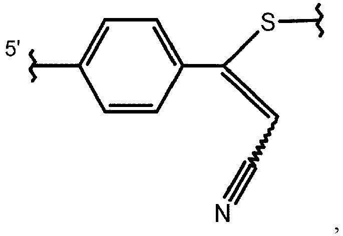
wherein S may be derived from a thiol residue of cysteine included in the lasting protein, and
5' may be linked with the second anchor moiety.

### [GLP1 variant-HSA conjugate]

In one embodiment of the present application, when the functional polypeptide variant of Formula 1 is a glucagon-like peptide-1 (GLP1) variant, and the lasting protein is human serum albumin (HSA) or a variant thereof, a GLP1 variant-HSA conjugate represented by Formula 5 is provided:

[Formula 5] (GLP1v)'-J₁-A₂-J₂-HSA',

wherein (GLP1v)' is derived from a GLP1 variant, and
HSA' is derived from HSA or a variant thereof.
Each of A₂, J₁, and J₂ is described above.

### GLP1 variant

As described above, in Formula 5, (GLP1v)' is derived from a GLP1 variant.

### Summary of GLP1 variant

The present application provides a GLP1 variant including one or more non-natural amino acids. Here, the non-natural amino acid includes a tetrazine or triazine group.

The GLP1 variant of the present application may be prepared by introducing a non-natural amino acid via tRNA in the process of translating RNA into a polypeptide.

The type of non-natural amino acid is disclosed in the part of the non-natural amino acid in [Functional polypeptide variant-lasting protein conjugate].

In one embodiment, a GLP1 variant on which dipeptidyl peptidase-4 (DPP-4) does not act or which is less affected thereby is provided.

DPP-4 cleaves the bond between the first amino acid and the second amino acid in the amino acid sequence of GLP1. Here, the bond between the amino acids cleaved by DPP-4 is called a DPP-4 recognition site.

In the GLP1 variant provided in the present application, as the DPP-4 recognition site is modified, the bond between amino acids in the GLP1 variant may no longer be cleaved by DPP-4. Such the characteristic has the effect of increasing stability by preventing the GLP1 variant from being easily cleaved by peptidase in the body.

Here, the DPP-4 recognition site may be modified by manipulating or modifying the amino acid sequence of GLP1. The modification of the DPP-4 recognition site may be caused by changing the type of amino acid directly involved in the bond cleaved by DPP-4, or changing an amino acid or adding a new amino acid at 1, 2 or 3 positions or farther away from the bond to be cleaved. In one example, the GLP1 variant represented by SEQ ID NO: 1, for example, a GLP1 variant, in which X2 is glycine, is no longer cleaved by DPP-4. Accordingly, the GLP1 variant according to one embodiment of the present application includes an amino acid sequence in which the DPP-4 recognition site is modified so it has a prolonged half-life.

GLP1 is a peptide consisting of 30 or 31 amino acids, and has a function of enhancing insulin secretion when the glucose concentration in the body is high.

The initial product, GLP1, made in L cells of the ileum and colon is formed in a GLP1 (7-37) or GLP1 (7-36) amide form by cleaving a part of the N-terminus for activation (unless particularly defined otherwise, GLP1 disclosed in the present application refers to activated GLP1).

However, the GLP1 becomes GLP1 (9-36) as two amino acids at the N-terminus are cleaved by an enzyme called dipeptidyl peptide IV (DPP-IV) and thus its activity disappears. The time for GLP1 activity to disappear is very short in the body, and quantitatively, the half-life of GLP1 in blood is only approximately 2 minutes.

In the present application, to prolong the half-life of GLP1 in blood, a GLP1 variant-lasting protein conjugate to which human serum albumin is site-specifically conjugated to GLP1 is provided.

### Method of preparing GLP1 variant

A method of preparing a functional polypeptide variant is the same as described above.

A vector encoding the GLP1 variant will be described below.

In one embodiment, the vector encoding a GLP1 variant may include one or more codons selected from an amber codon (5'-UAG-3'), an ocher codon (5'-UAA-3'), and an opal codon (5'-UGA-3') in the GLP1 variant sequence.

The vector encoding the GLP1 variant may include, for example, the following sequence:

ATGCATGGCGAAGGCACCTTTACCAGCGATGTGAGTAGCTATCTG
GAAGGTCAGGCGGCCAAAGAATTTATTGCCTGGCTGGTGCGGGGGCGTT
AGTAA (SEQ ID NO: 89)

In one embodiment, the vector encoding the GLP1 variant may be codon-optimized for an expression cell line. For example, the vector encoding the GLP1 variant may be codon-optimized for *E. coli.*

### Specific examples of GLP1 variant

In one embodiment of the present application, a GLP1 variant including the following amino acid sequence is provided. Here, the amino acid sequence is disclosed in the direction from the N-terminus to the C-terminus:

His-X2-X3-X4-X5-Phe-Thr-Ser-X9-X10-X11-X12-X13-Leu-X15-X16-Gln-
Ala-Ala-X20-X21-X22-ile-X24-X25-Leu-X27-X28-Gly-Arg-X31 (SEQ ID NO: 1)

Here, X2 may be selected from Ser, D-Ser, Gly, Ala, and D-Ala, X3 may be selected from Gln, Glu, His and Pro, X4 may be selected from L-Ala, D-Ala and Gly, X5 may be selected from TyR and Thr, X9 may be selected from Asp and Ser, X10 may be selected from Val, Tyr, Leu and a non-natural amino acid, X11 may be selected from Ser and Arg, X12 may be selected from Lys and Ser, X13 may be selected from Tyr and a non-natural amino acid, X15 may be selected from Glu and Asp, X16 may be selected from Glu and Gly, X20 may be selected from Lys and Cys, X21 may be selected from Glu, Asp and Gly, X22 may be selected from Phe and a non-natural amino acid, X24 may be selected from Ala, Glu, Lys and Cys, X25 may be selected from Trp and Asp, X27 may be selected from Val and Arg, X28 may be selected from Arg and Lys, and X31 may be selected from Gly and a non-natural amino acid, or may be absent. However, the present application is not limited thereto.

In one example, any one or more amino acids selected from the amino acids at positions 1, 2, 3, 4, 5, 6, 7, 8, 9,10, 11, 12, 13, 14, 15, 16, 17, 18, 19, 20, 21, 22, 23, 24, 25, 26, 27, 28, 29, 30 and 31 in SEQ ID NO: 1 may be a non-natural amino acid.

In one example, two or more amino acids selected from the amino acids at positions 1, 2, 3, 4, 5, 6, 7, 8, 9 ,10, 11, 12, 13, 14, 15, 16, 17, 18, 19, 20, 21, 22, 23, 24, 25, 26, 27, 28, 29, 30 and 31 in SEQ ID NO: 1 may be non-natural amino acids.

In one example, three or more amino acids selected from the amino acids at positions 1, 2, 3, 4, 5, 6, 7, 8, 9 ,10, 11, 12, 13, 14, 15, 16, 17, 18, 19, 20, 21, 22, 23, 24, 25, 26, 27, 28, 29, 30 and 31 in SEQ ID NO: 1 may be non-natural amino acids.

In one embodiment, the GLP1 variant of the present application may include any one amino acid sequence selected from the following amino acid sequences. Here, the amino acid sequence is disclosed in the direction from the N-terminus to the C-terminus. Here, X is a non-natural amino acid.
HGEGTFTSDVSSYLEGQAAKEFIAWLVKGRX (SEQ ID NO: 2)
HGEGTFTSDXSSYLEGQAAKEFIAWLVKGRG (SEQ ID NO: 3)
HGEGTFTSDVSSXLEGQAAKEFIAWLVKGRG (SEQ ID NO: 4)
HGEGTFTSDVSSYLEGQAAKEXIAWLVKGRG (SEQ ID NO: 5)
HGEGTFTSDVSSYLEGQAAKEFIAWLVRGRX (SEQ ID NO: 6)
HAEGTFTSDXSSYLEGQAAKEFIAWLVRGRG (SEQ ID NO: 7)
HAEGTFTSDVSSXLEGQAAKEFIAWLVRGRG (SEQ ID NO: 8)
HAEGTFTSDVSSYLEGQAAKEXIAWLVRGRG (SEQ ID NO: 9)
HGEGTFTSDXSSYLEGQAAKEFIAWLVRGRG (SEQ ID NO: 10)
HGEGTFTSDVSSXLEGQAAKEFIAWLVRGRG (SEQ ID NO: 11)
HGEGTFTSDVSSYLEGQAAKEXIAWLVRGRG (SEQ ID NO: 12)
HGEGTFTSDXSSYLEGQAAKEFIAWLVKGRX (SEQ ID NO: 13)
HGEGTFTSDVSSXLEGQAAKEFIAWLVKGRX (SEQ ID NO: 14)
HGEGTFTSDVSSYLEGQAAKEXIAWLVKGRX (SEQ ID NO: 15)
HAEGTFTSDXSSXLEGQAAKEFIAWLVRGRG (SEQ ID NO: 16)
HAEGTFTSDXSSYLEGQAAKEXIAWLVRGRG (SEQ ID NO: 17)
HAEGTFTSDVSSXLEGQAAKEXIA WL VRGRG (SEQ ID NO: 18)
HGEGTFTSDXSSXLEGQAAKEFIAWLVRGRG (SEQ ID NO: 19)
HGEGTFTSDXSSXLEGQAAKEFIA WL VKGRX (SEQ ID NO: 20)
HGEGTFTSDXSSYLEGQAAKEXIAWLVKGRX (SEQ ID NO: 21)
HGEGTFTSDVSSXLEGQAAKEXIAWLVKGRX (SEQ ID NO: 22)
HGEGTFTSDXSSYLEGQAAKEXIAWLVRGRG (SEQ ID NO: 23)
HGEGTFTSDVSSXLEGQAAKEXIA WL VRGRG (SEQ ID NO: 24)
HAEGTFTSDXSSXLEGQAAKEXIA WL VRGRG (SEQ ID NO: 25)
HGEGTFTSDXSSXLEGQAAKEXIAWLVRGRG (SEQ ID NO: 26)

In one embodiment, the GLP1 variant may include an amino acid sequence having an identity of approximately 80%, 81%, 82%, 83%, 84%, 85%, 86%, 87%, 88%, 89%, 90%, 91%, 92%, 93%, 94%, 95%, 96%, 97%, 98%, 99%, or 100% with the amino acid sequence represented by any one of SEQ ID NOs: 2 to 26.

In one embodiment, X may be frTet.

In one embodiment, X may be Tet_v2.0.

### Method of preparing GLP1 variant-HSA conjugate 1

In the present application, a method of preparing a GLP1 variant-HSA conjugate is disclosed. The following components are involved in the method of preparing a GLP1 variant-HSA conjugate:
a GLP1 variant; a linker; and HSA or a variant thereof. Here, each component is the same as described above.

In one embodiment, specifically, the method of preparing a GLP1 variant-HSA conjugate includes
preparing a GLP1 variant-linker complex by the reaction of a tetrazine group included in the GLP1 variant and a dienophile group of the linker (GLP1 variant-linker conjugation reaction); and preparing a HSA-linker complex by the reaction between a thiol residue of cysteine included in the HSA or a variant thereof and a thiol reactive group of the linker (HSA-linker conjugation reaction).

Here, the order of the GLP1 variant-linker conjugation reaction and the HSA-linker conjugation reaction is not limited, and both reactions may be performed at the same time. In addition, intermediate products of the reactions may be produced according to the order of the reactions.

### Method of preparing GLP1 variant-HSA conjugate 2: preparation using partner fusion protein

As described above, when there is difficulty in expressing a small-size polypeptide variant in host cells, the polypeptide variant is expressed in the form of a partner fusion protein, and a GLP1 variant-HSA conjugate of the present application may be prepared using the same. Here, the partner fusion protein includes a partner protein and a GLP1 variant.

Here, each component is the same as described above.

In one embodiment of the present application, a method of preparing a GLP1 variant-HSA conjugate including the following steps is provided:
(i) preparing a partner fusion protein having the structure of [partner protein]-[amino acid linking moiety]-[GLP1 variant] or [partner protein]-[GLP1 variant];
(ii) preparing a first conjugate (partner fusion protein-HSA conjugate) by conjugating HSA to the partner fusion protein, wherein
   the first conjugate (partner fusion protein-HSA conjugate) may be obtained by independently or simultaneously performing the following two conjugation reactions:
   - Partner fusion protein-linker conjugation reaction
      A partner fusion protein-linker complex is prepared by the reaction between a tetrazine or triazine group included in a GLP 1 variant of the partner fusion protein and a dienophile group of the linker, and
   - HSA-linker conjugation reaction
      An HSA-linker complex is prepared by the reaction between a thiol residue of cysteine included in the HSA or a variant thereof and a thiol reactive group of the linker; and
(iii) treating the partner fusion protein-HSA conjugate with a polypeptide cleavage enzyme.

In the case of a partner fusion protein having the structure of [partner protein]-[amino acid linking moiety]-[GLP1 variant], the polypeptide cleavage enzyme may have a function of cleaving an amino acid linking moiety.

In the case of a partner fusion protein having the structure of [partner protein]-[GLP1 variant], the polypeptide cleavage enzyme may directly act on the partner protein and thus may have a cleavage function.

In this step, the partner protein is separated and removed from the partner fusion protein-HSA conjugate, thereby obtaining the final product, that is, a second conjugate (GLP1 variant-HSA conjugate).

For a more specific example, reference may be made to the description of the example "Preparation of GLP1 variant-HSA conjugate" in the present specification.

### [hGH variant-HSA conjugate]

In one embodiment of the present application, in Formula 1, when the functional polypeptide variant is an hGH variant, and the lasting protein is human serum albumin (HSA) or a variant thereof, an hGH variant-HSA conjugate represented by Formula 6 below is provided:

[Formula 6] (hGHv)'-J₁-A₂-J₂-HSA',

wherein (hGHv)' is derived from the hGH variant, and
HSA' is derived from HSA or a variant thereof.
A₂, J₁, and J₂ are the same as described above.

### hGH variant

As described above, in Formula 6, (hGHv)' is derived from the hGH variant.

### Summary of hGH variant

The hGH variant includes one or more non-natural amino acids. Here, the non-natural amino acid includes a tetrazine or triazine group.

The hGH variant of the present application may be prepared by introducing a non-natural amino acid via tRNA in the process of translating RNA into a polypeptide.

The type of non-natural amino acid is the same as disclosed in the part of the non-natural amino acid in [Functional polypeptide variant-lasting protein conjugate].

Human growth hormone (hGH) is a peptide hormone consisting of approximately 191 amino acids having functions of stimulating cell regeneration and cell growth in humans.

The case of lack of hGH is called growth hormone deficiency (GHD) or human growth hormone deficiency. This is a medical condition caused by not having enough growth hormone. Generally, a subject with human GHD is short. In addition, human GHD is accompanied by problems such as hypoglycemia, a small penis, a loss of muscle mass, a high cholesterol level, and/or low bone density. These problems may be resolved by administering hGH into the body.

However, as described above, when a peptide is administered into the body, there is a problem of a short half-life, which is similar to the case of hGH.

Accordingly, in the present application, to prolong the half-life of hGH in blood, an hGH variant-lasting protein conjugate in which human serum albumin is site-specifically conjugated to hGH is provided.

### Specific examples of hGH variant

In one embodiment, the amino acid sequence of hGH includes the following sequence. Here, the amino acid sequence is disclosed in the direction from the N-terminus to the C-terminus:




 (SEQ ID NO: 27)

In one embodiment, in the hGH variant, one or more standard amino acids of hGH may be substituted with non-natural amino acid(s).

In one embodiment, the standard amino acid substituted with a non-natural amino acid may be any one or more selected from Q29, Y35, L52, E56, E66, Q69, E74, F92, S106, Q122, R127, L128, G131, R134, Q141, T142, Y143, K145, D147, N149, N152, D153, and E186.

In one embodiment, the standard amino acid substituted with a non-natural amino acid may be L52 and/or Q141.

In one embodiment, an hGH variant is provided including any one sequence of the following amino acid sequences. Here, the non-natural amino acid is represented by X. Here, the amino acid sequence is disclosed in the direction from the N-terminus to the C-terminus: 
FPTIPLSRLFDNAMLRAHRLHQLAFDTYXEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 28);
 
FPTIPLSRLFDNAMLRAHRLHQLAFDTYQEFEEAXIPKEQKYSFLQNP
QTSLCFSESIPTPSNREETQQKSNLELLRISLLLIQSWLEPVQFLRSVFANSLVY
 
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 29);
 
FPTIPLSRLFDNAMLRAHRLHQLAFDTYQEFEEAYIPKEQKYSFLQNP
QTSXCFSESIPTPSNREETQQKSNLELLRISLLLIQSWLEPVQFLRSVFANSLVY
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 30);
 
FPTIPLSRLFDNAMLRAHRLHQLAFDTYQEFEEAYIPKEQKYSFLQNP
QTSLCFSXSIPTPSNREETQQKSNLELLRISLLLIQSWLEPVQFLRSVFANSLVY
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 31);
 
FPTIPLSRLFDNAMLRAHRLHQLAFDTYQEFEEAYIPKEQKYSFLQNP
QTSLCFSESIPTPSNREXTQQKSNLELLRISLLLIQSWLEPVQFLRSVFANSLVY
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 32);
 
FPTIPLSRLFDNAMLRAHRLHQLAFDTYQEFEEAYIPKEQKYSFLQNP
QTSLCFSESIPTPSNREETQXKSNLELLRISLLLIQSWLEPVQFLRSVFANSLVY
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 33);
 
FPTIPLSRLFDNAMLRAHRLHQLAFDTYQEFEEAYIPKEQKYSFLQNP
QTSLCFSESIPTPSNREETQQKSNLXLLRISLLLIQSWLEPVQFLRSVFANSLVY
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 34);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQXLRSVFANSLVY
 
GASDSNVYDLLKDLEEGIQTLMGRLEDGSPRTGQIFKQTYSKFDTNSHNDDA
LLKNYGLLYCFRKDMDKVETFLRIVQCRSVEGSCGF (SEQ ID NO: 35);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GAXDSNVYDLLKDLEEGIQTLMGRLEDGSPRTGQIFKQTYSKFDTNSHNDD
ALLKNYGLLYCFRKDMDKVETFLRIVQCRSVEGSCGF (SEQ ID NO: 36);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIXTLMGRLEDGSPRTGQIFKQTYSKFDTNSHNDDA
LLKNYGLLYCFRKDMDKVETFLRIVQCRSVEGSCGF (SEQ ID NO: 37);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGXLEDGSPRTGQIFKQTYSKFDTNSHNDDA
LLKNYGLLYCFRKDMDKVETFLRIVQCRSVEGSCGF (SEQ ID NO: 38);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRXEDGSPRTGQIFKQTYSKFDTNSHNDD
ALLKNYGLLYCFRKDMDKVETFLRIVQCRSVEGSCGF (SEQ ID NO: 39);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDXSPRTGQIFKQTYSKFDTNSHNDDA
LLKNYGLLYCFRKDMDKVETFLRIVQCRSVEGSCGF (SEQ ID NO: 40);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
 
GASDSNVYDLLKDLEEGIQTLMGRLEDGSPXTGQIFKQTYSKFDTNSHNDDA

LLKNYGLLYCFRKDMDKVETFLRIVQCRSVEGSCGF (SEQ ID NO: 41);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKXTYSKFDTNSHNDDA
LLKNYGLLYCFRKDMDKVETFLRIVQCRSVEGSCGF (SEQ ID NO: 42);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQXYSKFDTNSHNDD
ALLKNYGLLYCFRKDMDKVETFLRIVQCRSVEGSCGF (SEQ ID NO: 43);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQTXSKFDTNSHNDDA
LLKNYGLLYCFRKDMDKVETFLRIVQCRSVEGSCGF (SEQ ID NO: 44);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQTYSXFDTNSHNDDA
LLKNYGLLYCFRKDMDKVETFLRIVQCRSVEGSCGF (SEQ ID NO: 45);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQTYSKFXTNSHNDDA
LLKNYGLLYCFRKDMDKVETFLRIVQCRSVEGSCGF (SEQ ID NO: 46);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
 
GASDSNVYDLLKDLEEGIQTLMGRLEDGSPRTGQIFKQTYSKFDTXSHNDDA
LLKNYGLLYCFRKDMDKVETFLRIVQCRSVEGSCGF (SEQ ID NO: 47);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQTYSKFDTNSHXDDA
LLKNYGLLYCFRKDMDKVETFLRIVQCRSVEGSCGF (SEQ ID NO: 48);
 
FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQTYSKFDTNSHNXDA
LLKNYGLLYCFRKDMDKVETFLRIVQCRSVEGSCGF (SEQ ID NO: 49);
 and

FPTIPLSRLFDNAMLRAHRLHQLAFDTYQEFEEAYIPKEQKYSFLQNP
QTSLCFSESIPTPSNREETQQKSNLELLRISLLLIQSWLEPVQFLRSVFANSLVY
GASDSNVYDLLKDLEEGIQTLMGRLEDGSPRTGQIFKQTYSKFDTNSHNDDA
LLKNYGLLYCFRKDMDKVETFLRIVQCRSVXGSCGF (SEQ ID NO: 50).

In one embodiment, the hGH variant may include an amino acid sequence having an identity of approximately 80%, 81%, 82%, 83%, 84%, 85%, 86%, 87%, 88%, 89%, 90%, 91%, 92%, 93%, 94%, 95%, 96%, 97%, 98%, 99%, or 100% with the amino acid sequence represented by any one of SEQ ID NOs: 27 to 50.

In one embodiment, X may be frTet.

In one embodiment, X may be Tet_v2.0.

### Method of preparing hGH variant

A method of preparing a functional polypeptide variant is the same as described above.

A vector encoding the hGH variant will be described below.

In one embodiment, the vector encoding the hGH variant may include one or more codons selected from an amber codon (5'-UAG-3'), an ocher codon (5'-UAA-3'), and an opal codon (5'-UGA-3') in the hGH variant sequence.

The vector encoding the hGH variant may include, for example, any one of the following sequences.

ATGTTTCCAACCATTCCACTGTCTCGCCTGTTTGATAATGCAATGC
TGCGTGCACATCGTCTGCATCAGCTGGCATTTGATACCTATCAGGAATTT
GAAGAAGCATATATTCCAAAAGAACAGAAATATTCTTTCCTGCAAAATCC
ACAGACCTCTTAGTGTTTTTCTGAATCTATTCCAACCCCATCCAATCGTGA
AGAGACCCAACAGAAATCTAATCTGGAACTGCTGCGTATCTCTCTGTTAC
TGATTCAGTCTTGGCTGGAACCAGTGCAGTTCCTGCGTTCTGTGTTCGCA
AATTCTCTGGTTTATGGTGCATCTGATTCTAATGTTTATGATCTGCTGAAA
GATTTAGAGGAAGGAATTCAGACCCTGATGGGTCGTCTGGAAGATGGTT
CTCCACGTACCGGTCAGATTTTTAAACAGACCTATTCTAAATTTGATACC
AATTCTCATAATGACGATGCACTGCTGAAAAACTATGGTCTGCTGTACTG
CTTTCGTAAAGATATGGATAAAGTTGAAACCTTTCTGCGTATTGTTCAGT
GTCGTTCTGTTGAAGGTTCTTGTGGTTTTTAA (SEQ ID NO: 90)

ATGTTTCCAACCATTCCACTGTCTCGCCTGTTTGATAATGCAATGC
TGCGTGCACATCGTCTGCATCAGCTGGCATTTGATACCTATCAGGAATTT
GAAGAAGCATATATTCCAAAAGAACAGAAATATTCTTTCCTGCAAAATCC

ACAGACCTCTCTGTGTTTTTCTGAATCTATTCCAACCCCATCCAATCGTGA
AGAGACCCAACAGAAATCTAATCTGGAACTGCTGCGTATCTCTCTGTTAC
TGATTCAGTCTTGGCTGGAACCAGTGCAGTTCCTGCGTTCTGTGTTCGCA
AATTCTCTGGTTTATGGTGCATCTGATTCTAATGTTTATGATCTGCTGAAA
GATTTAGAGGAAGGAATTCAGACCCTGATGGGTCGTCTGGAAGATGGTT
CTCCACGTACCGGTCAGATTTTTAAATAGACCTATTCTAAATTTGATACC
AATTCTCATAATGACGATGCACTGCTGAAAAACTATGGTCTGCTGTACTG
CTTTCGTAAAGATATGGATAAAGTTGAAACCTTTCTGCGTATTGTTCAGT
GTCGTTCTGTTGAAGGTTCTTGTGGTTTTTAA (SEQ ID NO: 91)

As a specific example, the vector encoding the hGH variant may be codon-optimized for an expression cell line. For example, the vector encoding the hGH variant may be codon-optimized for *E. coli.*

### Method of preparing hGH variant-HSA conjugate

In the present application, a method of preparing an hGH variant-HSA conjugate is disclosed.

The hGH variant-HSA conjugate of the present application may be prepared in a similar manner to the methods of preparing GLP1 variant-HSA conjugate 1 and 2.

In one embodiment, the hGH variant-HSA conjugate may be prepared using an hGH variant; a linker; and HSA.

In another embodiment, the hGH variant-HSA conjugate may be prepared using an hGH variant; a partner protein; a linker; and HSA. The hGH variant and the partner protein may be expressed and used in the form of a partner fusion protein. Here, the partner fusion protein may have the structure of [partner protein]-[amino acid linking moiety]-[hGH variant] or [partner protein]-[hGH variant],

### [sfGFP variant-ABD conjugate]

In one embodiment of the present application, in Formula 1, when the functional polypeptide variant is an sfGFP variant, and the lasting protein is an albumin-binding domain (ABD) or a variant thereof, an sfGFP variant-ABD conjugate represented by Formula 8 below is provided:

[Formula 8] (sfGFPv)'-J₁-A₂-J₂-ABD',

wherein (sfGFP)' is derived from the sfGFP variant, and
ABD' is derived from ABD or a variant thereof.
A₂, J₁, and J₂ are the same as described above.

### sfGFP variant

As described above, in Formula 8, (sfGFP)' is derived from the sfGFP variant.

### Summary of sfGFP variant

The sfGFP variant includes one or more non-natural amino acids. Here, the non-natural amino acid includes a tetrazine or triazine group.

The sfGFP variant of the present application may be prepared by introducing a non-natural amino acid via tRNA in the process of translating RNA into a polypeptide.

The type of non-natural amino acid is the same as disclosed in the part of the non-natural amino acid of [Functional polypeptide variant-lasting protein conjugate].

Superfolder GFP (sfGFP) is a fluorescent protein mutated from GFP consisting of approximately 238 amino acids, which has a maximum absorption wavelength of approximately 485 nm and a maximum emission wavelength of approximately 515 nm.

As described above, when a peptide diagnostic and/or therapeutic agent or a polypeptide diagnostic and/or therapeutic agent is administered into the body, there is a problem of a short half-life, which is similar to the case of sfGFP.

Accordingly, in the present application, to prolong the half-life of sfGFP in blood, an sfGFP variant-ABD conjugate in which an albumin-binding domain (ABD) or a variant thereof is site-specifically conjugated to sfGFP is provided.

### Specific examples of sfGFP variant

In one embodiment, the amino acid sequence of sfGFP is as follows. Here, the amino acid sequence is disclosed in the direction from the N-terminus to the C-terminus:

GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 51)

In one embodiment, in the sfGFP variant, one or more standard amino acids of sfGFP may be substituted with non-natural amino acid(s).

In one embodiment, here, the standard amino acid substituted with a non-natural amino acid may be any one or more selected from E8, T11, V13, G26, K28, T40, N41, T51, D78, K103, K109, G118, K128, D131, D135, H150, Q159, K168, D175, D192, V195, N200, P213, and G230.

In one embodiment, here, the standard amino acid substituted with a non-natural amino acid may be H150.

In one embodiment, an sfGFP variant including any one sequence of the following amino acid sequences is provided. Here, the non-natural amino acid is represented by X. Here, the amino acid sequence is disclosed in the direction from the N-terminus to the C-terminus: 
GSMSKGEXLFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 52);
 
GSMSKGEELFXGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 53);
 
GSMSKGEELFTGXVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
 
NVYITADKQKNGIKANFKIRHNVEDGSVQLADHYQQNTPIGDGPVLLPDNH

YLSTQSVLSKDPNEKRDHMVLLEFVTAAGITHGMDEL (SEQ ID NO: 54);
 
GSMSKGEELFTGVVPILVELDGDVNXHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 55);
 
GSMSKGEELFTGVVPILVELDGDVNGHXFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 56);
 
GSMSKGEELFTGVVPILVELDGDVNGHKFSVRGEGEGDAXNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 57);
 
GSMSKGEELFTGVVPILVELDGDVNGHKFSVRGEGEGDATXGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 58);
 
GSMSKGEELFTGVVPILVELDGDVNGHKFSVRGEGEGDATNGKLTL

KFICXTGKLPVPWPTLVTTLTYGVQCFSRYPDHMKRHDFFKSAMPEGYVQE
 
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 59);
 
GSMSKGEELFTGVVPILVELDGDVNGHKFSVRGEGEGDATNGKLTL
KFICTTGKLPVPWPTLVTTLTYGVQCFSRYPXHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 60);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFXDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 61);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYXTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 62);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEXDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 63);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELXGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 64);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIXFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 65);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEXGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 66);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSX
NVYITADKQKNGIKANFKIRHNVEDGSVQLADHYQQNTPIGDGPVLLPDNH
YLSTQSVLSKDPNEKRDHMVLLEFVTAAGITHGMDEL (SEQ ID NO: 67);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
 
NVYITADKXKNGIKANFKIRHNVEDGSVQLADHYQQNTPIGDGPVLLPDNH

YLSTQSVLSKDPNEKRDHMVLLEFVTAAGITHGMDEL (SEQ ID NO: 68);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFXIRHNVEDGSVQLADHYQQNTPIGDGPVLLPDNH
YLSTQSVLSKDPNEKRDHMVLLEFVTAAGITHGMDEL (SEQ ID NO: 69);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEXGSVQLADHYQQNTPIGDGPVLLPDNH
YLSTQSVLSKDPNEKRDHMVLLEFVTAAGITHGMDEL (SEQ ID NO: 70);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGXGPVLLPDNH
YLSTQSVLSKDPNEKRDHMVLLEFVTAAGITHGMDEL (SEQ ID NO: 71);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPXLLPDNH
YLSTQSVLSKDPNEKRDHMVLLEFVTAAGITHGMDEL (SEQ ID NO: 72);
 
GSMSKGEELFTGVVPILVELDGDVNGHKFSVRGEGEGDATNGKLTL

KFICTTGKLPVPWPTLVTTLTYGVQCFSRYPDHMKRHDFFKSAMPEGYVQE
 
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDXH
YLSTQSVLSKDPNEKRDHMVLLEFVTAAGITHGMDEL (SEQ ID NO: 73);
 
GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDXNEKRDHMVLLEFVTAAGITHGMDEL (SEQ ID NO: 74);
 and

GSMSKGEELFTGVVPILVELDGDVNGHKFSVRGEGEGDATNGKLTL
KFICTTGKLPVPWPTLVTTLTYGVQCFSRYPDHMKRHDFFKSAMPEGYVQE
RTISFKDDGTYKTRAEVKFEGDTLVNRIELKGIDFKEDGNILGHKLEYNFNSH
NVYITADKQKNGIKANFKIRHNVEDGSVQLADHYQQNTPIGDGPVLLPDNH
YLSTQSVLSKDPNEKRDHMVLLEFVTAAXITHGMDEL (SEQ ID NO: 75).

In one embodiment, the sfGFP variant may include an amino acid sequence having an identity of approximately 80%, 81%, 82%, 83%, 84%, 85%, 86%, 87%, 88%, 89%, 90%, 91%, 92%, 93%, 94%, 95%, 96%, 97%, 98%, 99%, or 100% with the amino acid sequence represented by any one of SEQ ID NOs: 52 to 75.

In one embodiment, X may be frTet.

In one embodiment, X may be Tet_v2.0.

### Method of preparing sfGFP variant

A method of preparing a functional polypeptide variant is the same as described above.

A vector encoding the sfGFP variant will be described below.

In one embodiment, the vector encoding the sfGFP variant may include one or more codons selected from an amber codon (5'-UAG-3'), an ocher codon (5'-UAA-3'), and an opal codon (5'-UGA-3') in the sfGFP variant sequence.

The vector encoding the sfGFP variant may include, for example, the following sequence.

ATGGGATCTATGAGTAAAGGAGAAGAACTTTTCACTGGAGTTGTC
CCAATTCTTGTTGAATTAGATGGTGATGTTAATGGGCACAAATTTTCTGTC
CGTGGAGAGGGTGAAGGTGATGCAACAAACGGAAAACTTACCCTTAAAT
TTATTTGCACTACTGGAAAACTACCTGTTCCATGGCCAACACTTGTCACT
ACTCTGACCTATGGTGTTCAATGCTTCTCCCGTTATCCGGATCATATGAAA
CGGCATGACTTTTTCAAGAGTGCCATGCCCGAAGGTTATGTACAGGAACG
CACTATAAGCTTCAAAGATGACGGGACCTACAAGACGCGTGCTGAAGTC
AAGTTTGAAGGTGATACCCTTGTTAATCGTATCGAGTTAAAAGGTATTGA
TTTTAAAGAAGATGGAAACATTCTCGGACACAAACTCGAGTACAACTTTA
ACTCACACTAGGTATACATCACCGCAGACAAACAAAAGAATGGAATCAA
AGCTAACTTCAAAATTCGCCACAACGTTGAAGATGGATCCGTTCAACTAG
CAGACCATTATCAACAAAATACTCCAATTGGCGATGGCCCTGTCCTTTTA
CCAGACAACCATTACCTGTCGACACAATCTGTCCTTTCGAAAGATCCCAA
CGAAAAGCGTGACCACATGGTCCTTCTTGAGTTTGTAACTGCTGCTGGGA
TTACACATGGCATGGATGAGCTCTAA (SEQ ID NO: 92)

As a specific example, the vector encoding the sfGFP variant may be codon-optimized for an expression cell line. For example, the vector encoding the sfGFP variant may be codon-optimized for *E. coli.*

### Method of preparing sfGFP variant-ABD conjugate

In the present application, a method of preparing the sfGFP variant-ABD conjugate is disclosed.

The sfGFP variant-ABD conjugate of the present application may be prepared in a similar manner to the methods of preparing GLP1 variant-HSA conjugate 1 and 2.

In one embodiment, the sfGFP variant-ABD conjugate may be prepared using an sfGFP variant; a linker; and ABD or a variant thereof.

In another embodiment, the sfGFP variant-ABD conjugate may be prepared using a partner fusion protein; a linker; and ABD or a variant thereof. Here, the partner fusion protein may have the structure of [partner protein]-[amino acid linking moiety]-[sfGFP variant] or [partner protein]-[sfGFP variant].

### [Use of functional polypeptide variant-long-acting protein conjugate]

In the specification, a use of the functional polypeptide variant-lasting protein conjugate is disclosed. The functional polypeptide variant-lasting protein conjugate is stable due to a long half-life in the body, and has low immunogenicity. Accordingly, the functional polypeptide variant-lasting protein conjugate may more effectively exhibit the desired efficacy of a functional polypeptide. That is, the conjugate of the present application may be used in treatment or diagnosis of a disease, disorder, and/or an indication that the functional polypeptide targets for the purpose of treatment or diagnosis.

### Composition

The present application provides a composition including a functional polypeptide variant-lasting protein conjugate. In one embodiment, the composition may be a pharmaceutical composition. In another embodiment, the composition may be a composition for diagnosis.

The present application provides a pharmaceutical composition including a functional polypeptide variant-lasting protein conjugate.

In one embodiment, the pharmaceutical composition of the present application may be a pharmaceutical composition for treating a target disease.

In one embodiment, the pharmaceutical composition provided by the present application may further include a pharmaceutically acceptable additive (excipient). The additive may be appropriately selected from a material well known in the art.

In addition, the pharmaceutical composition may further include a different therapeutically active material (e.g., a therapeutic agent) for co-treatment.

In one embodiment, the pharmaceutical composition provided by the present application may be administered in various formulations according to oral and/or parenteral methods in clinical application. Preferably, the pharmaceutical composition is administered in an injectable formulation for treating a disease.

In one embodiment, the pharmaceutical composition of the present application may be used for parenteral administration. In one embodiment, a formulation for parenteral administration including the pharmaceutical composition of the present application may be prepared and/or used.

The parenteral administration may be any one injection method selected from intrathecal (IT) injection, intracerebroventricular (ICV) injection, intracranial injection, subcutaneous injection, intravenous injection, IV infusion, intramuscular injection, injection within the substantia nigra pars compacta and the ventral tegmental area, intraparenchymal injection, cisterna magna injection, and intrathoracic injection, or a combination thereof.

The formulations for parenteral administration may be mixed with water in combination with a stabilizer or buffer and prepared in the form of a solution or suspension, and may be prepared for ampoule- or vial-unit administration. In addition, the formulations for parenteral administration may include a preservative, a stabilizer, a hydration agent or emulsion accelerator, an adjuvant such as a salt and/or buffer for adjusting osmotic pressure, and other therapeutically useful substances, and may be prepared according to a conventional method, such as mixing, granulation or coating. In addition, the pharmaceutical composition of the present application may be prepared as a formulation comprising polymer excipient or the like, for sustained release of active substance delivery vector and/or active substance.

In another embodiment, the pharmaceutical composition of the present application may be used for oral administration. In one embodiment, a formulation for oral administration including the pharmaceutical composition of the present application may be prepared and/or used.

In one embodiment, a preferred dose of the pharmaceutical composition of the present application may be appropriately selected according to the condition and body weight of a patient, the severity of a symptom, a drug type, an administration route, and a duration. For example, the active ingredient of the pharmaceutical composition of the present application may be administered at 0.2 to 200 mg/kg a day.

In addition, the pharmaceutical composition of the present application may be administered once a day or in several divided doses, but the present application is not limited thereto.

### Pharmaceutical composition including GLP1 variant-HSA conjugate

In one embodiment of the present application, a pharmaceutical composition including a GLP1 variant-HSA conjugate is provided.

In one embodiment of the present application, a pharmaceutical composition including the GLP1 variant-HSA conjugate represented by Formula 5 is provided.

In one embodiment, the pharmaceutical composition including the GLP1 variant-HSA conjugate may be a pharmaceutical composition for treating a target disease. Here, the target disease may be any one selected from diabetes, chronic diabetes, obesity, type 2 diabetes, prediabetes, a liver disease, a metabolic disease, and a cardiovascular disease, but the present application is not limited thereto.

As one exemplary embodiment of the present application, a pharmaceutical composition for treating long-acting diabetes including the GLP1 variant-HSA conjugate may be provided.

As one exemplary embodiment of the present application, a pharmaceutical composition for treating obesity including the GLP1 variant-HSA conjugate may be provided.

### Pharmaceutical composition including hGH variant-HSA conjugate

As one exemplary embodiment of the present application, a pharmaceutical composition including an hGH variant-HSA conjugate is provided.

As one exemplary embodiment of the present application, a pharmaceutical composition including the hGH variant-HSA conjugate represented by Formula 6 is provided.

In one embodiment, the pharmaceutical composition including the hGH variant-HSA conjugate may be a pharmaceutical composition for treating a target disease. Here, the target disease may be growth hormone deficiency (GHD), but the present application is not limited thereto.

In one embodiment of the present application, a pharmaceutical composition for treating GHD including the hGH variant-HSA conjugate may be provided.

### Composition including sfGFP variant-ABD conjugate

In one embodiment of the present application, a composition for diagnosis, which includes an sfGFP variant-ABD conjugate, is provided.

In one embodiment of the present application, a composition for diagnosis, which includes an sfGFP variant-ABD conjugate represented by Formula 7, is provided.

In one embodiment, the composition including the sfGFP variant-ABD conjugate may be a composition for diagnosing a disease. In one embodiment, the composition including the sfGFP variant-ABD conjugate may be a composition for diagnosing cancer cells requiring albumin.

### [Embodiments of the present application]

Embodiments of the present application will be described below. The following embodiments are examples of the present application that can be provided by the present application, and the scope of the present application is not limited thereto.
A01. Functional polypeptide variant-lasting protein conjugate represented by Formula 1:

   [Formula 1] P₁-J₁-A₂-J₂-P₂

   wherein,
   P₁ is a functional polypeptide variant moiety, which is derived from a functional polypeptide variant, and the functional polypeptide variant includes a first click chemistry functional group, which is tetrazine group or derivative thereof or triazine group or derivative thereof;
   A₂ is a second anchor moiety,
   wherein the second acchor moiey is any one selected from substituted or unsubstituted C₁₋₂₀ alkylene, substituted or unsubstituted C₁₋₂₀ heteroalkylene, - substituted or unsubstituted C₁₋₂₀ alkylene-[PEG]n-, -substituted or unsubstituted C₁₋₂₀ heteroalkylene-[PEG]n-, -substituted or unsubstituted C₁₋₂₀ alkylene-[PEG]n-substituted or unsubstituted C₁₋₂₀ alkylene-, -substituted or unsubstituted C₁₋₂₀ alkylene-[PEG]n-substituted or unsubstituted C₁₋₂₀ heteroalkylene-, and -substituted or unsubstituted C₁₋₂₀ heteroalkylene-[PEG]n-substituted or unsubstituted C₁₋₂₀ heteroalkylene-,
   wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S,
   wherein the substitution is substitution with one or more non-hydrogen substituents, in which the non-hydrogen substituent is any one or more selected from the group consisting of a halogen, C₁₋₃ alkyl, -NH₂, =O, and =S, and
   wherein n is an integer of 1 to 12,
   P₂ is a lasting protein moiety, which is derived from a long-acting protein;
   J₁ is a first conjugation moiety, which is formed by the reaction of the first click chemistry functional group including the tetrazine group or derivative thereof with a second click chemistry functional group which is a dienophile; and
   J₂ is a second conjugation moiety, which is formed by the reaction of a thiol reactive group with a thiol residue.
A02. The functional polypeptide variant-lasting protein conjugate according to A01, wherein
   the second anchor moiety is -A₂₁-A₂₂-A₂₃-, wherein
   A₂₁ is absent, or -OC(=O)NH-,
   A₂₂ is absent, or any one selected from substituted or unsubstituted C₁₋₁₀ alkylene, substituted or unsubstituted C₁₋₁₀ heteroalkylene, -substituted or unsubstituted C₁₋₁₀ alkylene-[PEG]n-, -substituted or unsubstituted C₁₋₁₀₋ heteroalkylene-[PEG]n-, -substituted or unsubstituted C₁₋₁₀ alkylene-[PEG]n-substituted or unsubstituted C₁₋₁₀ alkylene-, -substituted or unsubstituted C₁₋₁₀ heteroalkylene-[PEG]n-substituted or unsubstituted C₁₋₁₀ alkylene-, and -substituted or unsubstituted C₁₋₁₀ heteroalkylene-[PEG]n-substituted or unsubstituted C₁₋₁₀ heteroalkylene-, wherein n is an integer of 1 to 12,
   wherein the heteroalkylene includes one or more heteroatom groups selected from the group consisting of -NH-, -O-, -S-, -O-N=, -S(=O)-, and -S(=O)₂-, or one or more heteroatoms selected from the group consisting of O, N, and S, and
   wherein the substitution is substituted with one or more non-hydrogen substituents, wherein each of the substituents is any one selected from the group consisting of halogen, C₁₋₃ alkyl, -NH₂, =O, and =S,
   A₂₃ is absent, or -C(=O)NH- or -C(=O)O-, and
   there is no case in which A₂₁, A₂₂, and A₂₃ are all absent at the same time.
A03. The functional polypeptide variant-lasting protein conjugate according to A01, wherein
   the second anchor moiety is -A₂₁-A₂₂-A₂₃-, wherein
   A₂₁ is -OC(=O)NH-,
   A₂₂ is absent, or unsubstituted C₁₋₁₀ alkylene, -unsubstituted C₁₋₁₀ alkylene-[PEG]n-, or -substituted C₁₋₁₀ heteroalkylene-[PEG]n-, in which n is an integer of 1 to 12, and
   A₂₃ is absent, or -C(=O)NH- or -C(=O)O-.
A04. The functional polypeptide variant-lasting protein conjugate according to A01, wherein
   the second anchor moiety is -A₂₁-A₂₂-, wherein
   A₂₁ is -OC(=O)NH-, and
   A₂₂ is an unsubstituted C₁₋₁₀ alkylene.
A05. The functional polypeptide variant-lasting protein conjugate according to A01, which is represented by
   Formula 1-3: 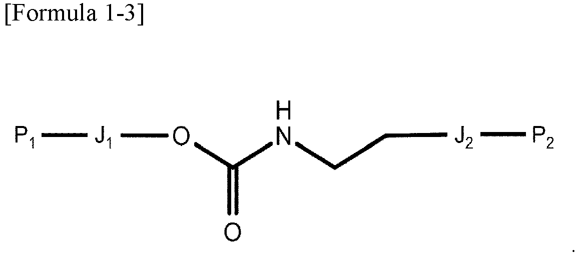
A06. The functional polypeptide variant-lasting protein conjugate according to A01, which is represented by
   Formula 1-4: 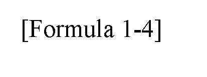 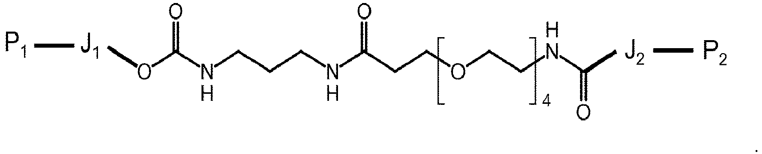
A07. The functional polypeptide variant-lasting protein conjugate according to A01, wherein
   the second anchor moiety is a substituted hydrocarbon chain including one or more heteroatoms, wherein
   the heteroatom is selected from N, O, and S, and
   the substitution is substitution with one or more non-hydrogen substituents, in which the non-hydrogen substituent is any one or more selected from the group consisting of halogen, C₁₋₃ alkyl, -NH₂, =O, and =S.
A08. The functional polypeptide variant-lasting protein conjugate according to A07, wherein
   the heteroatom is selected from N and O, and
   the non-hydrogen substituent is =O.
A09. The functional polypeptide variant-lasting protein conjugate according to any one of A01 to A08, wherein
   a non-natural amino acid included in the functional polypeptide variant is represented by the following structure: 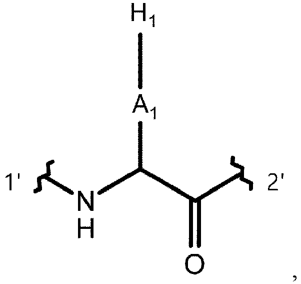
   H₁ is a first click chemistry functional group, which includes tetrazine group or derivative thereof, or triazine group or derivative thereof,
   A₁ is a first anchor moiety, which is absent or -A₁₁-A₁₂-, wherein
   A₁₁ is absent or C₁₋₅ alkylene,
   A₁₂ is absent, or any one selected from [arylene]p, -[arylene]p-C₁₋₅alkylene-, -[arylene]p-C₁₋₅heteroalkylene-, -arylene-C₁₋₅alkylene-arylene-, -arylene-C₁₋₅ heteroalkylene-arylene-, -arylene-heteroarylene-, [heteroarylene]p, - [heteroarylene]p-C₁₋₅alkylene-, -[heteroarylene]p-C₁₋₅heteroalkylene-, -heteroarylene-C₁₋₅alkylene-arylene-, -heteroarylene-C₁₋₅alkylene-heteroarylene-, -heteroarylene-C₁₋₅ heteroalkylene-arylene-, and -heteroarylene-C₁₋₅heteroalkylene-heteroarylene-, in which p is an integer of 1 to 3,
   wherein the heteroalkylene or heteroarylene includes one or more heteroatom groups selected from the group consisting -NH-, -O-, -S-, -O-N=, -S(=O)-, and - S(=O)₂-, or one or more heteroatoms selected from the group consisting of O, N, and S, and
   the non-natural amino acid is linked with neighboring amino acid via 1' and 2', within or at the end of an amino acid sequence of the functional polypeptide, so that the functional polypeptide variant of this application is formed. That is, at a specific site in the amino acid sequence constituting a functional polypeptide, instead of the originally present amino acid, the non-natural amino acid is substituted or the non-natural amino acid is inserted to constitute the functional polypeptide variant.
A10. The functional polypeptide variant-lasting protein conjugate according to any one of A01 to A09, wherein
   the first click chemistry functional group is represented by any one of the following structures: 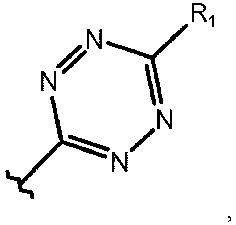 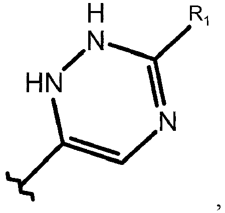 and 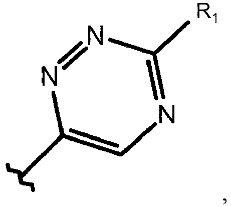
   wherein R₁ is any one selected from H, halogen, C₁₋₃ alkyl, C₃₋₆ cycloalkyl, C₃₋₆ heterocycloalkyl, aryl, and heteroaryl,
   wherein the heterocycloalkyl or heteroaryl includes one or more heteroatom groups selected from the group consisting of -NH-, -O-, -S-, -O-N=, -S(=O)-, and - S(=O)₂-, or one or more heteroatoms selected from the group consisting of O, N, and S.
A11. The functional polypeptide variant-lasting protein conjugate according to A09, wherein
   a non-natural amino acid included in the functional polypeptide variant is represented by the following structure: 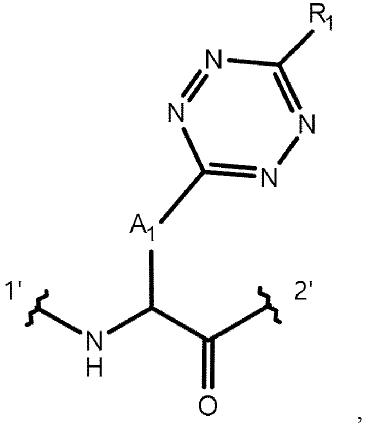
   wherein R₁ is any one selected from H, halogen, methyl, aryl, and heteroaryl.
A12. The functional polypeptide variant-lasting protein conjugate according to A09, wherein
   the non-natural amino acid included in the functional polypeptide variant is represented by the following structure: 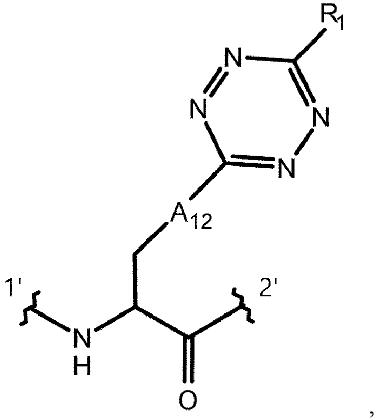
   wherein R₁ is any one selected from H, halogen, methyl, aryl, and heteroaryl.
A13. The functional polypeptide variant-long-acting protein conjugate according to A09, wherein
   the non-natural amino acid included in the functional polypeptide variant is represented by the following structure: 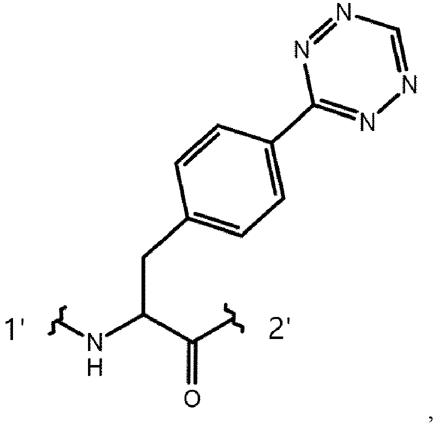 or 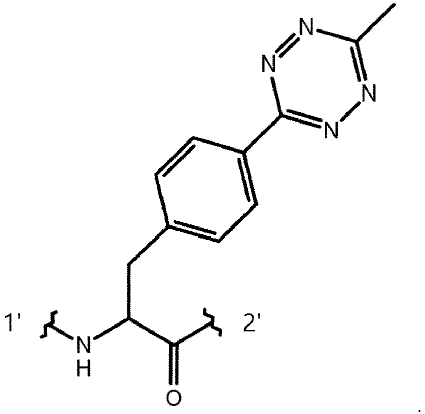
A14. The functional polypeptide variant-lasting protein conjugate according to any one of A01 to A08, wherein the second anchor moiety is derived from a linker, wherein the linker includes a second click chemistry functional group which is a dienophile group reacting with the first click chemistry functional group, and a thiol reactive group reacting with a thiol residue, and the second anchor moiety.
A15. The functional polypeptide variant-lasting protein conjugate according to A14, wherein
   the dienophile group is any one selected from the group consisting of a trans-bicyclo[6.1.0]nonene group, a trans-cyclooctene (TCO) group, a methylcyclopropene group, a bicyclo[6.1.0]nonyne group, a cyclooctyne group, a norbomene group, a cyclopentene group, and a styrene group.
A16. The functional polypeptide variant-lasting protein conjugate according to A14, wherein
   the dienophile group is a trans-cyclooctene (TCO) group.
A17. The functional polypeptide variant-lasting protein conjugate according to A14, wherein the thiol reactive group is a maleimide group, or an APN group.
A18. The functional polypeptide variant-lasting protein conjugate according to A14, wherein the thiol reactive group is represented by any one of the following structures: 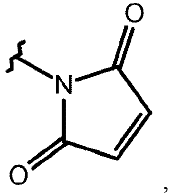 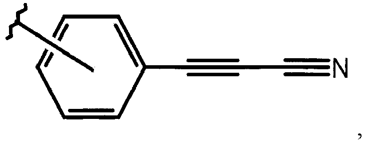 and 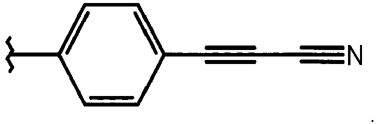
A19. The functional polypeptide variant-lasting protein conjugate according to any one of A01 to A08, wherein
   the first conjugation moiety is formed by the reaction between a tetrazine group and a trans-cyclooctene group, and
   the first conjugation moiety is any one selected from the following structures: 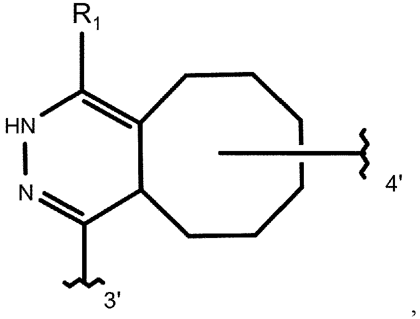 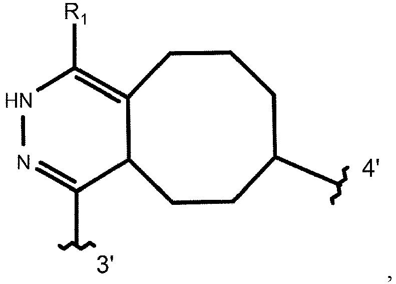 and 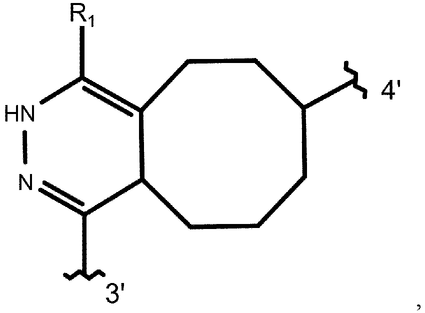
   wherein 3' is linked with the functional polypeptide variant moiety, and 4' is linked with the second anchor moiety, and
   wherein R₁ is any one selected from H, halogen, methyl, aryl, and heteroaryl.
A20. The functional polypeptide variant-lasting protein conjugate according to A19, wherein
   the first conjugation moiety is 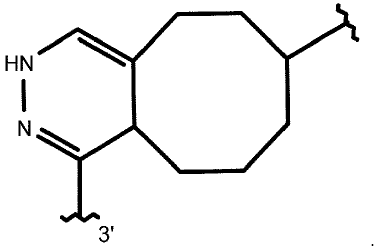
A21. The functional polypeptide variant-lasting protein conjugate according to any one of A01 to A08, wherein
   the second conjugation moiety is formed by the reaction between a thiol reactive group and a thiol residue included in cysteine of the lasting protein, and
   the second conjugation moiety is any one selected from the following structures: 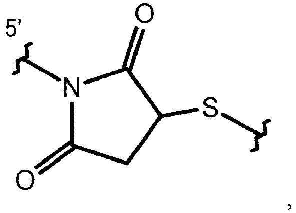 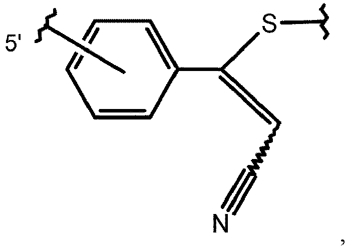 and 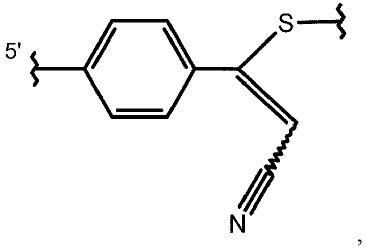
   wherein 5' is linked with a second anchor moiety, and S is derived from the thiol residue included in cysteine of the lasting protein.
A22. The functional polypeptide variant-lasting protein conjugate according to any one of A01 to A21, wherein the functional polypeptide variant is any one selected from a GLP1 variant, an hGH variant, a Uox subunit variant, and an sfGFP variant.
A23. The functional polypeptide variant-lasting protein conjugate according to A22, wherein the functional polypeptide variant is the GLP1 variant, and the GLP1 variant has any one sequence selected from the sequences of SEQ ID NOs: 1 to 26.
A24. The functional polypeptide variant-lasting protein conjugate according to A22, wherein the functional polypeptide variant is the hGH variant, and the hGH variant has a sequence in which one or more standard amino acids in sequence of SEQ ID NO: 27 are substituted with non-natural amino acid(s), wherein the standard amino acid substituted with a non-natural amino acid is any one selected from Q29, Y35, L52, E56, E66, Q69, E74, F92, S106, Q122, R127, L128, G131, R134, Q141, T142, Y143, K145, D147, N149, N152, D153, and E186.
A25. The functional polypeptide variant-lasting protein conjugate according to A22, wherein the functional polypeptide variant is the hGH variant, wherein the hGH variant has any one sequence selected from the sequences of SEQ ID NOs: 28 to 50.
A26. The functional polypeptide variant-lasting protein conjugate according to A22, wherein the functional polypeptide variant is an sfGFP variant, and the sfGFP variant has a sequence in which one or more standard amino acids in SEQ ID NO: 51 are substituted with non-natural amino acid(s), wherein the standard amino acid substituted with a non-natural amino acid is any one selected from E8, T11, V13, G26, K28, T40, N41, T51, D78, K103, K109, G118, K128, D131, D135, H150, Q159, K168, D175, D192, V195, N200, P213, and G230.
A27. The functional polypeptide variant-lasting protein conjugate according to A22, wherein the sfGFP variant has any one sequence selected from the sequences of SEQ ID NOs: 52 to 75.
A28. The functional polypeptide variant-lasting protein conjugate according to any one of A01 to A27, wherein the lasting protein is albumin or variant thereof, or an albumin-binding domain or variant thereof.
A29. The functional polypeptide variant-lasting protein conjugate according to A28, wherein the albumin or variant thereof has any one sequence of the sequences of SEQ ID NOs: 76 to 87.
A30. The functional polypeptide variant-lasting protein conjugate according to A28, wherein the albumin-binding domain or variant thereof has the sequence of SEQ ID NO: 88.
B01. A functional polypeptide variant-lasting protein conjugate represented by Formula 1-3: 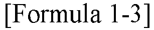 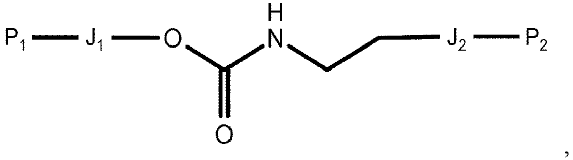 wherein
   P₁ is a functional polypeptide variant moiety, which is derived from a functional polypeptide variant, wherein the functional polypeptide variant includes a first click chemistry functional group, which is a tetrazine or derivative thereof;
   P₂ is a lasting protein moiety, which is derived from a lasting protein, wherein the lasting protein is albumin or variant thereof, or an albumin-binding domain or variant thereof, wherein the lasting protein includes cysteine which includes a thiol residue;
   J₁ is a first conjugation moiety, which is 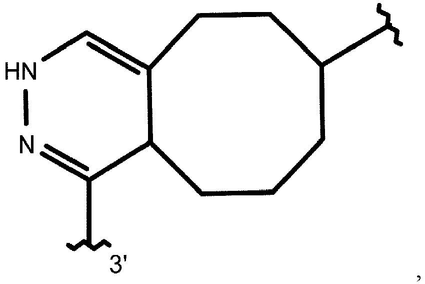 wherein
   3' is linked with P₁, and
   J₂ is a second conjugation moiety, which is 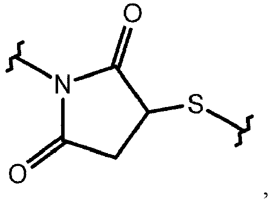 or 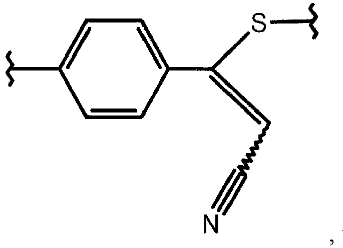 wherein
   S is derived from the thiol group of the lasting protein.
B02. The functional polypeptide variant-lasting protein conjugate according to B01, wherein
   the functional polypeptide variant is any one selected from a GLP1 variant, an hGH variant, a Uox subunit variant, and an sfGFP variant.
B03. The functional polypeptide variant-lasting protein conjugate according to B01, wherein the functional polypeptide variant is a GLP1 variant.
B04. The functional polypeptide variant-lasting protein conjugate according to B01, wherein the functional polypeptide variant is an hGH variant.
B05. The functional polypeptide variant-lasting protein conjugate according to B01, wherein the functional polypeptide variant is an sfGFP variant.
B06. The functional polypeptide variant-lasting protein conjugate according to B01, wherein the second conjugation moiety is 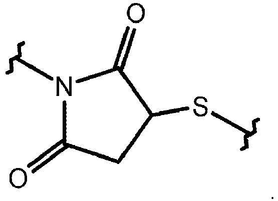
B07. The functional polypeptide variant-lasting protein conjugate according to B01, wherein the second conjugation moiety is 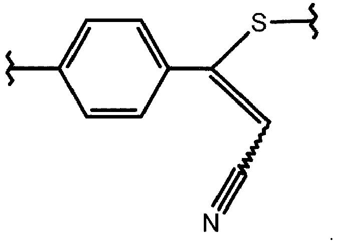
B08. The functional polypeptide variant-lasting protein conjugate according to B01, wherein the lasting protein is a human serum albumin or variant thereof.
B09. The functional polypeptide variant-long-acting protein conjugate according to B01, wherein the lasting protein is an albumin-binding domain or variant thereof.
C01. A method of preparing a functional polypeptide variant-lasting protein conjugate includes:
   mixing a first composition including a lasting protein and a second composition including a linker, wherein a first reaction composition is prepared;
   mixing the first reaction composition and a third composition including a functional polypeptide variant, wherein a second reaction composition is prepared; and
   obtaining a functional polypeptide variant-lasting protein conjugate from the second reaction composition.
C02. A method of preparing a functional polypeptide variant-long-acting protein conjugate includes:
   mixing a first composition including a lasting protein, a second composition including a linker, and a third composition including a functional polypeptide variant, wherein a first reaction composition is prepared; and
   obtaining a functional polypeptide variant-lasting protein conjugate from the first reaction composition.
C03. A method of preparing a functional polypeptide variant-lasting protein conjugate includes:
   mixing a third composition including a functional polypeptide variant, and a second composition including a linker, wherein a first reaction composition is prepared;
   mixing the first reaction composition and a first composition including a lasting protein, wherein a second reaction composition is prepared; and
   obtaining a functional polypeptide variant-lasting protein conjugate from the second reaction composition.
D01. A method of preparing a functional polypeptide variant-long-acting protein conjugate includes:
   mixing a first composition including a lasting protein and a second composition including a linker, wherein a first reaction composition is prepared;
   mixing the first reaction composition and a third composition including a partner fusion protein, wherein a second reaction composition including a partner fusion protein-lasting protein conjugate is prepared; and
   removing a partner protein from the partner fusion protein-lasting protein conjugate, wherein a functional polypeptide variant-lasting protein conjugate is obtained by the removal of the partner protein from the partner fusion protein-lasing protein conjugate.
D02. A method of preparing a functional polypeptide variant-lasting protein conjugate includes:
   mixing a first composition including a lasting protein, a second composition including a linker, and a third composition including a partner fusion protein, wherein a first reaction composition including a partner fusion protein-lasting protein variant is prepared; and
   removing the partner fusion protein from the partner fusion protein-lasting protein variant, wherein a functional polypeptide variant-lasting protein conjugate is obtained by removing the partner protein from the partner fusion protein-lasting protein conjugate.
D03. A method of preparing a functional polypeptide variant-long-acting protein conjugate includes:
   mixing a third composition including a partner fusion protein, and a second composition including a linker, wherein a first reaction composition is prepared;
   mixing the first reaction composition, and a first composition including a lasting protein, wherein a second reaction composition including a partner fusion protein-lasting protein is obtained; and
   removing a partner protein from the partner fusion protein-lasting protein conjugate, wherein a functional polypeptide variant-lasting protein conjugate is obtained by removal of the partner protein from the partner fusion protein-lasting protein conjugate.
E01. A pharmaceutical composition for treating a target disease of a subject includes any one of the functional polypeptide variant-lasting protein conjugates of A01 to A30, and B01 to B06.
E02. The pharmaceutical composition for treating the target disease of the subject according to E01, wherein
   the pharmaceutical composition includes any one of the functional polypeptide-lasting protein conjugates of A23 and B03, and
   the target disease is any one selected from diabetes, long-acting diabetes, obesity, type 2 diabetes, prediabetes, a liver disease, a metabolic disease, and a cardiovascular disease.
E03. A pharmaceutical composition for treating a target disease of a subject according to E01, wherein
   the pharmaceutical composition includes any one of the functional polypeptide-lasting protein conjugates of A24, A25, and B04, and
   the target disease is growth hormone deficiency (GHD).
E04. A composition for diagnosing albumin-requiring cancer cells of the subject, which includes any one of the functional polypeptide variant-lasting protein conjugates of A26, A27, and B05.

### [Examples]

Examples will be described below.

Information on components described in the examples is as follows:
- Human Serum Albumin (HSA, Sigma Aldrich #A3782) or rHSA (Albumedix)
- Linker 1: TCO-Maleimide (trans-cyclooct-2-ene-maleimide axially substituted, A) (MW: 292.33 g/mol; purchased from Future Chem (Seoul, KOREA))
- Linker 2: DBCO-maleimide (Dibenzocyclooctyne-PEG4-maleimide) (MW: 674.74 g/mol; purchased from Click Chemistry Tools LLC (Scottsdale, AZ, USA))
- frTet (4-(1,2,3,4-tetrazin-3-yl) phenylalanine) (purchased from Aldlab Chemicals (Woburn, MA, USA))
- AzF (Azido-L-phenylalanine) (purchased from Chem-Impex International Inc. (Wood Dale, IL, USA))
- Cy3 TCO (trans-cyclooctene) (purchased from AAT Bioquest (Sunnyvale, CA, USA))

### GLP1 variant-HSA conjugate

### 1. Preparation of GLP1 variant-HSA conjugate

The inventors of the present application prepared and identified a GLP1 variant-HSA conjugate. First, a sfGFP-GLP1 variant (partner fusion protein) was prepared, and then a sfGFP-GLP1 variant-HSA conjugate was prepared using a linker and HSA. Afterward, a GLP1 variant-HSA conjugate was prepared by removing sfGFP from the sfGFP-GLP1 variant-HSA conjugate.

### 1.1. Manufacture of strain capable of producing sfGFP-GLP1 variant (partner fusion protein)

A sfGFP-GLP1 expression vector was manufactured similarly as described in the PCT application with the application number PCT/KR2020/013348.

Specifically, as the sfGFP-GLP1 expression vector, a pBAD-sfGFP-GLP1 vector was manufactured by inserting the moiety (GGKKKKKGG) linked with the Factor Xa cleavage site (IEGR) at the C-terminus of GLP1.

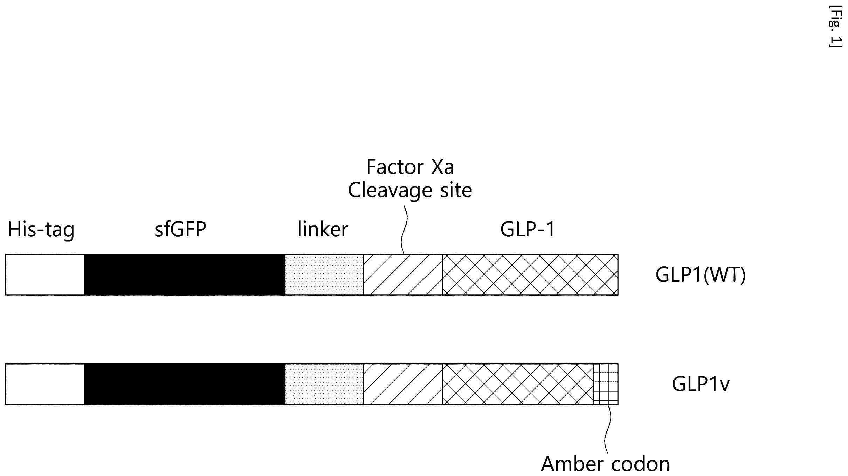

The schematic diagrams of the sfGFP-GLP1 and the sfGFP-GLP1 variant expression vector are illustrated in FIG. 1.

To site-specifically insert a non-natural amino acid, the base sequence was changed to the amber codon (TAG) at the V16, Y19, F28, and G37 positions of inactive GLP1 (V10, Y13, F22, and G31 positions in active GLP1) using a site-directed mutagenesis PCR technique. In addition, to prevent degradation by dipeptidyl peptidase IV, alanine (Ala) at position 2 in GLP1 (position 8 in inactive GLP1) was substituted with glycine (Gly) using a site-directed mutagenesis PCR technique. Here, oligonucleotide primer sequences used are listed in Table 1.

**[Table 1]**

| Primer Name | Oligonucleotide Sequence (5' → 3') | Generated Plasmid |
|---|---|---|
| V16UAG-frTET F | AAGGCACCTTTACCAGCGATTAGAGTAGCTATCTGGAAGG (SEQ ID NO: 93) | pBAD-sfGFP-GLP-1_V16UAG(frTET) |
| V16UAG-frTET_R | CCTTCCAGATAGCTACTCTAATCGCTGGTAAAGGTGCCTT (SEQ ID NO: 94) | |
| Y19UAG-frTET F | GCGATGTGAGTAGCTAGCTGGAAGGTCAGGC (SEQ ID NO: 95) | pBAD-sfGFP-GLP-1_Y19UAG(frTET) |
| Y19UAG-frTET R | GCCTGACCTTCCAGCTAGCTACTCACATCGC (SEQ ID NO: 96) | |
| F28UAG-frTet_F | GTCAGGCGGCCAAAGAATAGATTGCCTGGCTGGTGC (SEQ ID NO: 97) | pBAD-sfGFP-GLP-1_F28UAG(frTET) |
| F28UAG-frTet_R | GCACCAGCCAGGCAATCTATTCTTTGGCCGCCTGAC (SEQ ID NO: 98) | |
| G37UAG-frTet_F | GTGCGGGGGCGTTAGTAAGGTACCATATGG (SEQ ID NO: 99) | pBAD-sfGFP-GLP-1_G37UAG(frTET) |
| G37UAG-frTet_R | CCATATGGTACCTTACTAACGCCCCCGCAC (SEQ ID NO: 100) | |
| A8G_F | GCATCGAAGGTAGGCATGGTGAAGGCACCTTTACCAG (SEQ ID NO: 101) | |
| A8G_R | CTGGTAAAGGTGCCTTCACCATGCCTACCTTCGATGC (SEQ ID NO: 102) | |

One of the used pBAD-sfGFP-GLP1 variant vectors includes the sequence of SEQ ID NO: 89.

A pDule-C11RS vector, which can insert a non-natural amino acid, was provided by a research team of the Gwangju Institute of Science and Technology and used. The vector includes an frTet-specific engineered pair, which consists of *Methanococcus jannaschii-derived* tyrosyl-tRNA synthetase and amber suppressor tRNA. The provided vector was used as is without additional manipulation. Plasmids [pDule-C11RS] and [pBAD-sfGFP-GLP-1_Amb plasmid] were introduced into *E. coli* C321.A.exp (Addgene (Cambridge, MA)) to manufacture an expression strain.

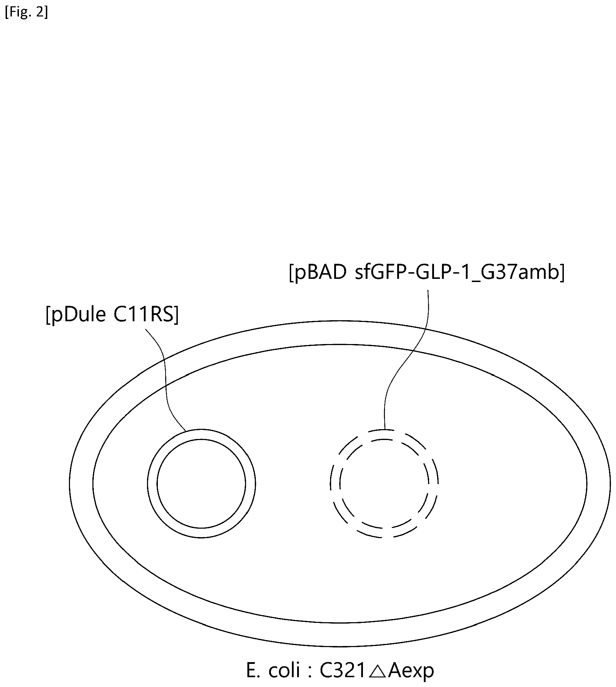

The transformed cell line C321delA.exp [pDule C11RS][pBAD sfGFP-GLP1-37Amb] for producing sfGFP-GLP1 variant is shown in FIG. 2.

### 1.2. Production of sfGFP-GLP1 variant

### (1) Production of sfGFP-GLP1 variant (partner fusion protein)

To produce sfGFP-GLP1 variant including frTet, the transformed cell line C321delA.exp [pDule C11RS][pBAD sfGFP-GLP1-37Amb] was grown in 2x YT broth (16 g/L tryptone, 10 g/L yeast extract, 5.0 g/L NaCl) containing ampicillin (100 µg/mL) and tetracycline (10 µg/mL) at 210 rpm and 37 °C for 16 hours, and then inoculated into fresh 2x YT broth to further culture under the same conditions for 3 hours. After adding frTet and L-(+)-arabinose to the culture reaching an OD (600 nm) of 0.5 at the final concentration of 1 mM (0.4%), the temperature was lowered to 25 °C, followed by inducing protein expression.

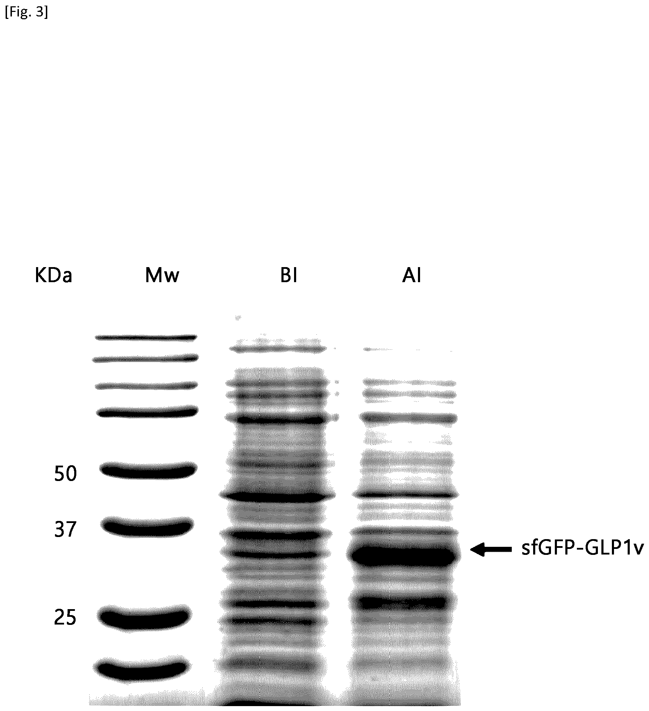

After 15 hours, the expressed protein was harvested (4 °C refrigerated centrifuge, 6000 rpm, 15 min). To confirm protein expression, a sample (non-induction sample) obtained before induction (arabinose induction) and a sample (induction sample) obtained after induction were acquired, and then SDS-PAGE was performed (FIG. 3). In the induction sample, sfGFP-GLP1 variant was observed. Here, the theoretical molecular weight of the sfGFP-GLP 1 variant is 32 kDa. In FIG. 3, BI indicates a non-induction sample, and AI indicates an induction sample. In addition, sfGFP-GLP1v indicates the sfGFP-GLP1 variant.

Subsequently, the inventors of the present application purified the sfGFP-GLP1 variant from the induction sample. To this end, immobilized ion metal affinity chromatography, which is the interaction between His-tag (polyhistidine-tag) included at the N-terminus of the sfGFP-GLP1 variant and Ni-nitrilotriacetic acid (NTA) agarose (Qiagen (Valencia, CA, USA)), was used. The sfGFP-GLP1 variant has the sequence of six consecutive histidines at the N-terminus site, and the sequence selectively binds to the Ni-NTA resin.

After the induction sample (0.8 g) was dissolved in 12 mL of lysis buffer (50 mM NaH₂PO₄ (5.99 g, MW119.98), 300 mM NaCl (17.54 g), 10 mM imidazole (0.68 g) in 1 liter), 1 mg/ml of lysozyme was added to lyse for 30 minutes. Subsequently, after sonication (150W power, 20 cycles of 10-sec lysis/20-sec resting, performed at 4 °C), centrifugation was performed at 10,000 xg for 20 minutes, Ni-NTA was added to a supernatant, and then His-tag and Ni-NTA were bound at 4 °C for 30 minutes at 300 rpm.

Afterward, a protein including a non-natural amino acid was eluted with elution buffer (50 mM NaH₂PO₄ (5.99 g, MW119.98), 300 mM NaCl (17.54 g), 250 mM imidazole (17 g) in 1 liter), thereby obtaining purified sfGFP-GLP1 variant. As the result of protein concentration measurement after elution, the concentration of the sfGFP-GLP1 variant was 14.6 µM. A purification yield was calculated by [weight of eluted protein]/[weight of used pellets], and was calculated to be 0.6%.

The purified sfGFP-GLP1 variant was desalted with PBS (pH 7.4) (NaCl (8 g), KCl (0.2 g), Na₂HPO₄ (1.44 g), KH₂PO₄ (0.24 g) in 1 liter) using a PD-10 column (PD-10 column packed with Sephadex G-25 resin, Cytiva).

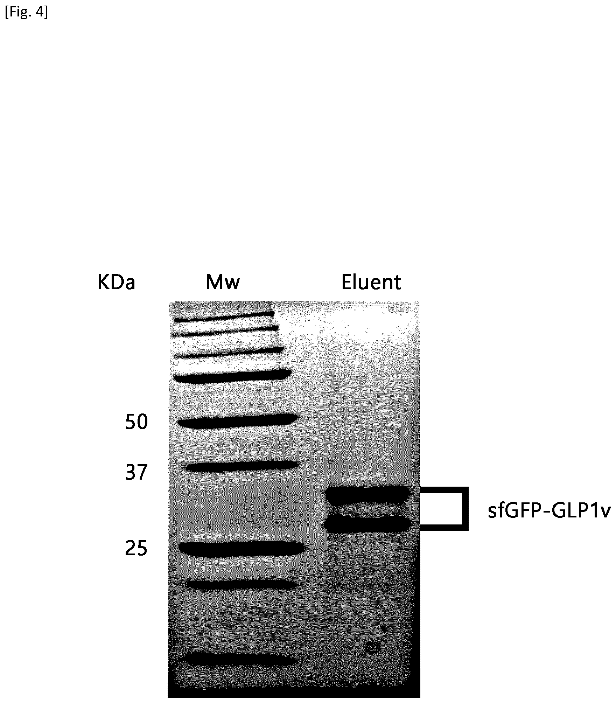

The result of analyzing the purified sfGFP-GLP1 variant through SDS-PAGE is shown in FIG. 4. Here, Eluent indicates a sample including the eluted sfGFP-GLP1 variant. Here, due to the charge difference caused by the difference in the oxidized state of the protein, sfGFP-GLP1 variants were observed at two molecular weights (Mørtz, Ejvind, et al. "Mass spectrometric characterization of glycosylated interferon-γ variants separated by gel electrophoresis." Electrophoresis 17.5 (1996): 925-931.).

### (2) Confirmation of frTet introduction

The inventors of the present application checked whether frTet was introduced into the sfGFP-GLP1 variant.

To confirm the included frTet, IEDDA reactivity was confirmed, and to this end, fluorescent labeling analysis was performed.

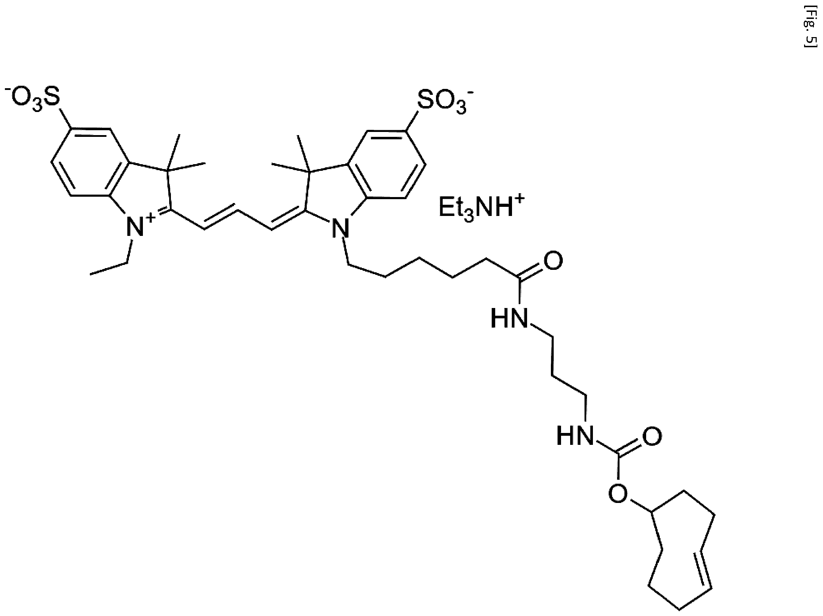

For fluorescent labeling analysis, the sfGFP-GLP1 variant was reacted with Cy3 TCO in a reaction ratio of 4:5 (final concentration - 20 µM : 100 µM) in PBS (pH 7.4) at room temperature for 2 hours. Cy3 TCO is shown in FIG. 5.

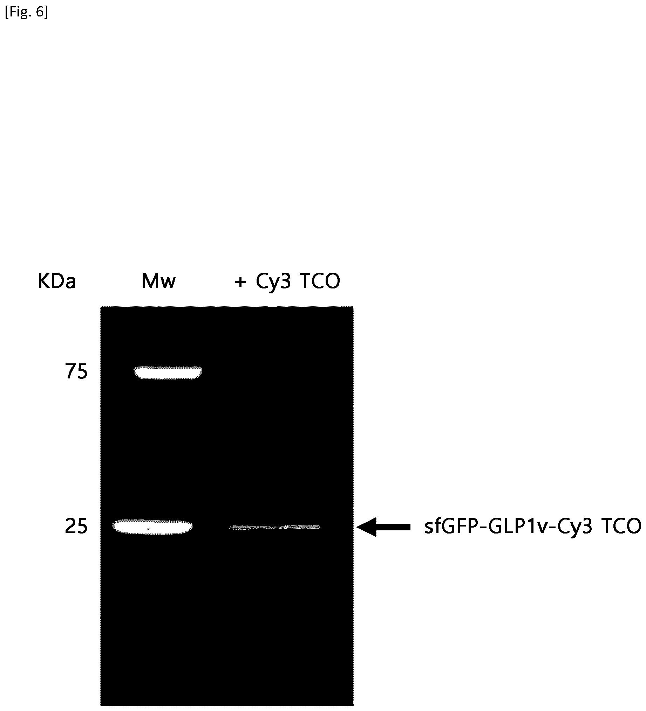

The reaction product was analyzed by SDS-PAGE to confirm the conjugation of Cy3 TCO. The analysis result is shown in FIG. 6. Here, +Cy3 TCO indicates a sample subjected to Cy3 TCO conjugation. As shown in FIG. 6, sfGFP-GLP1v-Cy3 TCO in which the sfGFP-GLP1 variant and the Cy3 TCO are conjugated is confirmed.

### 1.3. Preparation of GLP1 variant-HSA conjugate

The inventors of the present application prepared a sfGFP-GLP1 variant-HSA conjugate using the prepared sfGFP-GLP1 variant, linker1 (compound of Formula 2-5), and HSA, and sfGFP was removed therefrom, thereby preparing a GLP1 variant-HSA conjugate.

This conjugate is described in detail below.

### (1) Preparation of sfGFP-GLP1 variant-HSA conjugate (partner fusion protein-lasting protein conjugate)

### 1) Preparation of HSA-linker1 complex

First, the inventors of the present application prepared an HSA-linker1 complex by reacting linker1 and HSA. Here, a maleimide group at one end of linker1 reacts with a free thiol group included in cysteine 34 of HSA to form a covalent bond.

To prepare the HSA-linker1 complex, linker1 was reacted with HSA in 20 mM Bis-Tris buffer at a molar ratio of 4:1 (final concentration - 200 µM : 50 µM) for 3 hours at room temperature in the same manner as disclosed in the prior literature (Bak, Mijeong, et al. "Recombinant Peptide Production Platform Coupled with Site-Specific Albumin Conjugation Enables a Convenient Production of Long-Acting Therapeutic Peptide." Pharmaceutics 12.4 (2020): 364).

The HSA-linker1 complex generated after the reaction was desalted using a PD-10 column and 20 mM Tris pH 8.0 buffer to conjugate with the sfGFP-GLP1 variant.

### 2) Preparation of sfGFP-GLP1 variant-HSA conjugate

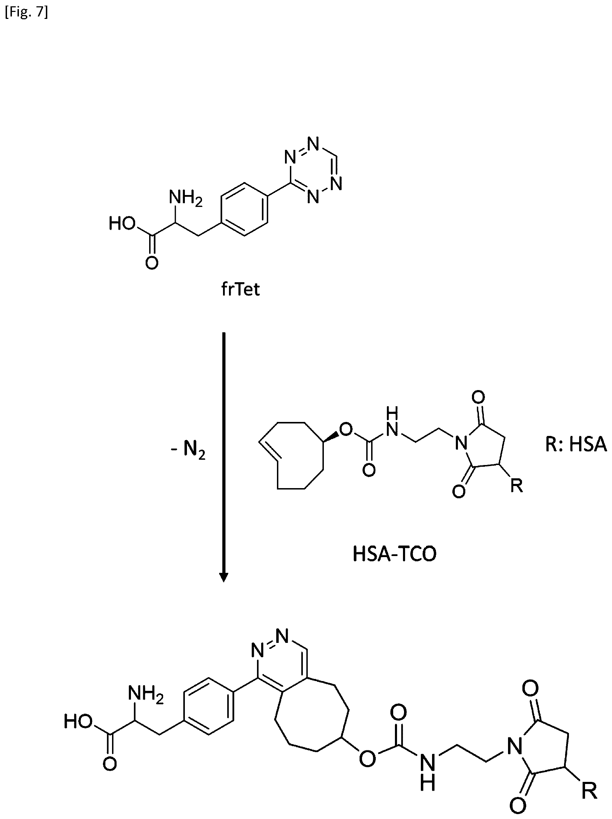

A sfGFP-GLP1 variant-HSA conjugate was prepared by the reaction of the sfGFP-GLP1 variant and the HSA-linker1 complex. Here, the sfGFP-GLP1 variant-HSA conjugate is prepared by the IEDDA reaction between a tetrazine group included in the part of the GLP1 variant of the sfGFP-GLP1 variant and a TCO group at one end of the HSA-linker1 complex. The reaction between a non-natural amino acid frTet and the HSA-linker1 complex is illustrated in FIG. 7. The reaction between the sfGFP-GLP1 variant including frTet and the HSA-linker1 complex also occurs in a similar manner to the above reaction.

To prepare the sfGFP-GLP1 variant-HSA conjugate, the reaction between the sfGFP-GLP1 variant and the HSA-linker1 complex occurred at a molar ratio of 1:2 (final concentration - 20 µM : 40 µM) for 16 hours at room temperature.

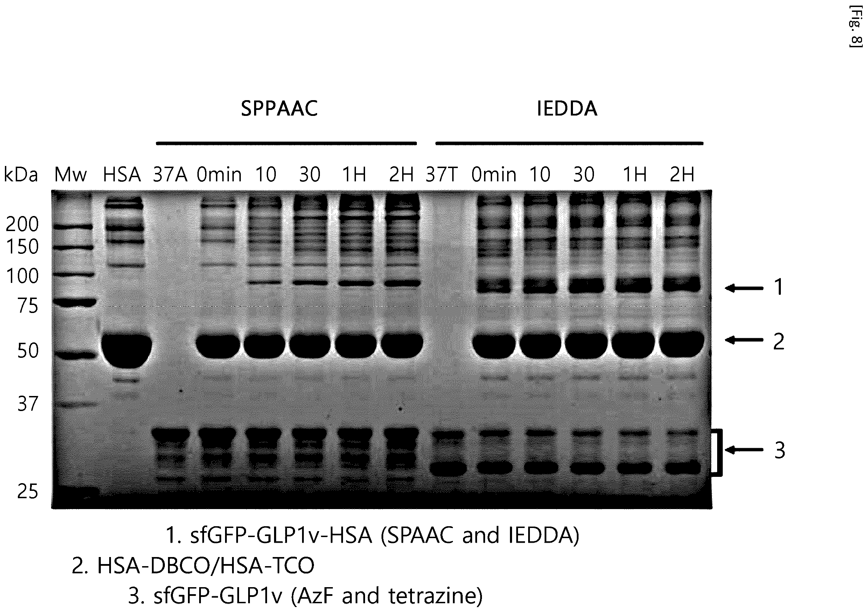

Afterward, the preparation of the sfGFP-GLP1 variant-HSA conjugate was confirmed through SDS-PAGE. This is shown in FIG. 8. FIG. 8 shows the SDS-PAGE result of samples obtained 0 minutes, 10 minutes, 30 minutes, 1 hour, and 2 hours after reacting the sfGFP-GLP1 variant and the HSA-linker1 complex.

In FIG. 8, the line indicated by IEDDA indicates the sfGFP-GLP1 variant-HSA conjugate (sfGFP-GLP1 variant-HSA conjugate).

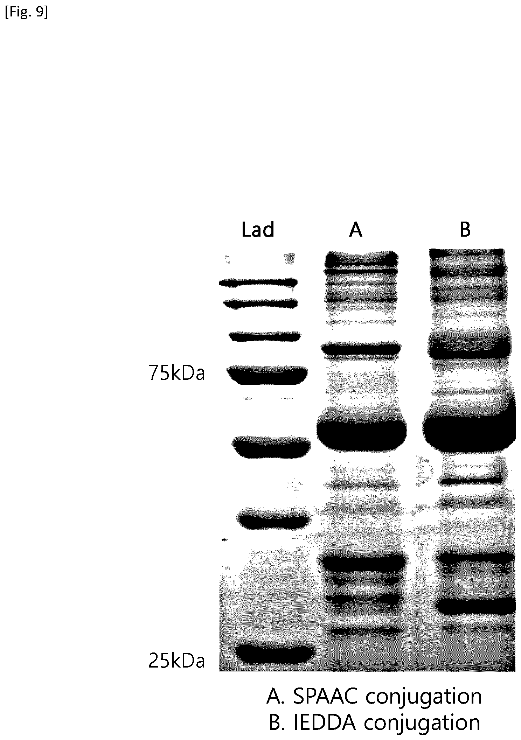

The result for a sfGFP-GLP1 variant-HSA conjugate sample obtained 16 hours after reacting the sfGFP-GLP1 variant and the HSA-linker1 complex is shown in FIG. 9. Here, the B line indicates a sfGFP-GLP1 variant-HSA conjugate formed by the IEDDA reaction.

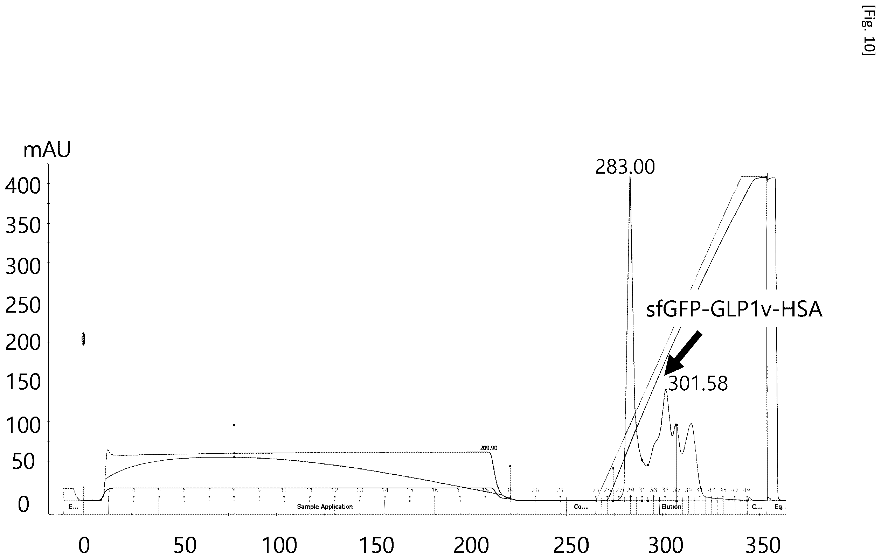

The inventors of the present application prepared a sfGFP-GLP1 variant-HSA conjugate and then purified it. To purify the sfGFP-GLP1 variant-HSA conjugate from the reaction product of the sfGFP-GLP1 variant and the HSA-linker1 complex, cation exchange chromatography was performed. Each reaction product was exchanged with a buffer (20 mM sodium phosphate, pH 6.0), and injected into a HiTrap SP-HP column to separate the sfGFP-GLP-1-HSA conjugate. The purification result is shown in FIG. 10.

### (2) Removal of sfGFP

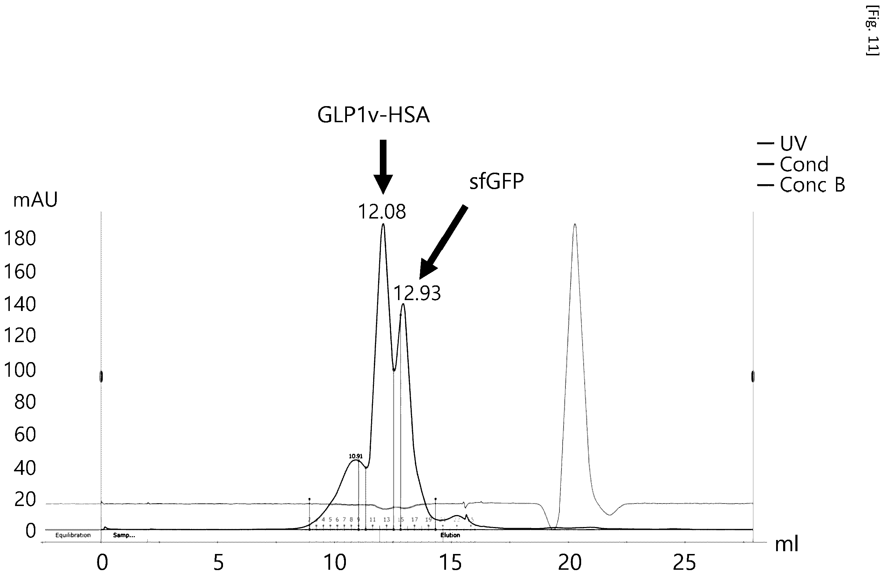

The inventors of the present application obtained a GLP1 variant-HSA conjugate by removing sfGFP from the purified sfGFP-GLP1 variant-HSA conjugate. To remove the sfGFP from the sfGFP-GLP1 variant-HSA conjugate, the Factor Xa (NEB, P8010L) enzyme was treated. Factor Xa serves to recognize a Factor Xa cleavage site (IEGR) site and cleave the site. The sfGFP-GLP1 variant-HSA conjugate was exchanged with Factor Xa reaction buffer (20 mM Tris, 2 mM CaCl2, 10 mM NaCl, pH 8.0), and concentrated to 10 µM or more. Afterward, the sfGFP-GLP-1-HSA conjugate and the Factor Xa enzyme were reacted in a ratio of 1:250 to 1:500 (w/w) at room temperature for 20 hours or more. After the reaction, the resulting product was exchanged with 20 mM Bis-Tris (pH 6.0) buffer, and the GLP1 variant-HSA conjugate and the sfGFP were separated using anion exchange chromatography (Q-HP). To increase the purity of the GLP1 variant-HSA conjugate, size exclusion chromatography (SEC) was used for separation and purification. An approximate 70 kDa GLP1 variant-HSA conjugate was obtained from an approximate 99 kDa sfGFP-GLP1 variant-HSA conjugate. The size exclusion chromatography result is shown in FIG. 11. Here, GLP1v-HSA indicates the GLP1 variant-HSA conjugate.

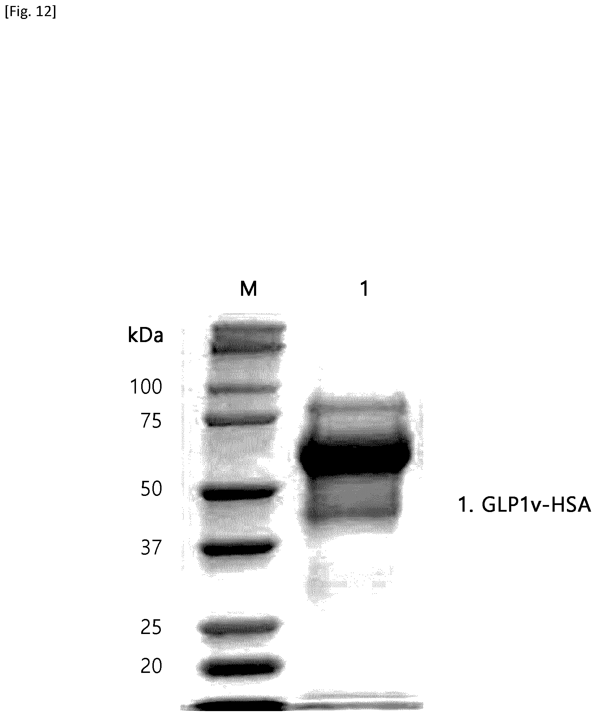

Data confirming the preparation of the GLP1 variant-HSA conjugate through SDS-PAGE is shown in FIG. 12. Here, GLP1v-HSA indicates the GLP1 variant-HSA conjugate.

### 2. Comparison between yields of preparation of albumin conjugate using SPAAC reaction and preparation of albumin conjugate prepared by IEDDA reaction

The inventors of the present application prepared a GLP1 variant-HSA conjugate using an SPAAC reaction in a similar manner to the preparation of a GLP1 variant-HSA conjugate using an IEDDA reaction. Here, the conjugate prepared using the SPAAC reaction is referred to as GLP1 variant2-HSA conjugate or GLP1 variant-HSA conjugate (SPAAC).

The GLP1 variant2-HSA conjugate was prepared in a similar manner to the GLP1 variant-HSA conjugate prepared by the IEDDA reaction except that GLP1 variant2 and linker2 were used. In the preparation of the GLP1 variant2-HSA conjugate, GLP1 variant2 into which a non-natural amino acid AzF was introduced; linker2 including DBCO at one end and maleimide at the other end; and HSA were used. Here, DBCO of the linker and an azide included in AzF were subjected to a SPAAC reaction.

The preparation of GLP1 variant2 is described below.

To introduce a non-natural amino acid, p-azido phenylalanine (AzF), including a bioorthogonal chemical handle at G37 of inactive GLP1, site-directed mutagenic PCR was performed using pQE80-sfGFP-GLP1 as a template so that a codon encoding a standard amino acid was replaced with the amber codon (UAG). 5'-CTGCAGGTCGACTTACTAACGCCCCCGCACCAG-3' (SEQ ID NO: 103) and 5'-CTGGTGCGGGGGCGTTAGTAAGTCGACCTGCAG-3' (SEQ ID NO: 104) primers were used.

To produce AzF-introduced GLP1 (sfGFP-GLP1 variant2), C321delA.exp [pEVOL-pAzF][pQE80 sfGFP-GLP1-37Amb] cells were prepared similarly as described in the prior literature (Bak, Mij eong, et al. "Recombinant Peptide Production Platform Coupled with Site-Specific Albumin Conjugation Enables a Convenient Production of Long-Acting Therapeutic Peptide." Pharmaceutics 12.4 (2020): 364).

To produce AzF-introduced GLP1 variant (sfGFP-GLP1 variant2), a transformed cell line C321delA.exp [pEVOL-pAzF][pQE80 sfGFP-GLP1-37Amb] was grown in 2x YT broth (16 g/L tryptone, 10 g/L yeast extract, 5.0 g/L NaCl) including ampicillin (100 µg/mL) and chloramphenicol (25 µg/mL) at 210 rpm and 37 °C for 16 hours, and inoculated into fresh 2x YT broth, and further cultured for 2.5 hours under the same conditions.

After AzF having the final concentration of 1 mM was added to the culture medium whose optical density (OD at 600 nm, using UV-Vis spectrometer) reached 0.4, each of IPTG and L-(+)-arabinose was added to the culture medium that reached OD 0.5 to a final concentration of 1 mM (0.2%), a temperature was lowered to 25 °C, and then protein expression was induced. Proteins expressed after 15 hours were harvested (4 °C refrigerated centrifuge, 6000 rpm, 15 min).

Subsequently, the prepared sfGFP-GLP1 variant2 was purified in the similar manner to that described above for the sfGFP-GLP1 variant. As the result of protein concentration measurement after elution, the concentration of sfGFP-GLP1 variant2 was 41.2 µM. Here, a purification yield was calculated by [weight of eluted protein]/[weight of used pellets], and calculated to be 1.95%.

Afterward, the inventors of the present application prepared a linker2-HSA complex by bonding linker2 and HSA. The maleimide group of linker2 was reacted with a free thiol group of cysteine 34 of HSA to form a covalent bond.

As described above, linker 2 and HSA were reacted in 20 mM Bis-Tris buffer at a molar ratio of 4:1 (final concentration - 200 µM : 50 µM) for 3 hours at room temperature.

After the reaction, the generated linker2-HSA complex was desalted with a PD-10 column and 20 mM Tris pH 8.0 buffer.

To prepare a sfGFP-GLP1 variant2-HSA conjugate through SPAAC, sfGFP-GLP1 variant2 and linker 2 were reacted at a molar ratio of 1:2 (final concentration -: 20 µM : 40 µM) at room temperature for 16 hours.

To analyze an albumin conjugation yield over time through SPAAC/IEDDA, SDS-PAGE was performed at 0 minutes (reaction start point), 10 minutes, 30 minutes, 1 hour and 2 hours. The result is shown in FIG. 8.

As a comparison result after obtaining samples during the progression of protein conjugation from 0 minutes to 2 hours through SDS-PAGE, it was confirmed that an albumin conjugation yield obtained by IEDDA (sfGFP-GLP1 variant 1-HSA conjugate preparation yield; 10.1%) is approximately 2.1-fold higher than that by SPAAC (sfGFP-GLP1 variant2-HSA conjugate preparation yield; 4.83%) (FIG. 8).

Accordingly, it was confirmed that the IEDDA click chemistry reaction proceeded at a higher rate in the initial reaction time period compared to the SPAAC click chemical reaction.

In addition, after reaction for 16 hours, as a result of analyzing a protein conjugation sample obtained through SDS-PAGE, it can be confirmed that the proportion of the albumin conjugate obtained by IEDDA was 2.58-fold larger than that of the albumin conjugate obtained by SPAAC (FIG. 9). Here, the A line indicates the result of the albumin conjugate obtained by SPAAC (sfGFP-GLP1 variant2-HSA conjugate), and the B line indicates the result of the albumin conjugate obtained by IEDDA (sfGFP-GLP1 variant-HSA conjugate).

In addition, the inventors of the present application compared the conjugation yields of the SPAAC and IEDDA reactions through size exclusion chromatography.

The inventors of the present application performed quantitative analysis through size exclusion chromatography (SEC) to compare quantitatively the binding yields of samples obtained by reacting human serum albumin, and sfGFP-GLP1 variant2 (sfGFP-GLP1-AzF) and sfGFP-GLP1 variant (sfGFP-GLP1-frTet) through SPAAC and IEDDA at room temperature for 16 hours, respectively.

For chromatography, a Superdex 200 10/300 GL size exclusion column of the AKTAprime plus (GE Healthcare; Little Chalfont, Buckinghamshire, U.K.) system was used. 200 µL of each sample whose reaction had completed was injected, and a flow rate of mobile phase was 0.1 ml/min (in PBS (pH 7.4)).

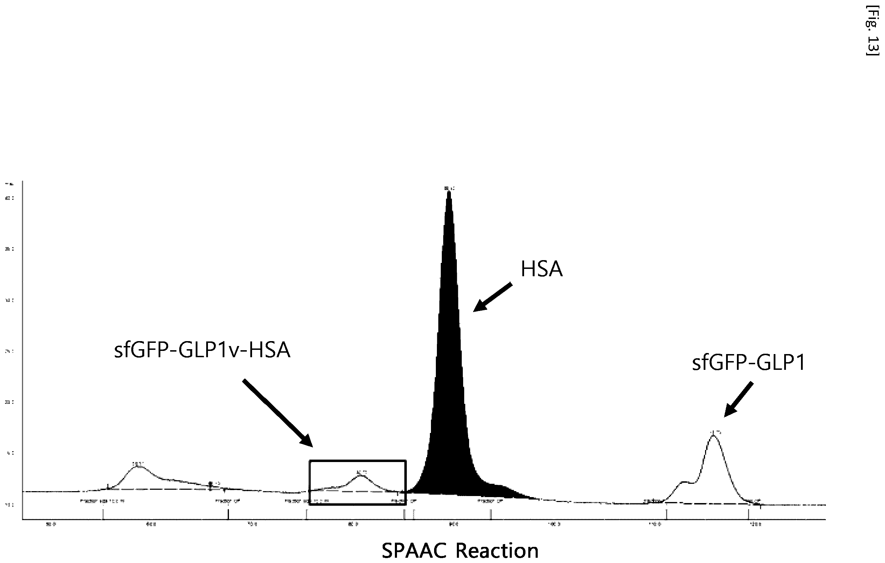

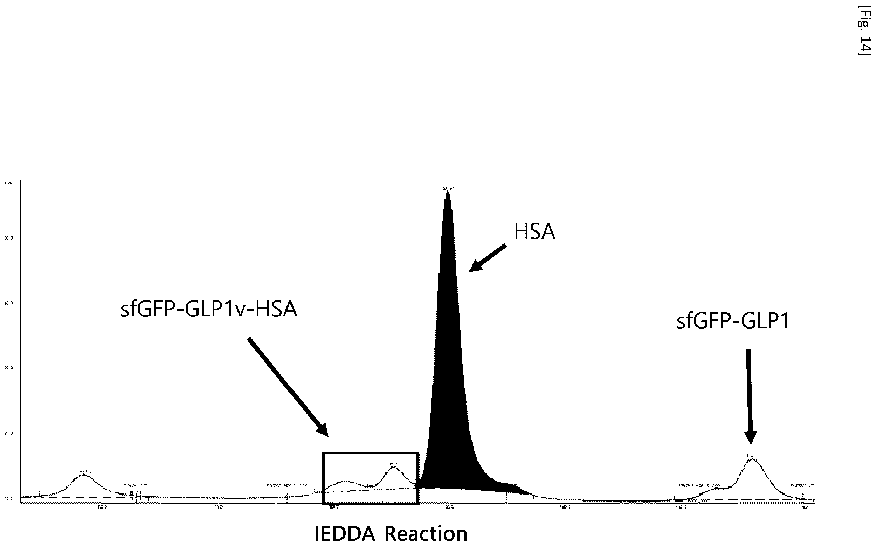

As a result of comparing chromatograms of the conjugates generated by the SPAAC click chemistry reaction and the IEDDA click chemistry reaction, it was confirmed that the binding yield for SPAAC was approximately 18%, and the binding yield for IEDDA was 33%, which is approximately 2-fold higher (FIGS. 13 and 14). The yields were calculated with a peak area produced in the chromatograms.

Therefore, it can be seen that the preparation yield of a GLP1 variant-HSA conjugate prepared by the IEDDA reaction is higher than that of the conjugate prepared by the SPAAC reaction.

### 4. In vitro activity of GLP1 variant-HSA conjugate

The *in vitro* activity of the GLP1 variant-HSA conjugate is evaluated.

The *in vitro* activity of the GLP1 variant-HSA conjugate is evaluated by a similar method to that disclosed in the literature entitled "Effect of C-terminus Conjugation via Different Conjugation Chemistries on *In Vivo* Activity of Albumin-Conjugated Recombinant GLP-1" (Park, Junyong, et al. "Effect of C-terminus Conjugation via Different Conjugation Chemistries on In Vivo Activity of Albumin-Conjugated Recombinant GLP-1." Pharmaceutics 13.2 (2021): 263).

Specifically, a method of evaluating the *in vitro* activity of the GLP1 variant-HSA conjugate is as follows.

The *in vitro* activity of the GLP1 variant-HSA conjugate may be measured by analyzing the amount of cAMP generated using HEK293 cells in which a GLP1 receptor (GLP1R) is highly expressed. The cells is added to a 48-well plate so as to become 5×10⁴ cells/well, and cultured for 16 hours by adding Iscove's modified Dulbeco's medium (10% fetal bovine serum and 1% antibiotic-antimycotic), and GLP1R_tango (Addgene, Cambridge, MA, USA) plasmids is transfected using a DNA transfection reagent (X-tremeGENE^{™} HP DNA transfection reagent). After 48 hours, GLP-1-HSA is treated at various concentrations, and cAMP is measured using a cAMP parameter assay kit (R&D Systems, Minneapolis, MN, USA).

### 5. In vivo activity of GLP1 variant-HSA conjugate

The *in vivo* activity of the GLP1 variant-HSA conjugate is evaluated.

A method of evaluating the *in vivo* activity of the GLP1 variant-HSA conjugate is as follows.

The GLP1 variant-HSA conjugate is subcutaneously injected into C57BL6/J female mice (n=6), and the change in blood glucose over time is analyzed. More specifically, 6-week-old C57BL6/J mice is acclimated for 1 week and fasted for 15 hours before the administration of an experimental material, and then a GLP1 variant-HSA conjugate is subcutaneously administered at 50 nmol/kg. As a comparative group, liraglutide (Saxenda, Novo Nordisk) is administered, and the results is compared. One hour after drug administration, glucose (2.0 g/kg dose) is intraperitoneally administered. After administration, blood is collected from a mouse tail to measure blood glucose using a glucose tester (Accu-check, Roche), thereby evaluating the *in vivo* activity of the GLP1 variant-HSA conjugate.

### 6. Pharmacokinetics (PK) of GLP1 variant-HSA conjugate

For pharmacokinetic evaluation of the GLP1 variant-HSA conjugate, a 6xhis-sfGFP-GLP1 variant-HSA conjugate was administered to 7-week-old female ICR mice (n=5). 10 nmol/kg of the 6xhis-sfGFP-GLP1 variant-HSA conjugate was intravenously injected into the tail, and blood was collected at 0.5, 4, 8, 12, and 24 hours after injection and centrifuged at 2,500 rpm for 10 minutes and then stored at - 20 °C. As a comparative group, liraglutide (Saxenda, Novo Nordisk) was administered, and the results were compared. The 6xhis-sfGFP-GLP1variant-HSA conjugate in the blood was analyzed by Sandwich ELISA. An immunoplate was coated with a polyclonal anti-albumin rabbit antibody at 4°C overnight, and the reaction was blocked using 5% skim milk at 37 °C for 1 hour. The collected blood sample was diluted on the washed plate and left for 2 hours, and treated with a monoclonal anti-6x his mouse antibody at room temperature for 2 hours. The plate was treated with anti-mouse IgG antibody-horseradish peroxidase (HRP) at room temperature for 1 hour, treated with 100 µL of a substrate for 5 minutes, treated with 2N H₂SO₄ to stop the reaction. Afterward, an optical density was measured at 450 nm using a microplate reader (Hidex Sense, Finland).

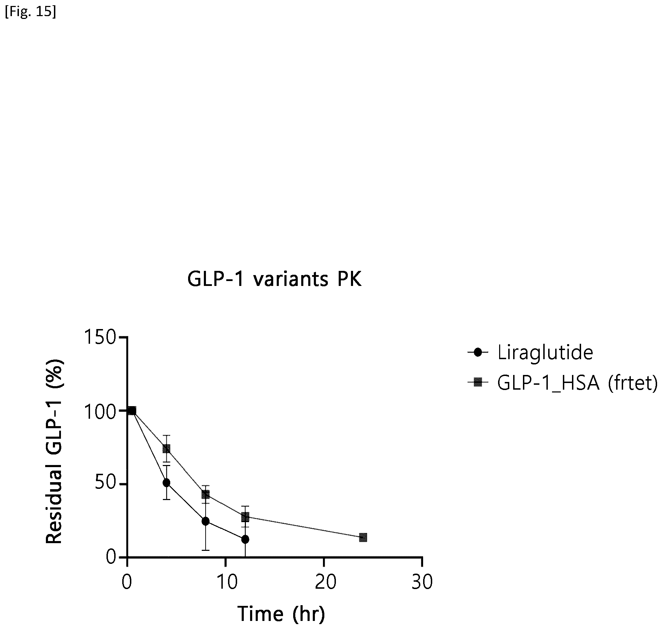

The PK analysis result of the 6xhis-sfGFP-GLP 1 variant-HSA conjugate is shown in FIG. 15. Here, in FIG. 15, GLP-1-HSA (frTet) indicates the 6xhis-sfGFP-GLP1v-has conjugate.

Regarding the result of the half-life for the PK analysis, the half-life of the control liraglutide was 3.92 hr, the half-life of the 6xhis-sfGFP-GLP1 variant-HSA conjugate was 10.9 hr. The half-life of the albumin-binding 6xhis-sfGFP-GLP1 variant-HSA conjugate was 2.8-fold higher than that of the control liraglutide.

### hGH variant-HSA conjugate

### 1. Preparation of hGH variant-HSA conjugate

### 1.1 Preparation of sfGFP-hGH variant (partner fusion protein)

### (1) Strain capable of producing sfGFP-hGH variant (partner fusion protein)

When hGH undergoes a protein reaction in *E. coli,* many of its own aggregate are formed, so the solubility in buffer is very low. Accordingly, to increase the solubility of hGH, after genetically combining the highly soluble sfGFP protein, the gene was introduced to construct a recombinant plasmid (sfGFP-hGH variant).

Screening of the introduction position of frTet in hGH was performed by a combination of point mutation and an energy scoring function using molecular modeling software (ROSETTA design) using an hGH structure (PDB ID: 1hgu) as a template.

In a 3D energy field space, after point mutation with tyrosine or tryptophan which has most similar structural and hydrophobic characteristics to frTet, the overall change of energy of the protein was calculated. Through this, the amino acid sites were selected so that they were most similar in energy to hGH-WT.

The sites of amino acids 52 and 141 of hGH, such as leucine (L), glutamine (Q) were selected to introduce a non-natural amino acid frTet, including a bioorthogonal chemical handle.

To introduce frTet into hGH, amino acids 52 and 141, leucine and glutamine, were substituted with the amber codon (UAG) through site-directed mutagenic PCR.
pTac-sfGFP-hGH plasmid was used as a template, and
5'-GTTCTCCACGTACCGGTCAGATTTTTAAATAGACCTATTCTAAATTT-3' (SEQ ID NO: 105) and
5'-AAATTTAGAATAGGTCTATTTAAAAATCTGACCGGTACGTGGAGAAC-3' (SEQ ID NO: 106) primers were used to manufacture a pTac-sfGFP-hGH-141Amb plasmid. In the same manner, sfGFP-hGH-52Amb was manufactured. Here, the pTac-sfGFP-hGH-141Amb plasmid includes the sequence of SEQ ID NO: 91, and the sfGFP-hGH-52Amb plasmid includes the sequence of SEQ ID NO: 90.

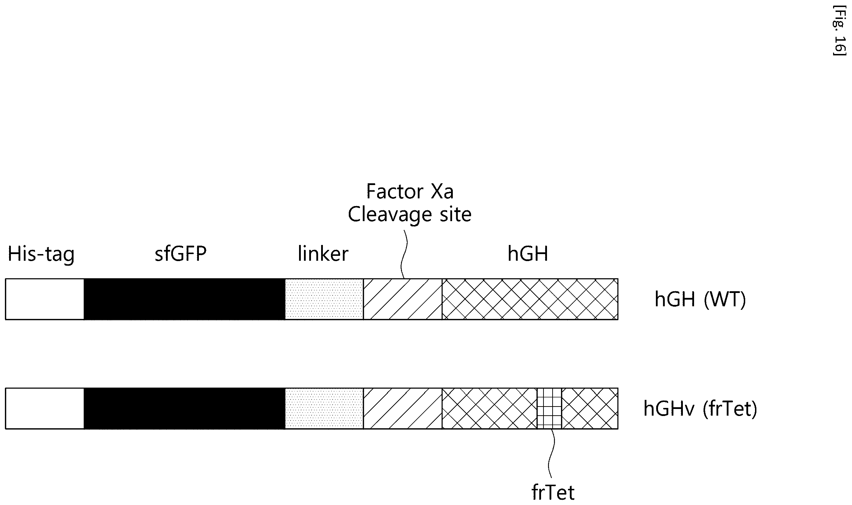

The schematic diagram of the gene sequence of sfGFP-hGH variant in which frTet is introduced at a specific position (e.g., position 52 or 141) of the amino acid sequence and the gene sequence (hGH-WT) of wild-type hGH genetically recombined with sfGFP is shown in FIG. 16.

To express the sfGFP-hGH variant in which a non-natural amino acid frTet is introduced, the manufactured pTac-sfGFP-hGH-141Amb was introduced into C321delA.exp [pDule C11RS] cells, thereby manufacturing C321delA.exp [pDule C11RS][pTac-sfGFP-hGH-141Amb] cells. Korean Patent Application No. 10-2021-0089516 was referenced.

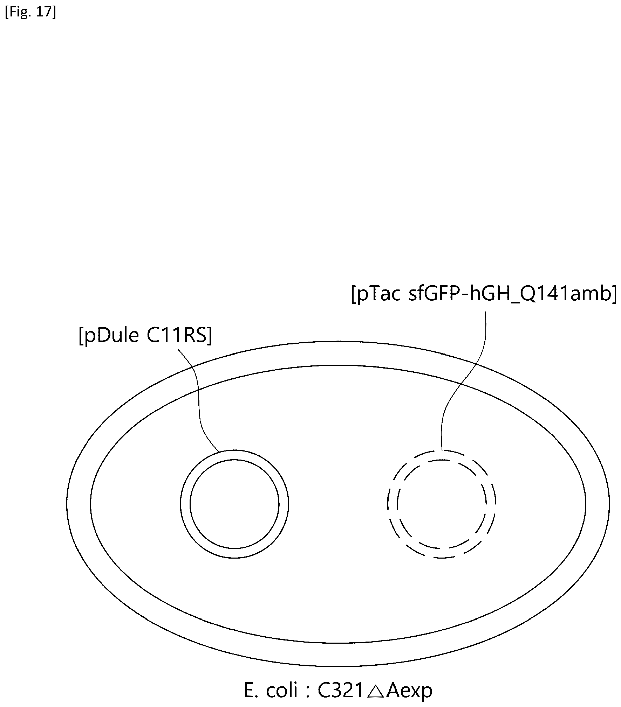

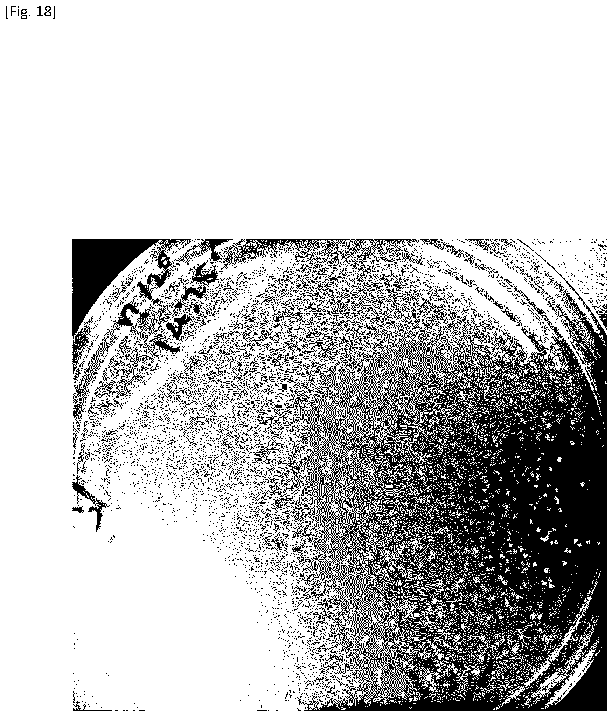

The schematic diagram of C321delA.exp [pDule C11RS][pTac-sfGFP-hGH-141Amb] is shown in FIG. 17. In addition, the result of confirming the transformed C321delA.exp [pDule C11RS][pTac-sfGFP-hGH-141Amb] cell colony is shown in FIG. 18.

### (2) Production of sfGFP-hGH variant

To produce sfGFP-hGH variant in which frTet is introduced into hGH, a transformed cell line C321delA.exp [pDule C11RS][pTac sfGFP-hGH_141amb] was grown in 2x YT broth (16 g/L tryptone, 10 g/L yeast extract, 5.0 g/L NaCl) containing tetracycline (10 µg/mL) and kanamycin (50 µg/mL) at 210 rpm and 37 °C for 16 hours, and then inoculated into fresh 2x YT broth, followed by further culture under the same conditions for 2.5 hours.

When an OD value reached 0.4 by measurement using a UV-Vis spectrometer, each of frTet and L-(+)-arabinose was input to the culture to the final concentration of 1 mM and 0.2%, and then when an OD value reached 0.6, IPTG was added to the culture to a final concentration of 1 mM, and a culturing temperature was lowered to 25 °C. After 16 hours, the expressed protein was harvested (centrifuged at 4 °C and 10000 rpm for 10 minutes).

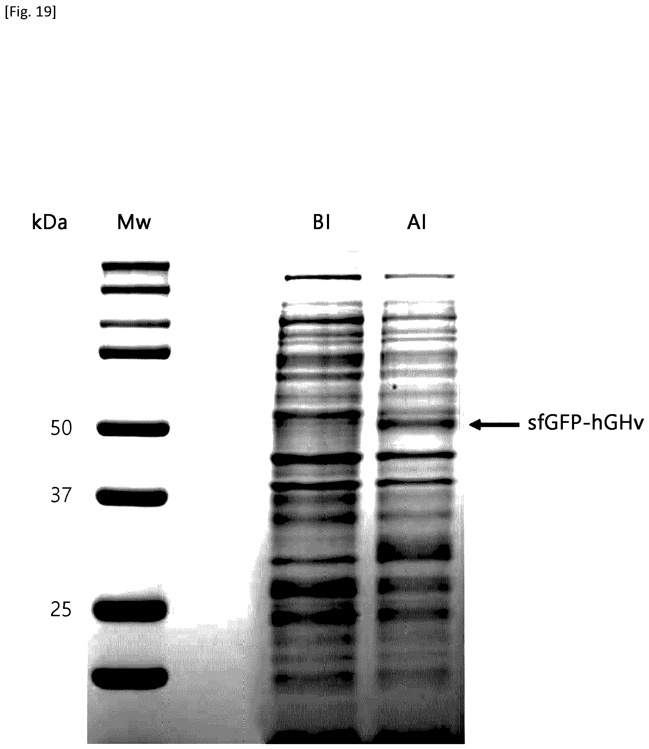

To confirm protein expression, a sample before induction and a sample after induction were acquired and subjected to SDS-PAGE, a band corresponding to the theoretical molecular weight of 50 kDa of sfGFP-hGH was identified in the lane after induction. This result is shown in FIG. 19. In FIG. 19, BI indicates the sample obtained before induction, and AI indicates the sample obtained after induction. sfGFP-hGHv indicates the sfGFP-hGH variant.

A sample including the sfGFP-hGH variant was acquired, and the inventors of the present application purified it. Here, the purification was performed through affinity chromatography using the binding strength between His-tag at the N-terminus of the sfGFP-hGH variant and an Ni-NTA resin.

The acquired sfGFP-hGH variant sample was lysed in a lysis buffer (50 mM NaH₂PO₄ (5.99 g, MW119.98), 300 mM NaCl (17.54 g) 10 mM imidazole (0.68 g) in 1 liter), and 1 mg/mL of lysozyme was added to lyse the sample for 30 minutes. Afterward, after sonication (150 W output, 20 cycles of 10-sec lysis/20-sec resting, 4 °C), centrifugation was performed at 10,000xg for 20 minutes, and Ni-NTA was added to a supernatant, followed by binding His-Tag with Ni-NTA at 4 °C for 30 minutes at 300 rpm.

Afterward, a protein in which a non-natural amino acid had been introduced was eluted with an elution buffer (50 mM NaH₂PO₄ (5.99 g, MW119.98), 300 mM NaCl (17.54 g), and 250 mM imidazole (17g) in 1 liter).

A eluted purified protein (eluent) was desalted with PBS (pH 7.4; NaCl(8g), KCl(0.2g), Na₂HPO₄ (1.44g), KH₂PO₄ (0.24g) in 1 liter) using a PD-10 column (Sephadex G-25 resin-packaged PD-10 column, purchased from Cytiva).

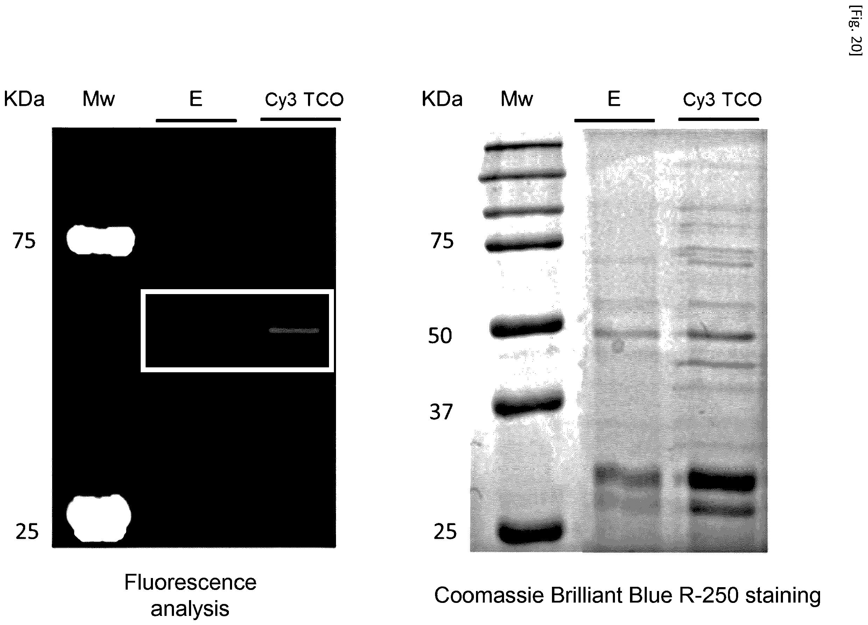

The eluted sfGFP-hGH variant sample was analyzed through SDS-PAGE. The result is shown in the right side of FIG. 20. From the SDS-PAGE result, a band corresponding to a molecular weight of 50 kDa was confirmed. The reason why an additional band was detected in the range of 25 to 37 kDa is that the amino acid sequence between sfGFP and hGH is randomly cleaved by an intracellular protease of *E. coli*, and it is presumed that the cleaved sfGFP protein region is shown by SDS-PAGE.

Subsequently, the inventors of the present application conducted fluorescent labeling analysis for confirming whether frTet introduced into the purified and separated sfGFP-hGH variant has IEDDA reactivity.

For fluorescent labeling analysis, the sfGFP-hGH variant and cyanin 3 (Cy3) trans-cyclooctene (Cy3 TCO), and AAT Bioquest (Sunnyvale, CA, USA) were reacted at a reaction ratio of 1:5 (final concentration: 20 µM : 100 µM) in PBS (pH 7.4) at room temperature for 2 hours.

The reaction product was identified through SDS-PAGE to confirm the conjugation of Cy3 TCO. This result is shown in FIG. 20. As a result of analysis using a fluorescent analysis system (ChemiDoc XPS+ system, BIORAD), a band with fluorescence was identified only in the Cy3 TCO-added conjugate lane. Therefore, it was confirmed that the sfGFP-hGH variant including frTet has IEDDA reactivity.

### 1.2. Preparation of hGH variant-HSA conjugate

### (1) Preparation of sfGFP-hGH variant-HSA conjugate (partner fusion protein-long-acting protein conjugate)

The inventors of the present application prepared an hGH variant-HSA conjugate in a similar manner to that of a GLP1 variant-HSA conjugate.

First, after preparing the sfGFP-hGH variant-HSA conjugate, sfGFP was removed, thereby preparing an hGH variant-HSA conjugate.

An HSA-linker1 complex was prepared based on the method disclosed in the prior literature ((Bak, Mijeong, et al. "Recombinant Peptide Production Platform Coupled with Site-Specific Albumin Conjugation Enables a Convenient Production of Long-Acting Therapeutic Peptide." Pharmaceutics 12.4 (2020): 364). The linker1 was reacted with HSA at a molar ratio of 4:1 (final concentration: 200 µM : 50 µM) in a 20 mM Bis-Tris buffer (pH 7.0) for 3 hours.

The HSA-linker1 complex sample (the reaction product of HSA and linker1) generated after reaction was desalted using a PD-10 column for conjugation with sfGFP-hGH-frTet and PBS (pH 7.4).

To prepare a sfGFP-hGH variant-HSA conjugate by IEDDA, sfGFP-hGH variant and a HSA-linker1 complex were reacted at room temperature for 16 hours at a molar ratio of 1:4 (final concentration: reacted at 15 µM : 60 µM or 20 µM : 80 µM), that is, reacted at the final concentration ratio of 15 µM : 60 µM (concentration1) or 20 µM : 80 µM (concentration 2), followed by analyzing an albumin binding yield.

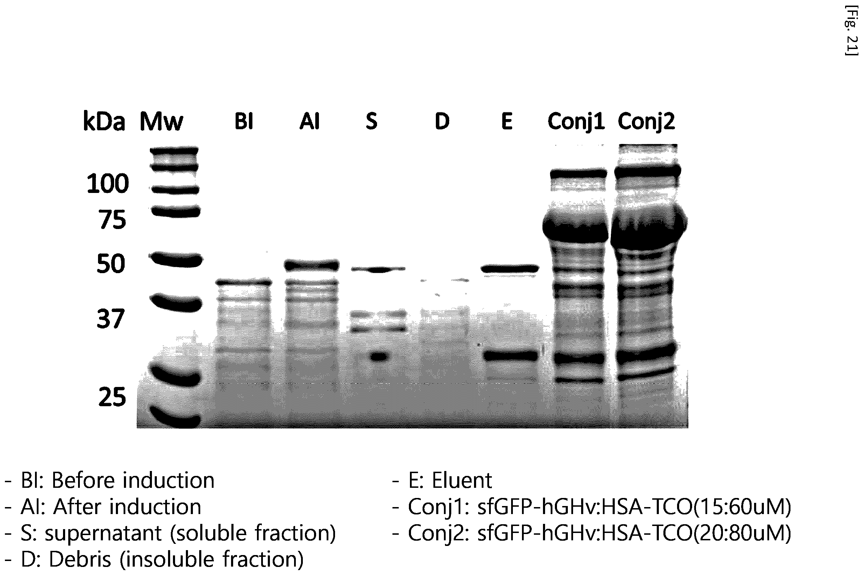

The sfGFP-hGH variant-HSA conjugate that had been prepared by conjugation through IEDDA was analyzed by SDS-PAGE. The SDS-PAGE analysis result is shown in FIG. 21. Here, Conj 1 and Conj2 indicate the Concentration 1 sample and Concentration 2 sample. By comparing and analyzing information including the thicknesses of bands shown herein, the binding degree of HSA in the sfGFP-hGH variant-HSA conjugate was calculated. The thicknesses of the protein bands shown herein were compared through image analysis to calculate an albumin binding degree.

When conditions were set differently, the albumin binding yield of Conj 1 (sfGFP-hGH variant : HSA-linker1 complex = 15 µM : 60 µM) and Conj2 (sfGFP-hGH variant : HSA-linker1 complex = 20 µM : 80 µM) was analyzed differently. Particularly, the albumin binding yield was confirmed to be approximately 75% based on intact sfGFP-hGH participating in the reaction under Conj2 condition.

Afterward, the inventors of the present application conducted cation exchange chromatography to purify the sfGFP-hGH variant-HSA conjugate from the reaction product of the sfGFP-hGH variant-HSA conjugate (HiTrap SP-HP, 5 mL).

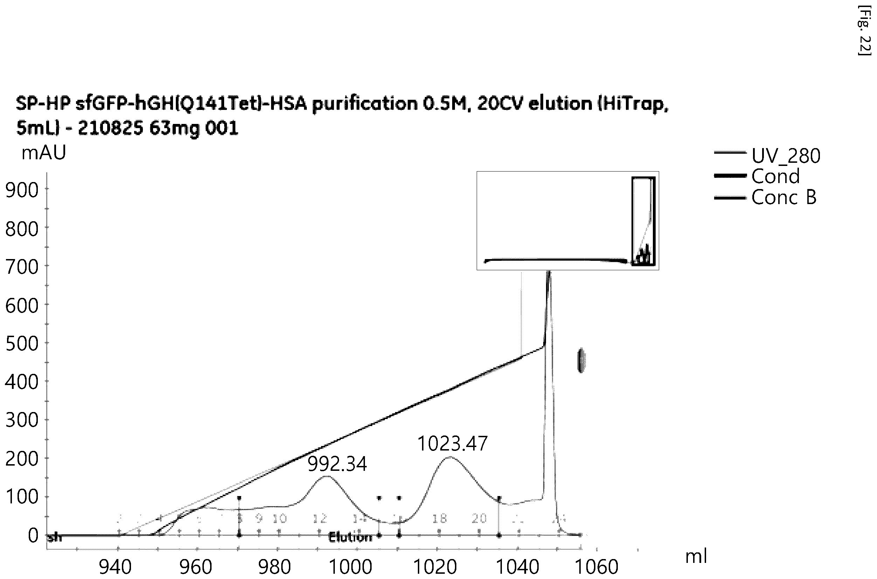

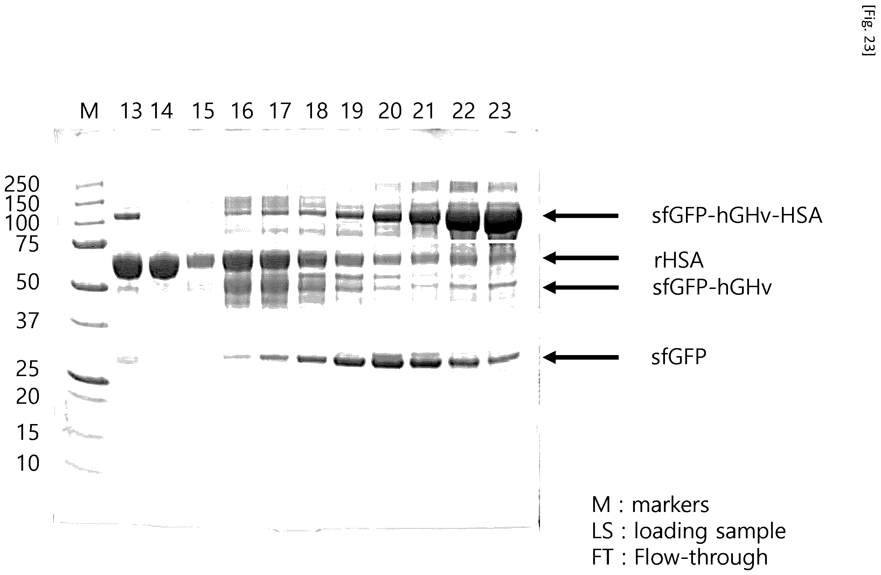

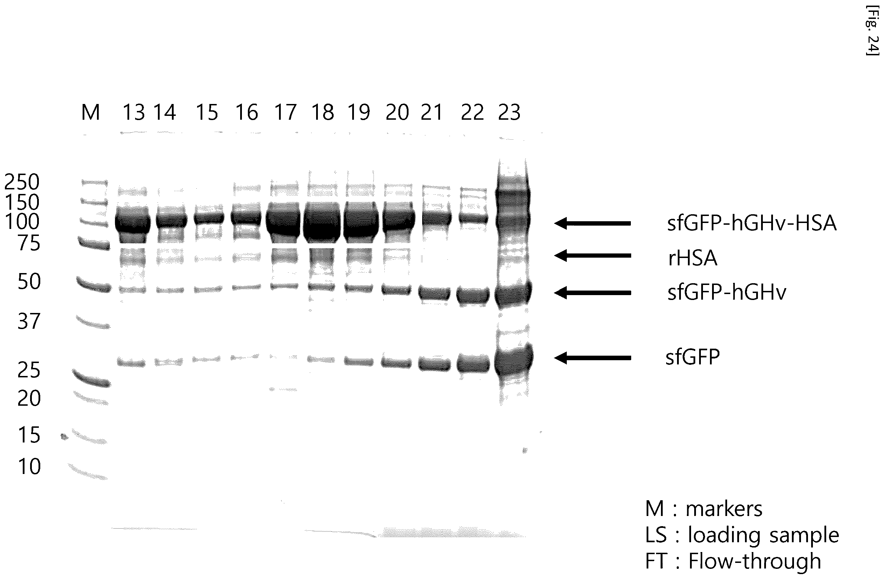

Each reaction product was exchanged with a buffer (20 mM sodium phosphate, pH 6.0), and injected into a HiTrap SP-HP column to separate the sfGFP-hGH variant-HSA conjugate. The results are shown in FIGS. 22, 23 and 24.

### (2) sfGFP removal

The inventors of the present application removed sfGFP from the sfGFP-hGH variant-HSA conjugate.

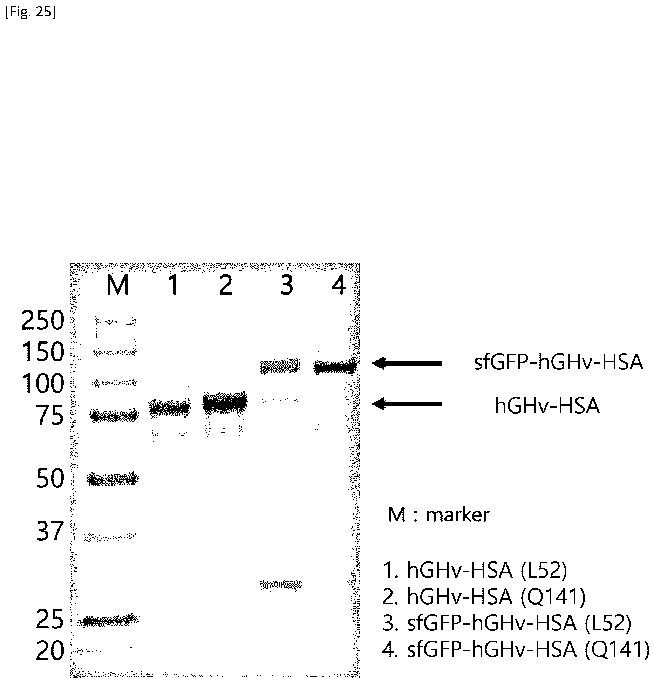

Afterward, fractions of the sfGFP-hGH variant-HSA conjugate were collected and exchanged with a Factor Xa reaction buffer (20 mM Tris, 2 mM CaCl₂, 10 mM NaCl, pH 8.0), and the sfGFP-hGH variant-HSA conjugate and the Factor Xa enzyme were reacted in a ratio of 1:250(w/w) at room temperature for 20 hours or more. Subsequently, through SDS-PAGE, the cleavage of sfGFP was confirmed. The result is shown in FIG. 25. In FIG. 25, hGHv-HSA indicates the hGH variant-HSA conjugate, and sfGFP-hGHv-HSA indicates the sfGFP-hGH variant-HSA conjugate. In addition, L52 and Q141 indicate the insertion of a non-natural amino acid at position 52 of hGH and the insertion of a non-natural amino acid at position 141 of hGH, respectively. As a result of treatment of an approximate 117 kDa sfGFP-hGH variant-HSA conjugate with Factor Xa, an approximate 89 kDa hGH variant-HSA conjugate was obtained.

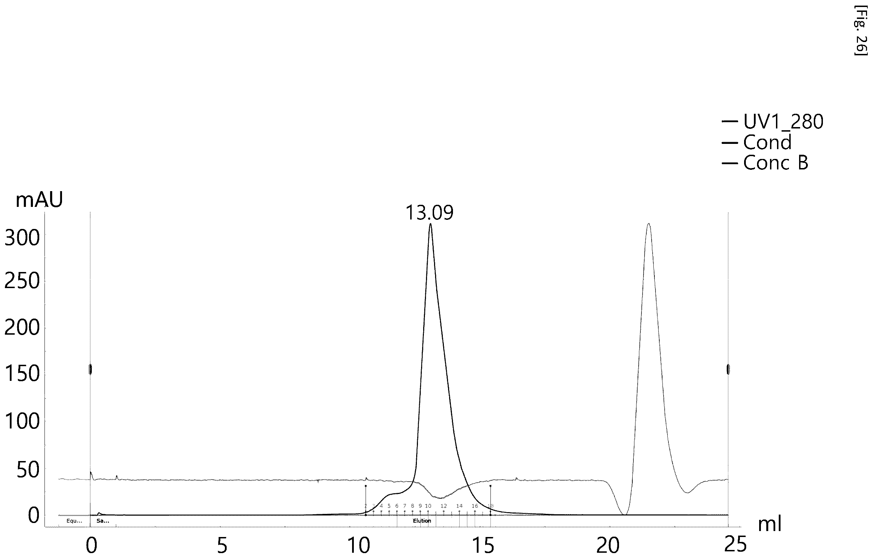

Afterward, the inventors of the present application separated and purified the hGH variant-HSA conjugate. The reaction product treated with Factor Xa was exchanged with 20 mM Bis-Tris buffer (pH 6.0), and the hGH variant-HSA conjugate and sfGFP were separated using anion exchange chromatography (Q-HP). To increase the purity of the hGH variant-HSA conjugate, size exclusion chromatography (SEC) was used for separation and purification. The result is shown in FIG. 26.

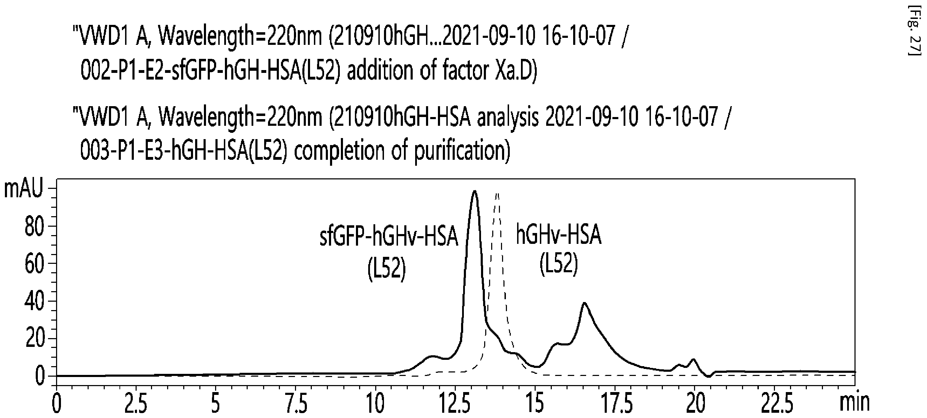

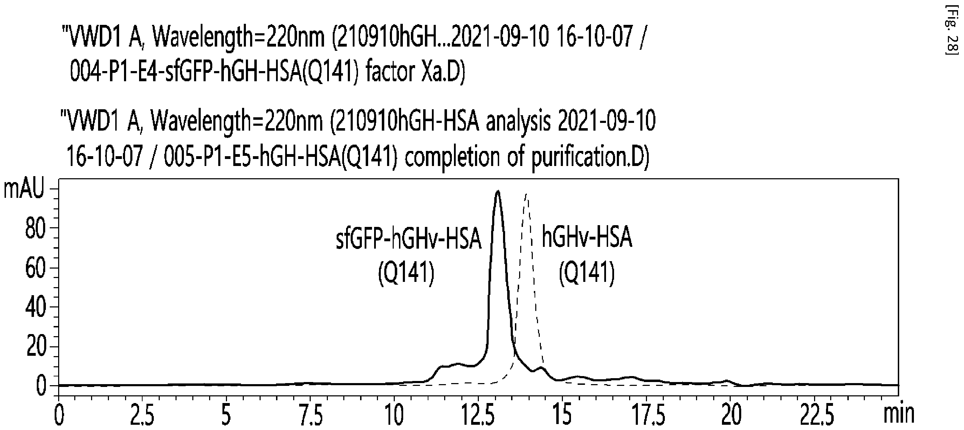

In addition, high performance liquid chromatography (HPLC) was used to confirm the purity of the hGH variant-HSA conjugate. The result is shown in FIGS. 27 and 28.

### 2. In vitro activity of hGH variant-HSA conjugate

The *in vitro* activity of the hGH variant-HSA conjugate is evaluated.

For example, the *in vitro* activity of the hGH variant-HSA conjugate may be evaluated by the following method.

The activity of the hGH variant-HSA conjugate is evaluated by confirming cell growth using mouse melanoma cells, that is, B 16F 10 cells (Korean Cell Line Bank, KCLB 80008). The cells is plated in a 90 mm dish, serum free RPMI 1640 (Lonza, Belgium; 1% antibiotic-antimycotic) is added to culture for 24 hours, and then the resulting cells is seeded in a 96-well plate at 5×10³ cells/well. The cells is treated with the hGH variant-HSA conjugate by concentration, incubated for 24 hours, and treated with a 3-(4,5-dimethylthiazol-2-yl)-2,5-diphenyltetrazolium bromide (MTT) solution, followed by further culture for 4 hours. Afterward, the medium is removed, DMSO is added to lyse the cells for 15 minutes, and then absorbance is measured at 570 nm. As a control, a recombinant growth hormone, Eutropin Injection (LG Chem) is used.

### 3. In vivo activity of hGH variant-HSA conjugate

The *in vivo* activity of the hGH variant-HSA conjugate is evaluated.

The *in vivo* activity of the hGH variant-HSA conjugate is tested with mice from which the pituitary gland is removed.

### 4. Pharmacokinetics (PK) of hGH variant-HSA conjugate

For pharmacokinetic evaluation of the hGH variant-HSA conjugate, 7-week-old male ICR mice (n=6) were used. 65 nmol/kg each of hGH variant-HSA (L52) and hGH variant-HSA (Q141) was intravenously injected into the tail, blood collection was performed at 0.5, 4, 8, 12, and 24 hours after injection, and centrifugation was performed at 2,500 rpm for 10 minutes, followed by storing at -20 °C. As a comparative group, Norditropin (somatropin, Novo Nordisk) was administered at the same concentration, and then compared with the above sample.

The hGH variant-HSA conjugate in blood was analyzed by sandwich ELISA (human growth hormone (GH) DouSet ELISA, R&D Systems). An anti-hGH capture antibody was added to coat an immunoplate at 4 °C overnight, and a reaction was blocked with 1% BSA in PBS at 37 °C for 1 hour. The blood sample collected and was diluted on the washed plate and left for 2 hours, and then treated with an anti-hGH detection antibody at room temperature for 2 hours. After treating the detection antibody with streptavidin-HRP at room temperature for 1 hour, 100 µL of a substrate was treated for 5 minutes and then 2N H₂SO₄ was treated to stop the reaction. Afterward, a microplate reader (Hidexsense, Finland) was used to measure an optical density at 450 nm.

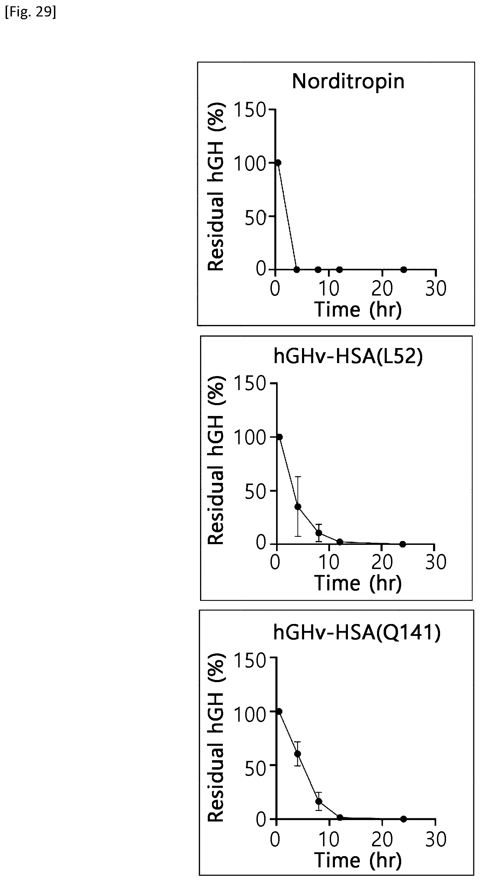

The pharmacokinetic evaluation result of the hGH variant-HSA conjugate is shown in FIG. 29. Here, hGHv-HSA (L52) indicates the result for an hGH variant-HSA conjugate prepared with the hGH variant (L52), and hGHv-HSA (Q141) indicates the result for an hGH variant-HSA conjugate prepared with the hGH variant (Q141).

As a result, the hGH variant-HSA conjugate prepared with the hGH variant (L52) (hGH variant-HSA conjugate (L52)) showed a half-life of 2.09 hours, the hGH variant-HSA conjugate prepared with the hGH variant (Q141) (hGH variant-HSA conjugate (Q141)) showed a half-life of 1.51 hours, and Norditropin showed a half-life of 0.5 hours, and it was confirmed that, compared with the positive control Noditrophin, the 2 types of albumin-binding hGH variant-HSA conjugates had an approximate 3- to 4-fold longer half-life.

### sfGFP variant-ABD conjugate

### 1. Expression and purification of frTet-introduced sfGFP variant and albumin-binding domain-cysteine (ABD-C) variant

To express sfGFP containing frTet at position 150 of the frTet-introduced sfGFP variant (sfGFP-frTet), Top10 [pDule C11] [pBAD sfGFP 150Amb] cells were manufactured based on the method disclosed in the prior literature (B. Yang, K. Kwon, S. Jana, S. Kim, S. Avila-Crump, G. Tae, R.A. Mehl, I. Kwon, Temporal Control of Efficient In Vivo Bioconjugation Using a Genetically Encoded Tetrazine-Mediated Inverse-Electron-Demand Diels-Alder Reaction, Bioconjug Chem, 31 (2020) 2456-2464.).

A pBAD-sfGFP-150Amb vector used for expression of the sfGFP variant includes the sequence of SEQ ID NO: 92.

For the expression and purification of sfGFP-frTet, a transformant was grown in 2x YT broth containing ampicillin (100 µg/mL) and tetracycline (10 µg/mL) at 200 rpm and 37 °C for 8 hours, inoculated into fresh 2x YT broth, and further cultured under the same conditions for 2.5 hours. When a cell density (optical density at 600 nm, OD 600) reached 0.4, frTet was added to the culture medium at the final concentration of 1 mM. Afterward, when OD 600 reached 0.6, L-(+)-arabinose was added to induce sfGFP-frTet expression. After 5-hour culture, cells were harvested by centrifugation at 5,000 rpm and 4 °C for 10 minutes.

The purification of sfGFP-frTet was performed through affinity chromatography (Ni-NTA) using 6xHis tag according to a manufacturer's protocol (Qiagen). A protein in an elution fraction was desalted with PBS (pH 7.4) at 4 °C using a PD-10 column.

To express an ABD variant (ABD-C) containing cysteine at position 4 of ABD, Top10 *E. coli* host cells were transformed with a pQE80 ABD-C plasmid (J. Cho, S.H. Kim, B. Yang, J.M. Jung, I. Kwon, D.S. Lee, Albumin affibody-outfitted injectable gel enabling extended release of urate oxidase-albumin conjugates for hyperuricemia treatment, J Control Release, 324 (2020) 532-544.). Top10 [pQE80 ABD-C] cells were inoculated into TSB+YE medium containing ampicillin (100 µg/mL) at 37 °C. When OD 600 reached 0.8, isopropyl β-D-1-thiogalactopyranoside (IPTG) was added at the final concentration of 1 mM to induce. After 20 hours, expressed cells were harvested by centrifugation at 5,000 rpm and 4 °C for 10 minutes, and affinity chromatography (Ni-NTA) was performed to purify ABD-C.

### 2. Preparation of sfGFP variant-ABD conjugate

The purified ABD-C and linker1 were added to PBS (pH 7.0) in a molar ratio of 1 : 4, and reacted at room temperature for 2 hours. To remove linker1, the reaction mixture was desalted with PBS using a centrifugal concentrator (MWCO 3K), thereby obtaining an ABD-linker1 complex mixture. Subsequently, the sfGFP variant and the ABD-linker1 complex were added to 20 mM sodium phosphate buffer (pH 6.5) in a molar ratio of 1 : 4 and reacted at room temperature for 5 hours, and the sfGFP variant and ABD-linker1 were conjugated to prepare an sfGFP variant-ABD conjugate. Afterward, the mixture was subjected to cation exchange chromatography (SP-HP) using the NGC Quest 10 Plus chromatography system at pH 6.0 to remove an unreacted product from an elution fraction. Elution fractions containing the sfGFP variant-ABD conjugate were pooled and concentrated to a high concentration using a centrifugal concentrator (MWCO 10K).

The net charge of the sfGFP variant at pH 6.0 was positive, and the sfGFP variant-ABD conjugate was positively charged at pH 6.0, higher than the sfGFP variant. Therefore, the sfGFP variant-ABD conjugate was eluted under sodium chloride gradient elution conditions after the sfGFP variant.

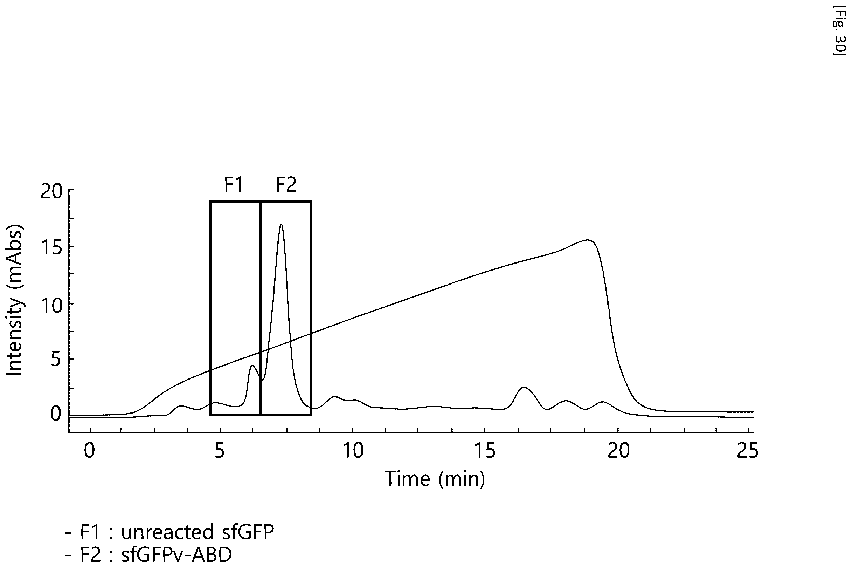

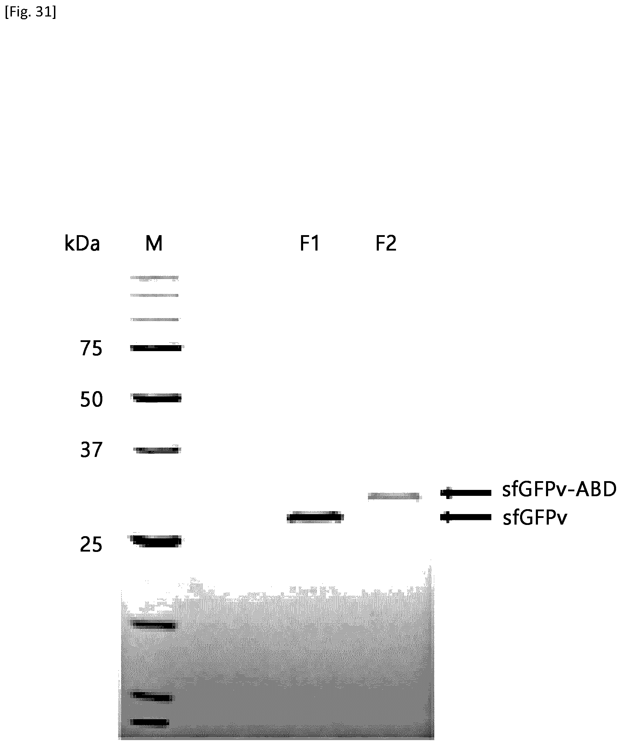

The result of separation and purification of the sfGFP variant and the sfGFP variant-ABD conjugate using cation exchange chromatography is shown in FIG. 30. In addition, the SDS-PAGE analysis result is shown in FIG. 31. Here, sfGFPv-ABD indicates the sfGFP variant-ABD conjugate, and sfGFPv indicates the sfGFP variant.

### 3. PK of sfGFP variant-ABD conjugate

sfGFP-WT or an sfGFP variant-ABD conjugate (0.8 nmol in 200 µL PBS at pH 7.4) were injected into the tail veins of young female BALB/c mice (n = 4). Blood samples were obtained 10 minutes, 2, 4, 8 and 12 hours after injection of the sfGFP-WT and the sfGFP variant-ABD conjugate through posterior orbital blood collection. To quantify residual sfGFP in serum, the samples were centrifuged and analyzed using a GFP ELISA kit (Cell Biolabs Inc, San Diego, CA).

As a result, the serum half-life of sfGFP-WT was 1.2 hours, similar to the previously reported half-life (B. Yang, S.I. Lim, J.C. Kim, G. Tae, I. Kwon, Site-Specific Albumination as an Alternative to PEGylation for the Enhanced Serum Half-Life in Vivo, Biomacromolecules, 17 (2016) 1811-1817.). The serum half-life of sfGFP-frTet-ABD was 12.5 hours. Compared with the serum half-life (1.2 hours) of sfGFP-WT, the serum half-life of the sfGFP variant-ABD was extended 10.4-fold, showing that the conjugation of the albumin-binding domain molecule significantly improves the serum half-life of a target protein.

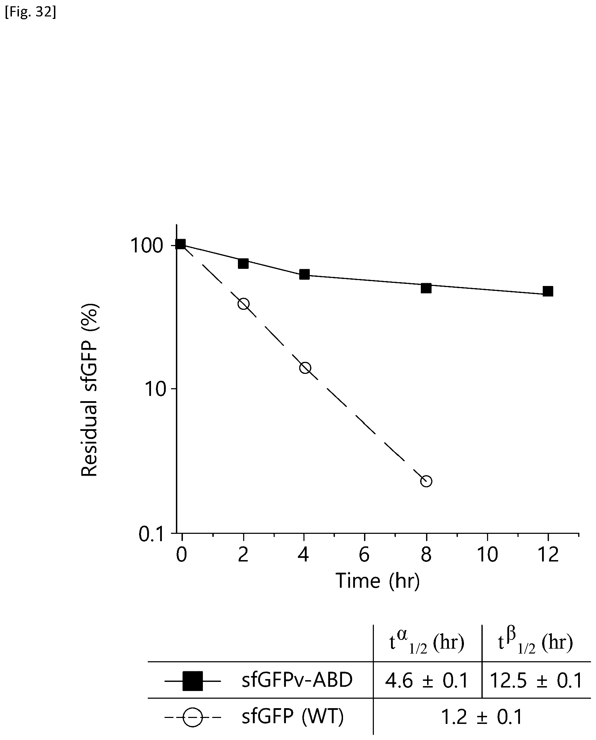

The result of analyzing the half-lives of sfGFP-WT and the sfGFP variant-ABD conjugate is shown in FIG. 32. In FIG. 32, sfGFPv-ABD indicates the sfGFP variant-ABD conjugate.

### Preparation of non-natural amino acid (frTet)

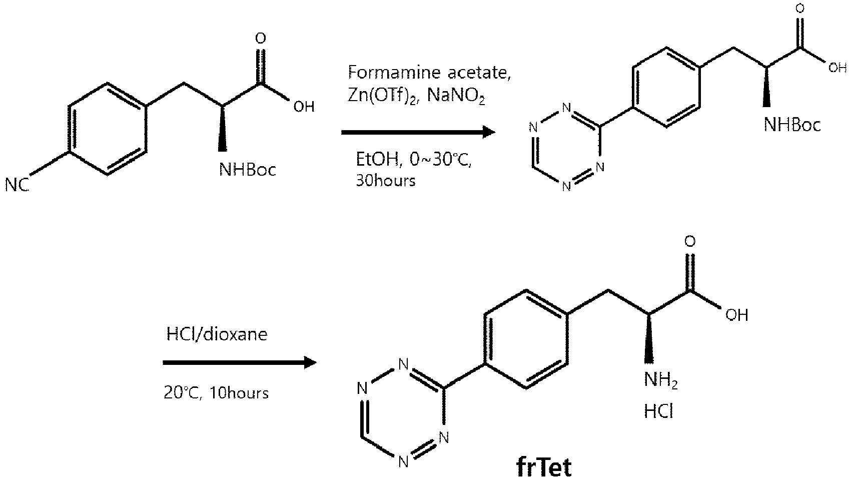

Starting materials (S)-2-((tert-butoxycarbonyl)amino)-3-(4-isocyanophenyl)propanoic acid, formamidine acetate, Zn(OTf)₂, and NaNO₂ were added to ethanol and reacted at 0 to 30 °C for 30 hours, thereby preparing (S)-2-((tert-butoxycarbonyl)amino)-3-(1,2,4,5-tetrazin-3-yl)propanoic acid as an intermediate, HCl and dioxane were added thereto and reacted at 20 °C for 10 hours, and separation and purification were performed using a silica column, thereby preparing frTet.

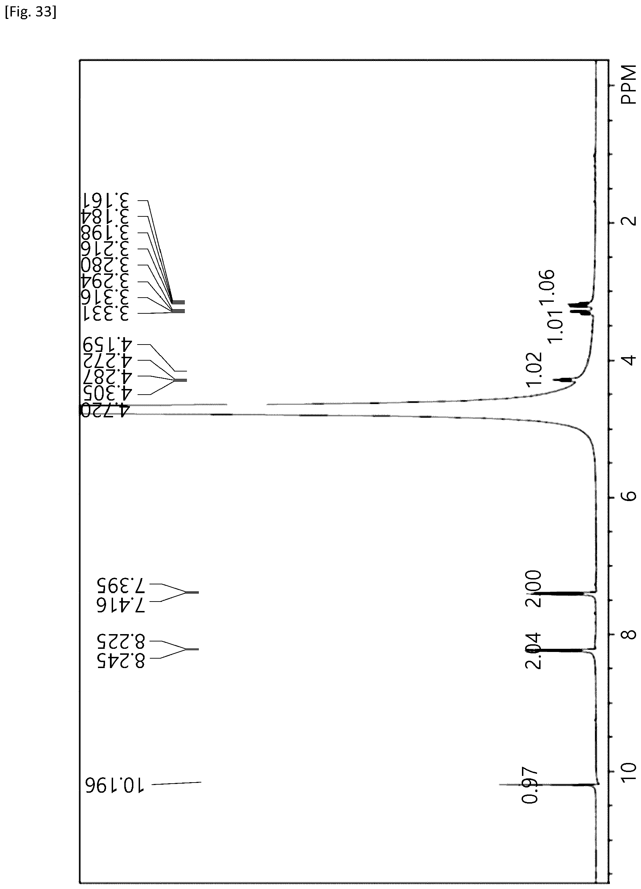

The H-NMR data of frTet is shown in FIG. 33.

### Acquisition of Arthrobacter globiformis-derived urate oxidase-albumin conjugate and confirmation of its effect

### 1. Experimental materials

4-(1,2,3,4-tetrazin-3-yl) phenylalanine (frTet) was purchased from Aldlab Chemicals (Woburn, MA, USA). Trans-cylooctene (TCO)-Cy3 was purchased from AAT Bioquest (Sunnyvale, CA, USA). TCO-PEG4-maleimide (TCO-PEG4-MAL) and amine-axially substituted TCO (TCO-amine) were purchased from FutureChem (Seoul, Korea). Pentafluor-ophenyl ester (PFP)-PEG4-APN was purchased from CONJU-PROBE (San Diego, CA, USA). Disposable PD-10 desalting columns and Superdex 200 increase 10/300 GL columns were purchased from Cytiva (Uppsala, Sweden). Vivaspin 6 centrifugal concentrators having molecular weight cut-offs (MWCOs) of 10 and 100 kDA were purchased from Sartorius (Gcpttingen, Germany). Human serum albumin (HSA) and all other chemical reagents were purchased from Sigma-Aldrich unless defined otherwise.

### 2. Construction of vector for acquiring Arthrobacter globiformis-derived urate oxidase variant (AgUox-frTet)

Genes encoding *Arthrobacter globiformis-derived* urate oxidase (AgUox) and its variant were synthesized by commissioning Macrogen (Seoul, South Korea). To express wild-type AgUox (AgUox-WT), or an AgUox variant in which a non-natural amino acid frTet is introduced into the sequence (AgUox-frTet), the synthesized gene was used as a template, and amplified through polymerase chain reaction (PCR) using primers pBAD-AgUox F (5'-GCCGCCATGGTGTCTGCTGTGAAGG-3', SEQ ID NO: 107) and pBAD-AgUox R (5'-GCCGAGATCTTTAATGGTGATGGTG-3', SEQ ID NO: 108). The amplified gene was digested with two restriction enzymes (NcoI and BglIII), and inserted into NcoI and BgIII sites of the pBAD vector, thereby synthesizing pBAD_AgUox. To replace the glutamic acid codon at position 196 in the AgUox-WT sequence with the amber codon (UAG), the pBAD-AgUox was used as a template, and pBAD-AgUox_196amb was prepared using primers AgUox-196Amb_F (5'-GTCGAAGTCCACCTATACGGTGTTGTAACGCCAACGG-3' SEQ ID NO: 109) and AgUox-196Amb_R (5'-CCGTTGGCGTTACAACACCGTATAGGTGGACTTCGAC-3', SEQ ID NO: 110).

### 3. Acquisition of AgUox-frTet

To express AgUox-frTet, referring to the method disclosed in Yang et.al, Temporal Control of Efficient In Vivo Bioconjugation Using a Genetically Encoded Tetrazine-Mediated Inverse-Electron-Demand Diels-Alder Reaction, Bioconjugate Chemistry, 2020, 2456-2464, AgUox-frTet was expressed by the following method using C321△A.exp, pDule_C11, and pBAD_AgUox-196amb. *E. coli* cells having MjtRNA^{Tyr}/MjTyrRS optimized for frTet were prepared.

*E. coli* cells cultured in Luria broth (LB) containing ampicillin (100 µg/mL) and tetracycline (10 µg/mL) were shaken at 37 °C overnight, and then inoculated into 2x YT medium under the same conditions. After 2.5 hours of shaking culture, when the medium containing the cells reached an optical density of 0.5 at 600 nm, frTet and L-(+)-arabinose were added to the medium so as to become the final concentrations of 1mM and 0.4%(w/v), respectively. After culturing for 5 hours, the cells were centrifuged at 5000 rpm and 4 °C for 10 minutes, thereby acquiring AgUox-frTet. The AgUox-frTet was purified at 4 °C using fixed metal affinity chromatography according to a manufacturer's protocol (Qiagen). The purified AgUox-frTet was desalted with PBS (pH 7.4) using a PD-10 column. The process of expressing and purifying AgUox-WT follows a method similar to that of AgUox-frTet, but tetracycline and frTet were not added to the culture medium during the expression stage.

### 4. Analysis and confirmation of prepared AgUox-frTet

To confirm the prepared AgUox-frTet and AgUox-WT, they were digested with trypsin according to a manufacturer's protocol. A total of 0.4 mg/mL each of Uox variants (AgUox-WT and AgUox-frTet) was digested overnight at 37 °C, and desalted with ZipTip C18. The mixture digested with trypsin was mixed with a 2,5-dihydroxybenzoic acid (DHB) matrix solution (20 mg/mL of DHB in 30:70(v/v) acetonitrile: trifluoroacetic acid 0.1%), and analyzed using a Microflex MALDI-TOF/MS device (Bruker Corporation, Billerica, MA, USA).

### 5. Site-specific fluorescent dye labeling of AgUox-WT and AgUox-frTet

The purified AgUox-WT and AgUox-frTet were reacted with a TCO-Cy3 fluorescent dye in a 1:2 molar ratio in PBS (pH 7.4) at room temperature. After 2 hours, the reaction mixture was subjected to sodium dodecyl sulfate polyacrylamide gel electrophoresis (SDS-PAGE). The fluorescent image of a protein gel was obtained using a ChemiDoc XRS+ system (illumination at 302 nm, 510-610 nm filter, Bio-Rad Laboratories, Hercules, CA, USA). After fluorescent analysis, the protein gel was stained with Coomassie Brilliant Blue R-250 dye. A protein gel image was obtained using a ChemiDoc XRS+ system using white light illumination.

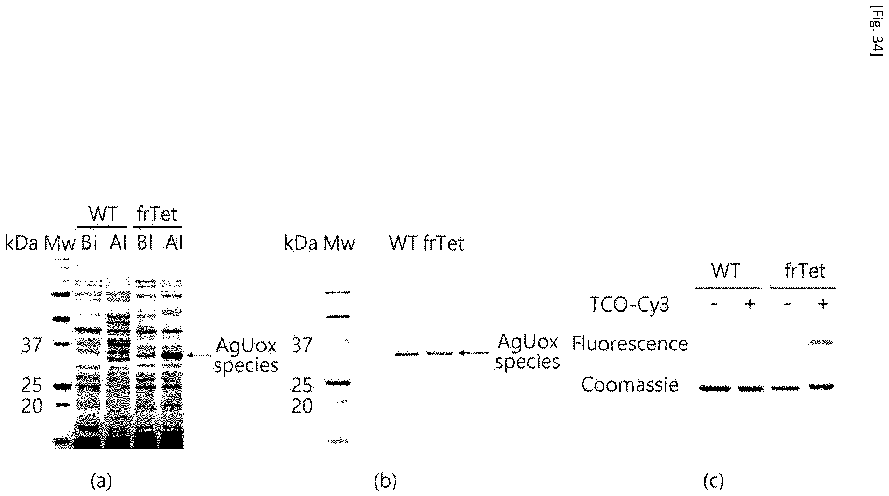

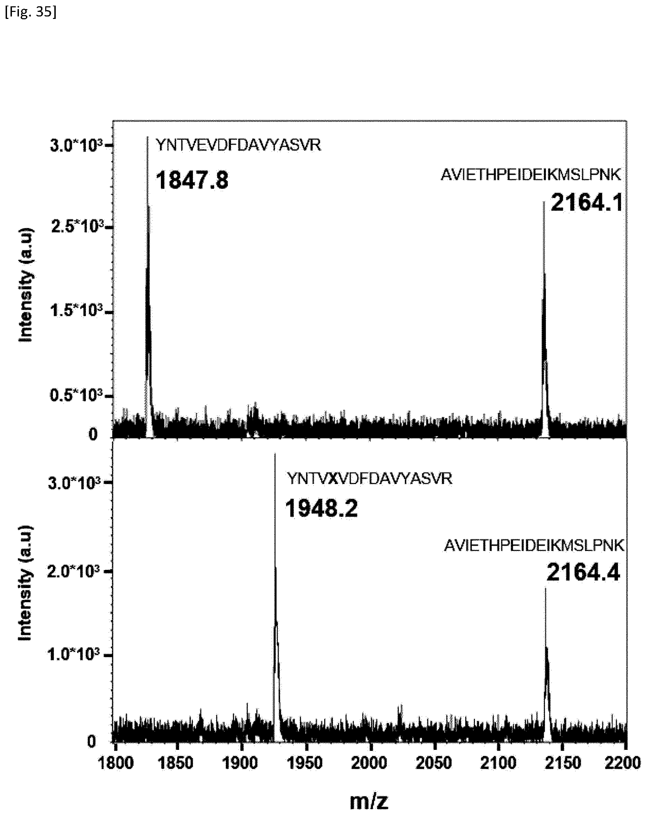

The results of analyzing the AgUox-WT and AgUox-frTet prepared in 3. to 5. above are shown in FIGS. 34 and 35.

### 6. Preparation of AgUox-HSA conjugates (AgUox-MAL-HSA and AgUox-APN-HSA)

To perform site-specific albumination on AgUox, HSA was purified by anion exchange chromatography using a HiTrap Q HP anion exchange column. The purified HSA was desalted with PBS (pH 7.0), reacted with TCO-MAL at room temperature in PBS (pH 7.0) at a molar ratio of 1:4. After 2 hours, the reaction mixture was desalted with PBS (pH 7.4) using a PD-10 column, and an unreacted TCO-PEG4-MAL linker was removed, thereby obtaining a MAL-HSA conjugate. The purified Uox-frTet was reacted with MAL-HSA in PBS (pH 7.4) at a molar ratio of 1:4 at room temperature for 5 hours. After conjugation, the reaction mixture was subjected to size exclusion chromatography (SEC) using an NGC Quest 10 Plus chromatography system (Bio-Rad Laboratories Inc., Berkeley, CA, USA). The molecular weight and purity of the eluted fraction were analyzed by SDS-PAGE, fractions corresponding to AgUox-frTet conjugated to four MAL-HSA molecules (AgUox-MAL-HSA) were selected, and concentrated for further analysis. To generate an AgUox-HSA conjugate through a hetero-bifunctional cross-linker containing TCO and APN, TCO-amine was reacted with pentafluorophenyl ester-PEG₄-APN (PFP-PEG₄-APN) at 1:1 molar ratio in DMSO for 30 minutes at room temperature. The buffer of the purified HSA was exchanged with 50 mM sodium borate buffer (pH 9.0). In 50 mM sodium borate buffer (pH 9.0), the purified HSA was reacted with TCO-PEG4-APN in a 1:4 molar ratio at room temperature for 2 hours. To remove an unreacted TCO-APN linker, the reaction mixture was desalted with PBS (pH 7.4) using a PD-10 column. The conjugation of AgUox-frTet conjugated to four HSA molecules via linkers containing APN (AgUox-APN-HSA) and purification were performed in a similar manner to that of AgUox-MAL-HSA.

TCO-PEG4-APN is represented by Formula 2-11 below.

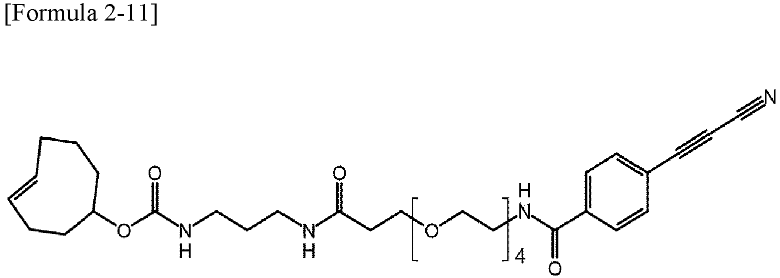

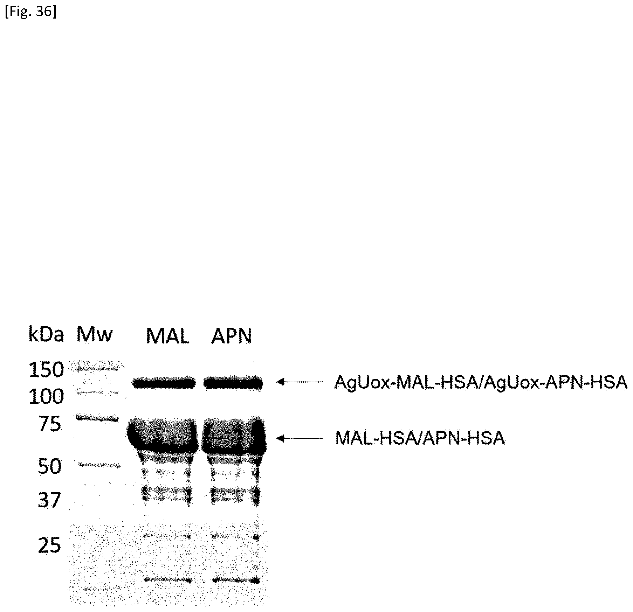

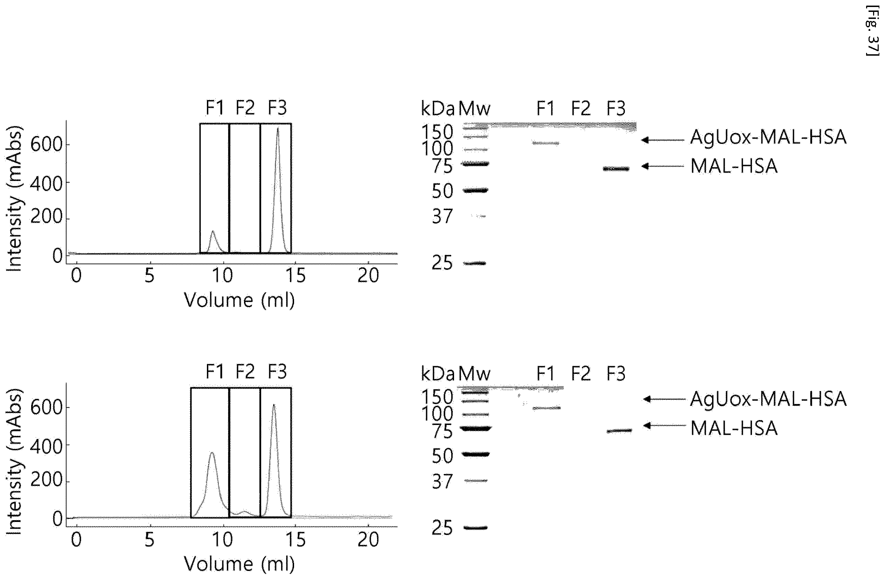

The result of preparing an AgUox-HSA conjugate according to 6. is shown in FIGS. 36 and 37.

### 7. Confirmation of in vivo half-life for AgUox-HSA conjugate (PK profile)

The stability analysis of an AgUox-HSA conjugate in mice was performed according to the guidelines of the Animal Care and Use Committee of the Gwangju Institute of Science and Technology (GIST-2020-037). Based on AgUox, 5.0 nmol of AgUox-WT, AgUox-MAL-HSA, or AgUox-APN-HSA in 200 µL PBS (pH 7.4) was injected into the tail vein of young female BALB/c mice (n=4). In the case of AgUox-WT, after 15 minutes, 3 hours, 6 hours and 12 hours, blood samples were collected through retro-orbital bleeding. In the case of the AgUox-HSA conjugate, blood samples were collected at 15 minutes, 12 hours, 24 hours, 48 hours, 72 hours, 84 hours, 96 hours, 108 hours and 120 hours by the same method as above. After separating serum from the collected blood, the serum activity of AgUox-WT, the AgUox-MAL-HSA, and the AgUox-APN-HSA was measured according to an absorbance change at 293 nm by adding 100 µL of enzyme active analysis buffer containing 100 µM uric acid to 100 µL of enzyme activity buffer containing 5 µL serum.

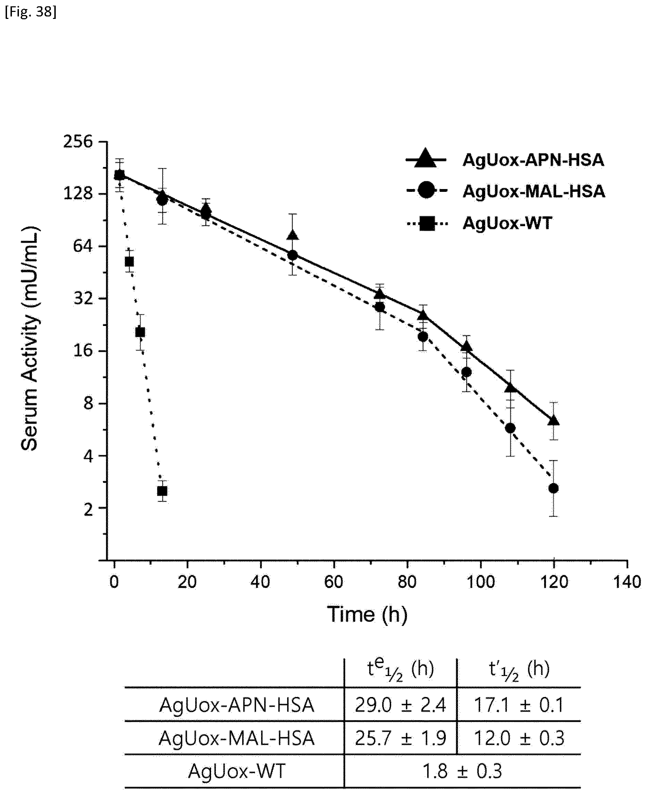

The result of confirming *in vivo* half-lives is shown in FIG. 38. As a result of the experiment, it was confirmed that significant half-life increases are shown in AgUox-APN-HSA and AgUox-MAL-HSA, compared to AgUox-WT to which albumin is not conjugated.

As described in the present application, a functional polypeptide variant-long-acting protein conjugate is provided. The functional polypeptide variant-long-acting protein conjugate has a longer half-life in the body and less immunogenicity.

In addition, the functional polypeptide variant-long-acting protein conjugate of the present application is prepared by an inverse electron-demand Diels-Alder reaction (IEDDA) reaction using a tetrazine or triazine group of a non-natural amino acid included in a functional polypeptide variant, so it has higher production yield, and/or a higher production rate than when using a strain-promoted alkyne-azide cycloaddition (SPAAC) reaction.

## Claims

1. A functional polypeptide variant-lasting protein conjugate represented by formula 1:
[Formula 1] P₁-J₁-A₂-J₂-P₂,
wherein,
Pi is a functional polypeptide variant moiety, wherein the functional polypeptide variant moiety is derived from a functional polypeptide variant, wherein the functional polypeptide variant comprises a first click chemistry functional group, wherein the first click chemistry functional group is tetrazine or its derivative, or triazine or its derivative,
A₂ is a second anchor moiety,
wherein the second anchor moiety is any one selected from substituted or unsubstituted C₁₋₂₀ alkylene, substituted or unsubstituted C₁₋₂₀ heteroalkylene, - substituted or unsubstituted C₁₋₂₀ alkylene-[PEG]n-, - substituted or unsubstituted C₁₋₂₀ heteroalkylene-[PEG]n-, -substituted or unsubstituted C₁₋₂₀ alkylene-[PEG]n-substituted or unsubstituted C₁₋₂₀ alkylene-, -substituted or unsubstituted C₁₋₂₀ alkylene-[PEG]n-substituted or unsubstituted C₁₋₂₀ heteroalkylene-, and -substituted or unsubstituted C₁₋₂₀ heteroalkylene-[PEG]n-substituted or unsubstituted C₁₋₂₀ heteroalkylene-,
wherein the heteroalkylene comprises one or more heteroatom groups selected from -NH-, -O-, -S-, -O-N=, -S(=O)-, and -S(=O)₂-, or one or more heteroatoms selected from O, N, and S,
wherein the substitution is a substitution with one or more non-hydrogen substituents, wherein the non-hydrogen substituent is any one selected from halogen, C₁₋₃ alkyl, -NH₂, =O, and =S,
wherein n is an integer of 1 to 12,
P₂ is a lasting protein moiety, wherein the lasting protein moiety is derived from a lasting protein,
J₁ is a first conjugation moiety, wherein the first conjugation moiety is formed by a reaction of the first click chemistry functional group comprising the tetrazine group or its derivative or the triazine group or its derivative with a second click chemistry functional group which is a dienophile, and
J₂ is a second conjugation moiety, wherein the second conjugation moiety is formed by a reaction of a thiol reactive group with a thiol residue.

2. The functional polypeptide variant-lasting protein conjugate of claim 1,
wherein the second anchor moiety is -A₂₁-A₂₂-A₂₃-,
wherein A₂₁ is -OC(=O)NH-,
wherein A₂₂ is absent, or unsubstituted C₁₋₁₀ alkylene, -unsubstituted C₁₋₁₀ alkylene-[PEG]n-, or -substituted C₁₋₁₀ heteroalkylene-[PEG]n-, wherein n is an integer of 1 to 12, and
wherein A₂₃ is absent, or -C(=O)NH- or -C(=O)O-.

3. The functional polypeptide variant-lasting protein conjugate of claim 1,
wherein the functional polypeptide variant-lasting protein conjugate is represented by formula 1-3:

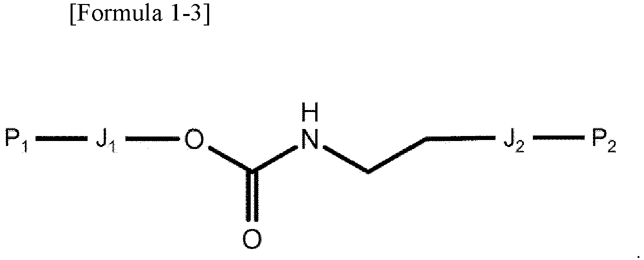

4. The functional polypeptide variant-lasting protein conjugate of claim 1,
wherein the functional polypeptide variant-lasting protein conjugate is represented by formula 1-4:

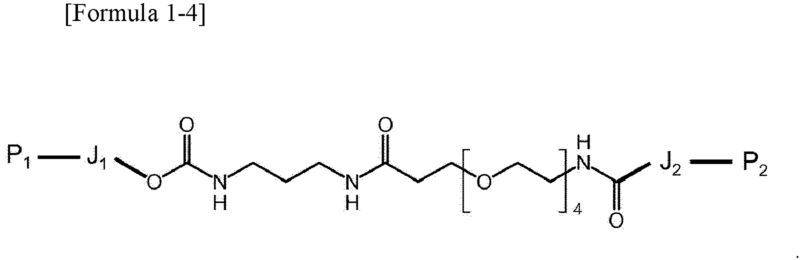

5. The functional polypeptide variant-lasting protein conjugate of claim 1,
wherein the second anchor is a substituted hydrocarbon chain comprising one or more heteroatoms,
wherein each of the heteroatom is selected from N, O, and S,
wherein the substitution is a substitution with one or more non-hydrogen substituents, wherein each of the non-hydrogen substituents is any one selected from the group consisting of halogen, C₁₋₃ alkyl, -NH₂, =O, and =S.

6. The functional polypeptide variant-lasting protein conjugate of claim 1,
wherein the first click chemistry functional group is represented by any one of the following structures: 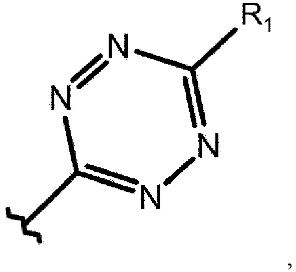 and 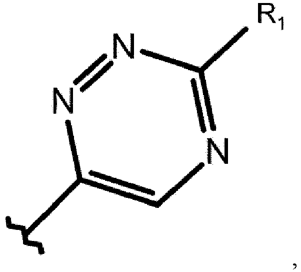
wherein Ri is any one selected from H, halogen, C₁₋₃ alkyl, C₃₋₆ cycloalkyl, C₃₋₆ heterocycloalkyl, aryl, and heteroaryl,
wherein the heterocycloalkyl or the heteroaryl comprises one or more heteroatom groups selected from the group consisting of -NH-, -O-, -S-, -O-N=, - S(=O)-, and -S(=O)₂-, or one or more heteroatoms selected from the group consisting of O, N, and S.

7. The functional polypeptide variant-lasting protein conjugate of claim 1,
wherein a non-natural amino acid comprised in the functional polypeptide variant is represented by the following structure: 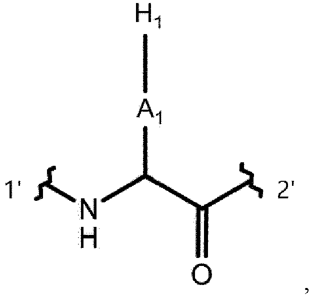 wherein,
Hi is the first click chemistry functional group, wherein the first click chemistry functional group comprises the tetrazine group or its derivative or the triazine group or its derivative,
A₁ is the first anchor moiety, wherein the first anchor moiety is absent or -A₁₁-A₁₂₋,
wherein A₁₁ is absent or C₁₋₅ alkylene,
wherein A₁₂ is absent, or is any one selected from [arylene]p, -[arylene]p-C₁₋₅ alkylene-, -[arylene]p-C₁₋₅ heteroalkylene-, -arylene-C₁₋₅ alkylene-arylene-, -arylene-C₁₋₅ heteroalkylene-arylene-, -arylene-heteroarylene-, [heteroarylene]p,-[heteroarylene]p-C₁₋₅ alkylene-, -[heteroarylene]p-C₁₋₅ heteroalkylene-,-heteroarylene-C₁₋₅ alkylene-arylene-, -heteroarylene-C₁₋₅ alkylene-heteroarylene-,-heteroarylene-C₁₋₅ heteroalkylene-arylene-, and -heteroarylene-C₁₋₅ heteroalkylene-heteroarylene-, wherein p is an integer of 1 to 3,
wherein the heteroalkylene or the heteroarylene comprises one or more heteroatom groups selected from the group consisting of -NH-, -O-, -S-, -O-N=, - S(=O)-, and -S(=O)₂-, or one or more heteroatoms selected from the group consisting of O, N, and S, and
wherein the non-natural amino acid is linked with neighboring amino acids through 1' and 2' within or at the end of an amino acid sequence of the functional polypeptide, to form the functional polypeptide variant of the present application.

8. The functional polypeptide variant-lasting protein conjugate of claim 7,
wherein the non-natural amino acid comprised in the functional polypeptide variant is represent by the following structure: 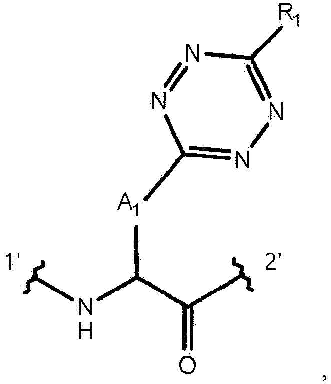
wherein Ri is any one selected from H, halogen, methyl, aryl, and heteroaryl.

9. The functional polypeptide variant-lasting protein conjugate of claim 7,
wherein the non-natural amino acid comprised in the functional polypeptide variant is represented by the following structure: 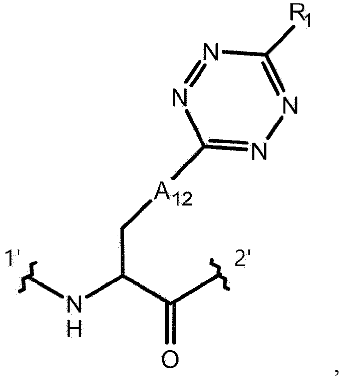 wherein Ri is any one selected from H, halogen, methyl, aryl, and heteroaryl.

10. The functional polypeptide variant-lasting protein conjugate of claim 7,
wherein the non-natural amino acid comprised in the functional polypeptide variant is represented by the following structure: 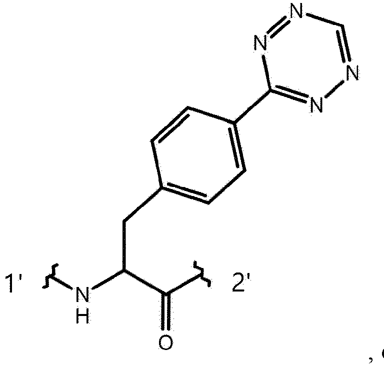 or

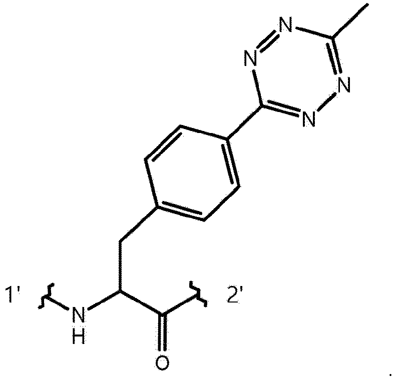

11. The functional polypeptide variant-lasting protein conjugate of claim 1,
wherein the second anchor moiety is derived from a linker, wherein the linker comprises the second click chemistry group that is the dienophile group reacting with the first click chemistry group, the thiol reactive group reacting with the thiol residue, and the second anchor moiety.

12. The functional polypeptide variant-lasting protein conjugate of claim 11,
the dienophile group is any one selected from trans-bicyclo[6.1.0]nonene group, trans-cyclooctene(TCO) group, methylcyclopropene group, bicyclo[6.1.0]nonyne group, cyclooctyne group, norbornene group, cyclopentene group, and styrene group.

13. The functional polypeptide variant-lasting protein conjugate of claim 11,
wherein the dienophile group is trans-cyclooctene (TCO) group.

14. The functional polypeptide variant-lasting protein conjugate of claim 11,
wherein the thiol reactive group is maleimide group or APN group.

15. The functional polypeptide variant-lasting protein conjugate of claim 11,
wherein the thiol reactive group is any one selected from the following structures: 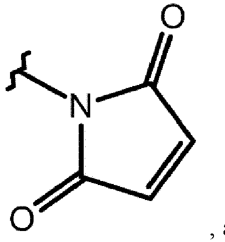 and

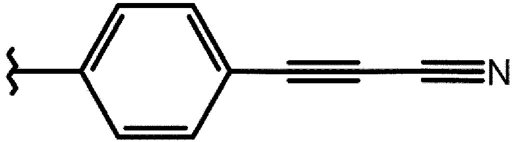

16. The functional polypeptide variant-lasting protein conjugate of claim 1,
wherein the first conjugation moiety is formed by the reaction of the tetrazine group with the trans-cyclooctene group,
wherein the first conjugation moiety is any one selected from the following structures: 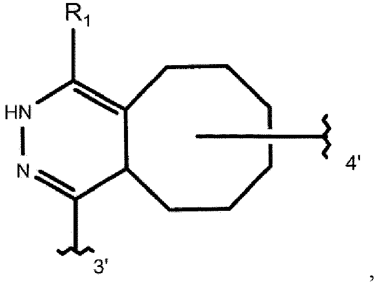 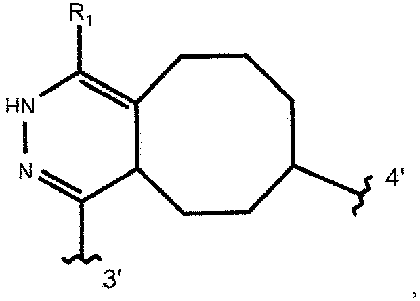 and 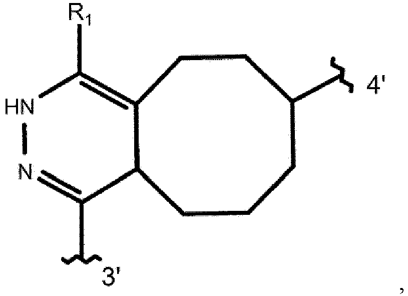
wherein 3' is linked to the functional polypeptide variant moiety, and 4' is linked to the second anchor moiety, and
wherein R₁ is any one selected from H, halogen, methyl, aryl, heteroaryl.

17. The functional polypeptide variant-lasting protein conjugate of claim 16,
wherein the first conjugation moiety is

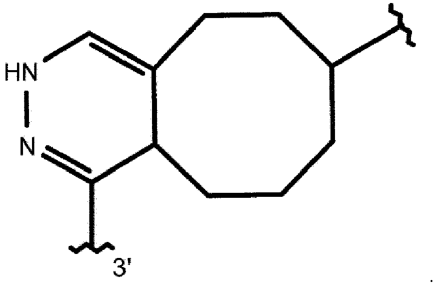

18. The functional polypeptide variant-lasting protein conjugate of claim 1,
wherein the second conjugation moiety is formed by the reaction of the thiol reactive group with the thiol residue comprised in the cysteine of the lasting protein,
wherein the second conjugation moiety is any one selected from the following structures: 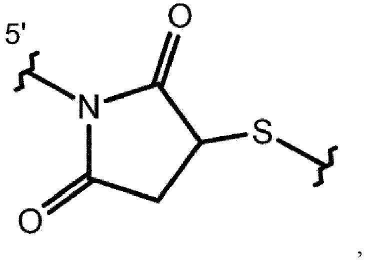 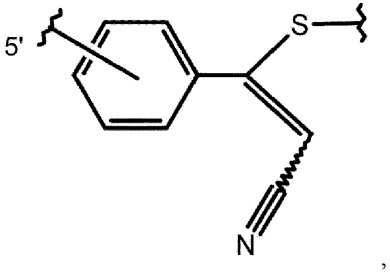 and 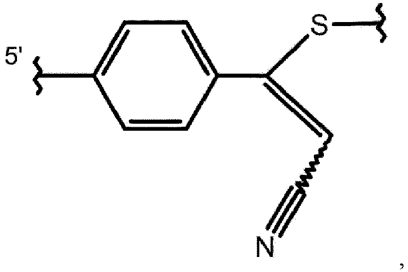
wherein 5' is linked to the second anchor moiety, and S is derived from the thiol residue comprised in the cysteine of the lasting protein.

19. The functional polypeptide variant-lasting protein conjugate of claim 1,
wherein the functional polypeptide variant is any one selected from GLP1 variant, hGH variant, Uox subunit variant, and sfGFP variant.

20. The functional polypeptide variant-lasting protein conjugate of claim 19,
wherein the functional polypeptide variant is the GLP1 variant,
wherein the GLP variant comprises any one sequence selected from SEQ ID NOs: 1 to 26.

21. The functional polypeptide variant-lasting protein conjugate of claim 20,
wherein the GLP1 variant comprises the sequence of SEQ ID NO: 2.

22. The functional polypeptide variant-lasting protein conjugate of claim 19,
wherein the functional polypeptide variant is the hGH variant,
wherein the hGH variant comprises a sequence in which one or more standard amino acids in SEQ ID NO: 27 are substituted with non-natural amino acids, wherein the standard amino acid substituted with non-natural amino acid is any one selected from Q29, Y35, L52, E56, E66, Q69, E74, F92, S106, Q122, R127, L128, G131, R134, Q141, T142, Y143, K145, D147, N149, N152, D153, and E186.

23. The functional polypeptide variant-lasting protein conjugate of claim 19,
wherein the functional polypeptide variant is the hGH variant,
wherein the hGH variant comprises any one sequence selected from SEQ ID NOs: 28 to 50.

24. The functional polypeptide variant-lasting protein conjugate of claim 23,
wherein the hGH variant comprises the sequence of SEQ ID NO: 28.

25. The functional polypeptide variant-lasting protein conjugate of claim 19,
wherein the functional polypeptide variant is the sfGFP variant,
wherein the sfGFP variant comprises a sequence in which one or more standard amino acids in SEQ ID NO: 51 are substituted with non-natural amino acids, wherein the standard amino acid substituted with non-natural amino acid is any one selected from E8, T11, V13, G26, K28, T40, N41, T51, D78, K103, K109, G118, K128, D131, D135, H150, Q159, K168, D175, D192, V195, N200, P213, and G230.

26. The functional polypeptide variant-lasting protein conjugate of claim 19,
wherein the functional polypeptide variant is the sfGFP variant,
wherein the sfGFP variant comprises any one sequence selected from SEQ ID NOs: 52 to 75.

27. The functional polypeptide variant-lasting protein conjugate of claim 26,
wherein the sfGFP variant comprises the sequence of SEQ ID NO: 52.

28. The functional polypeptide variant-lasting protein conjugate of claim 1,
wherein the lasting protein is an albumin or its variant, or an albumin binding domain or its variant.

29. The functional polypeptide variant-lasting protein conjugate of claim 1,
wherein the lasting protein is an albumin or its variant,
wherein the albumin or its variant comprises any one sequence selected from SEQ ID NOs: 76 to 87.

30. The functional polypeptide variant-lasting protein conjugate of claim 1,
wherein the lasting protein is an albumin binding domain or its variant,
wherein the albumin binding domain or its variant comprises a sequence of SEQ ID NO: 88.

31. The functional polypeptide variant-lasting protein conjugate of claim 1,
wherein the functional polypeptide variant-lasting protein conjugate is represented by the formula 1-3: 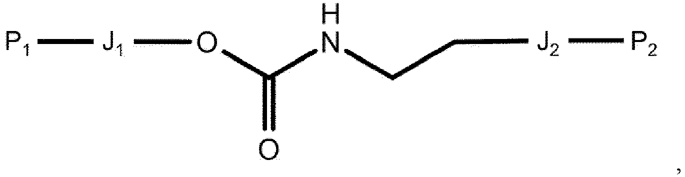 wherein,
the lasting protein is an albumin or its variant, or an albumin binding domain or its variant, wherein the lasting protein comprises a cysteine, wherein the cysteine comprises the thiol residue,
the first conjugation moiety is 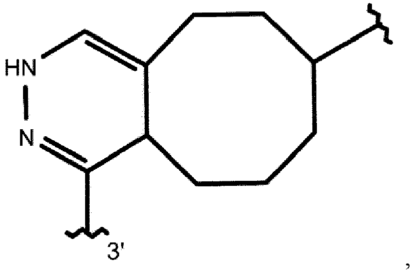
wherein 3' is linked to P₁, and
the second conjugation moiety is 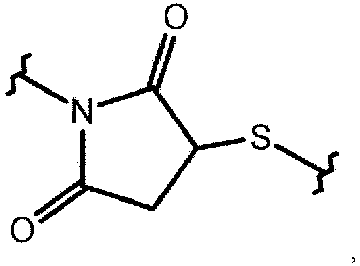 or 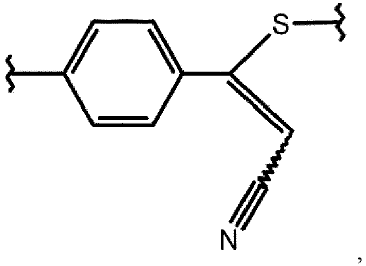
wherein S is derived from the thiol residue of the lasting protein.

32. The functional polypeptide variant-lasting protein conjugate of claim 31,
wherein the functional polypeptide variant is any one selected from a GLP1 variant, a hGH variant, an Uox subunit variant, and a sfGFP variant.

33. The functional polypeptide variant-lasting protein conjugate of claim 31,
wherein the functional polypeptide variant is a GLP1 variant,
wherein the GLP1 variant comprises a sequence of SEQ ID NO: 2, and
wherein the lasting protein comprises a sequence of SEQ ID NO: 76.

34. The functional polypeptide variant-lasting protein conjugate of claim 31,
wherein the functional polypeptide variant is a hGH variant,
wherein the hGH variant comprises a sequence of SEQ ID NO: 28, and
wherein the lasting protein comprises a sequence of SEQ ID NO: 76.

35. The functional polypeptide variant-lasting protein conjugate of claim 31,
wherein the functional polypeptide variant is a sfGFP variant,
wherein the sfGFP variant comprises a sequence of SEQ ID NO: 52, and
wherein the lasting protein comprises a sequence of SEQ ID NO: 88.

36. A method for preparing a functional polypeptide variant-lasting protein conjugate, comprising mixing a first composition comprising a lasting protein, a second composition comprising a linker, and a third composition comprising a functional polypeptide variant, wherein:
the lasting protein is an albumin or its variant or an albumin binding domain comprising a thiol residue,
the linker comprises a dienophile group which reacts inverse electron demand diels-alder reaction (IEDDA) with tetrazine group or its derivative or triazine group or its derivarive, and a thiol reactive group which reacts with the thiol residue, and
the functional polypeptide variant comprises a non-natural amino acid comprising the tetrazine or its derivative or the triazine or its derivative.

37. The method for preparing the functional polypeptide variant-lasting protein conjugate of claim 36,
wherein the method comprises:
mixing the first composition and the second composition, wherein a first reaction composition is prepared;
mixing the first reaction composition and the third composition, wherein a second reaction composition is prepared; and
obtaining the functional polypeptide variant-lasting protein conjugate from the second reaction composition.

38. A method for preparing a functional polypeptide variant-lasting protein conjugate, the method comprising:
preparing a partner fusion protein-lasting protein conjugate by mixing a first composition comprising a lasting protein, a second composition comprising a linker, and a third composition comprising a partner fusion protein; and
removing a partner protein from the partner fusion protein-lasting protein conjugate,
wherein the lasting protein is an albumin or its variant or an albumin binding domain comprising a thiol residue,
wherein the linker comprises a dienophile group which reacts inverse electron demand diels-alder reaction with a tetrazine group or its derivative or a triazine group or its derivative, and a thiol reactive group which reacts with the thiol residue,
wherein the partner fusion protein has a structure of [partner protein]-[amino acid linking moiety]-[functional polypeptide variant], or [partner protein]-[functional polypeptide variant], and
wherein the functional polypeptide variant comprises one or more non-natural amino acids comprising the tetrazine or its derivative or the triazine or its derivative.

39. The method for preparing the functional polypeptide variant-lasting protein conjugate of claim 38,
wherein the method comprises:
mixing the first composition and the second composition, wherein a first reaction composition is prepared;
mixing the first reaction composition and the third composition, wherein a second reaction composition comprising the partner fusion protein-lasting protein conjugate is prepared;
removing the partner protein from the partner fusion protein-lasting protein conjugate, wherein a third reaction composition is prepared by adding a polypeptide cleavage enzyme to the second reaction composition; and
obtaining the functional polypeptide variant-lasting protein conjugate from the third reaction composition.

40. A pharmaceutical composition for treating a target disease comprising a functional polypeptide variant-lasting protein conjugate of claim 33,
wherein the target disease is any one selected from diabetes, persistent diabetes, obesity, type 2 diabetes, prediabetes, liver disease, metabolic disease, and cardiovascular disease.

41. A pharmaceutical composition for treating growth hormone deficiency comprising a functional polypeptide variant-lasting protein conjugate of claim 34.
